# EUROPEAN PATENT SPECIFICATION

(11) **EP 4 493 226 B1**
(45) Date of publication and mention of the grant of the patent: **02.09.2026**
(21) Application number: 23718578.0
(22) Date of filing: 13.03.2023
(51) Int. Cl.: A61K 38/00, C07K 14/47, C12N 15/86, A61K 48/00, A61P 21/00

(54) **MODIFIED MUSCLE-SPECIFIC PROMOTERS**
MODIFIZIERTE MUSKELSPEZIFISCHE PROMOTOREN
PROMOTEURS SPÉCIFIQUES À UN MUSCLE MODIFIÉS

(30) Priority: 13.03.2022 US 202263319360 P
(43) Date of publication of application: 22.01.2025
(73) Proprietor: RegenxBio Inc., Rockville, MD 20850 (US)
(72) Inventor: LIU, Ye, Rockville, MD 20850 (US); QIAO, Chunping, Rockville, MD 20850 (US); BYRNE, Michael, Rockville, MD 20850 (US); BIDZHIEVA, Bella, Rockville, MD 20850 (US); QIAN, Randolph, Rockville, MD 20850 (US); DANOS, Olivier, Rockville, MD 20850 (US)
(74) Representative: Appleyard Lees IP LLP
(86) International application number: PCT/US2023/064261
(87) International publication number: WO 2023/178053

(56) References cited:
- WO-A1-2020/208032
- WO-A1-2021/146711
- WO-A1-2022/232141
- WO-A1-2022/241030

## Description

### BACKGROUND

Muscle-directed gene transfer has proven advantageous in the treatment of muscle disorders, such as dystrophinopathies. The use of regulatory elements to drive gene expression within specific tissues is advantageous, yet highly complex. The Spc5-12 promoter has been shown to be effective in promoting muscle-specific expression from adeno-associated viral (AAV) vectors. However, the need for higher yield of full genome AAV vectors exists as many AAV vectors using a Spc5-12 promoter for transgene expression can result in a high yield of AAV vectors with partial genomes.

Thus, there remains a need for tissue-targeted gene expression elements for use in vectors that are highly productive and manufacturable, especially muscle-specific elements.

### BRIEF SUMMARY

The inventors have unexpectedly discovered that modifying certain muscle-specific promoter sequences can improve packaging of AAV vectors having these muscle specific promoters within AAV virions, and, thus, result in enhanced production and recovery of fully packaged viral (capsids) particles, as well as provide AAV vectors exhibiting efficient expression of genes encoding therapeutic proteins from the AAV genomes in muscle tissue in the treatment of muscle disorders. Although not intending to be bound by any particular theory, promoter sequences forming hairpin loop secondary structure may inhibit genome packaging into AAV capsids. Thus, provided are modified muscle-specific promoter sequences that have reduced hairpin loop secondary structure, compared to parental promoter sequences. AAV genomes incorporating these modified promoter sequences into an expression cassette exhibit improved packaging efficiency as compared to AAV genomes with the parental promoter sequence in comparable expression cassettes, while still exhibiting muscle-specific expression of the therapeutic protein encoded by the expression cassettes. In embodiments, provided are modified Spc-5-12 promoter sequences, for example, promoter sequences having a nucleotide sequence of SEQ ID NO: 1 (v1) or SEQ ID NO: 2 (v2). Also provided are expression cassettes, recombinant AAV genomes, and recombinant AAV virus particles in which a nucleotide sequence encoding a gene of interest for expression in muscle tissue is operably linked to a modified Spc5-12 promoter, including a promoter having a nucleotide sequence of SEQ ID NO: 1 (v1) or SEQ ID NO: 2 (v2), and methods of manufacturing or using the same.

Additional advantages of the disclosed method and compositions will be set forth in part in the description which follows, and in part will be understood from the description, or may be learned by practice of the disclosed method and compositions. The advantages of the disclosed method and compositions will be realized and attained by means of the elements and combinations particularly pointed out in the appended claims. It is to be understood that both the foregoing general description and the following detailed description are exemplary and explanatory only and are not restrictive of the invention as claimed.

### BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed method and compositions.

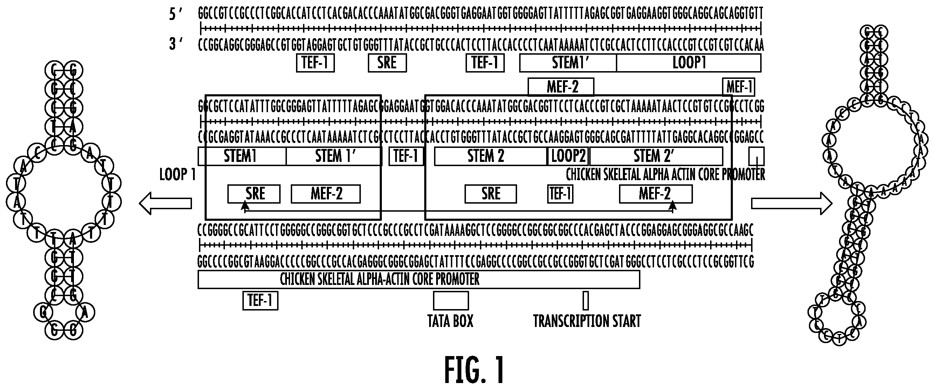

FIG. 1 shows predicted secondary structure of the represented (boxed) nucleotide sequences of Spc5-12 Mutant1 (SEQ ID NO:1) in which the positions of the MEF-2 and SRE elements are modified (labelled as STEM1-STEM1' (SEQ ID NO:61) and STEM2-LOOP2-STEM2' (SEQ ID NO:62)) as compared to the Spc5-12 promoter (SEQ ID NO: 3).

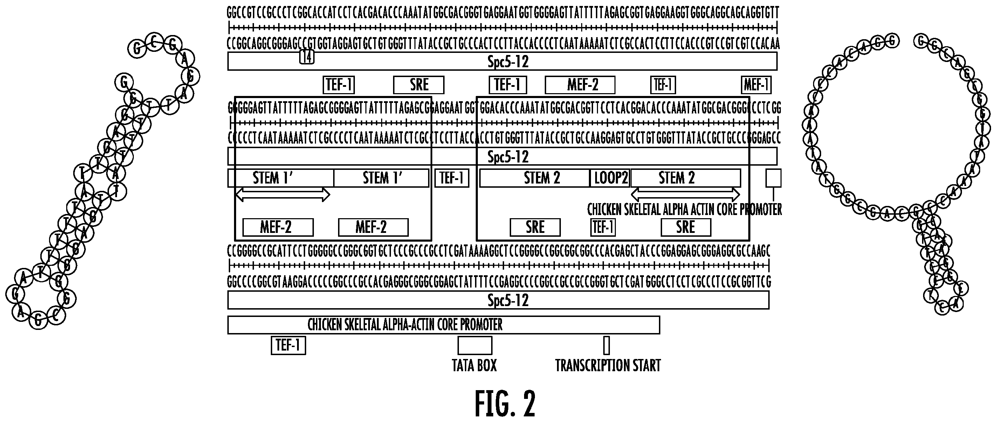

FIG. 2 shows predicted secondary structure of the represented (boxed) nucleotide sequences of Spc5-12 Mutant2 (SEQ ID NO:2) which modifies the orientation of the MEF-2 and SRE elements (labelled as STEM1'-STEM1' (SEQ ID NO:63) and STEM2-LOOP2-STEM2, SEQ ID NO: 64)) compared to the Spc5-12 promoter.

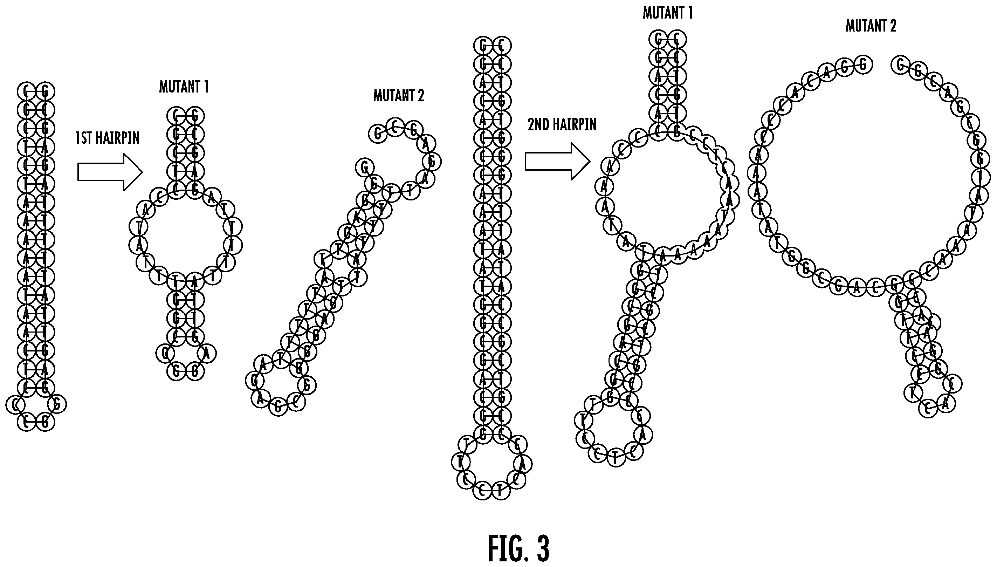

FIG. 3 shows the predicted first and second hairpin structures for the Spc5-12 promoter (SEQ ID NOs: 65 and 66, respectively) and the predicted change in secondary structure for the first and second hairpins of the Spc5-12vl promoter ("mutant 1") (SEQ ID NOs: 61 and 62, respectively) and for the first and second hairpins of the Spc5-12v2 promoter ("mutant 2") (SEQ ID NOs: 63 and 64, respectively).

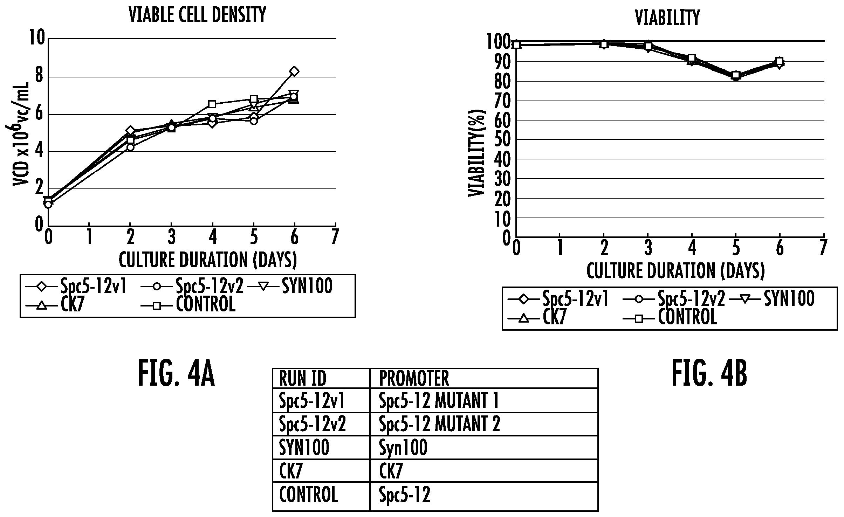

FIGs. 4A and 4B show the impact of the different promoters (Spc5-12v1, Spc5-12v2, syn100, and CK7) on the production process titers and quality, including viable cell density (FIG. 4A) and cell viability (FIG. 4B) for production of AAV virions having a microdystrophin transgene as compared to AAV virions having an Spc5-12 promoter operably linked to the microdystrophin transgene

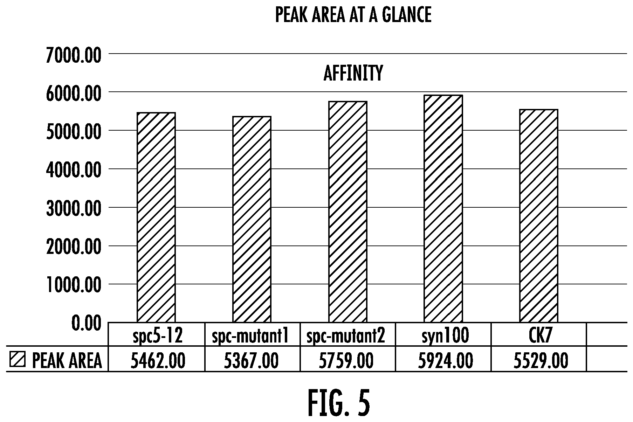

FIG. 5 shows the total capsids produced from constructs having the different promoters operably linked to the microdystrophin encoding sequence after an affinity column purification step.

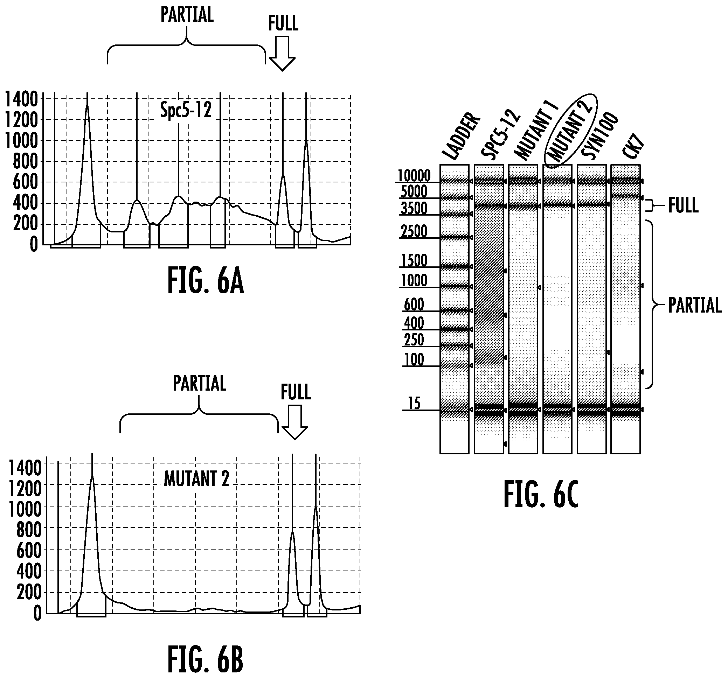

FIGs. 6A-6C show the results of a DNA TapeStation analysis (Agilent Technologies, Santa Clara, CA) for analyzing annealed double strand DNA. For example, a representative graph (FIG. 6A) provides a plot of sequence reads from analysis of vector particles packaging a genome containing Spc5-12 promoter, and a representative graph (FIG. 6B) provides a plot of sequence reads from analysis of vectors packaging a genome containing Spc5-12v2 promoter. The relative amounts of full genome vectors and partial genome vectors are represented by TapeStation analysis (FIG. 6C) for constructs having Spc5-12 promoter compared to the modified Spc5-12v1 and Spc5-12v2 were used to generate AAV particles.

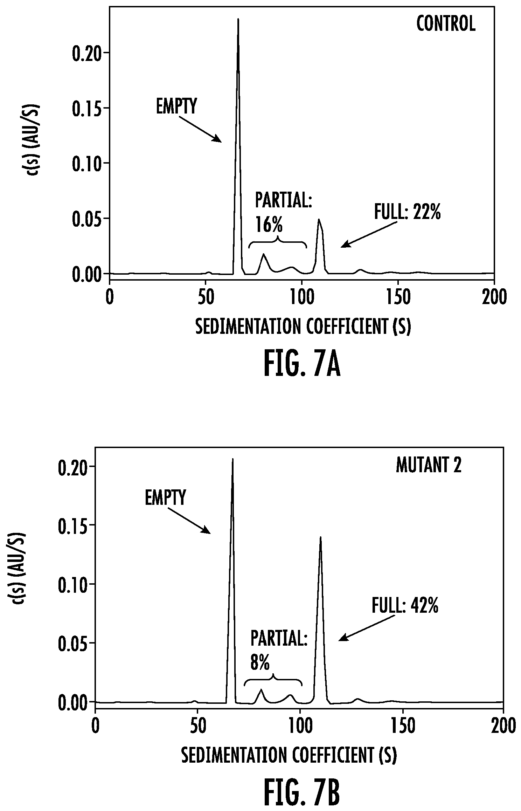

FIGs. 7A and 7B show an example of the sedimentation profile of ultracentrifugation separation of capsids generated from *cis* plasmids having the microdystrophin coding sequence operably linked to the control (Spc5-12, A) and the Spc5-12v2 promoter ("mutant 2", B), labeling peaks representing empty, partial and full capsids. The mutant promoter Spc5-12v2 resulted in a higher percentage of full genome vector compared to the control.

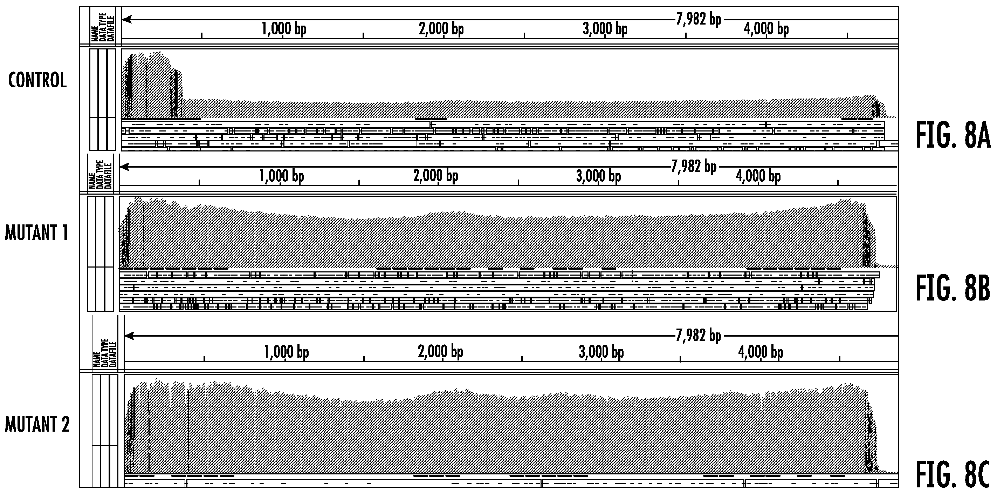

FIGs. 8A-8C show long read next generation sequencing (NGS) (by Oxford Nanopore method) of AAV virions generated from constructs in which the transgene is operably linked to the Spc5-12 promoter (FIG. 8A), Mutant 1 (Spc5-12v1) (FIG. 8B) and Mutant 2 (Spc5-12v2) (FIG. 8C) promoters.

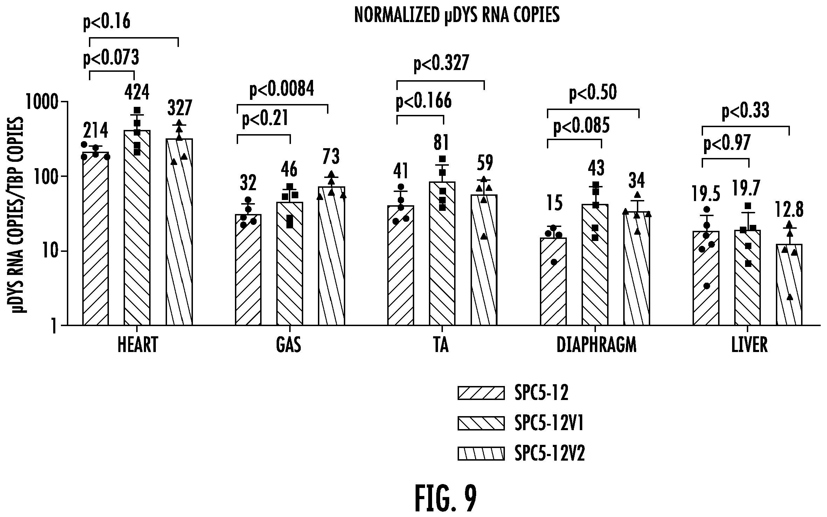

FIG. 9 shows microdystrophin RNA copies in different tissues (heart, gastrocnemius, tibialis anterior, diaphragm and liver) of mice administered recombinant AAV virions wherein the microdystrophin coding sequence is operably linked to control (Spc5-12), Spc5-12v1 or Spc5-12v2. Gas = gastrocnemius, TA = tibialis anterior.

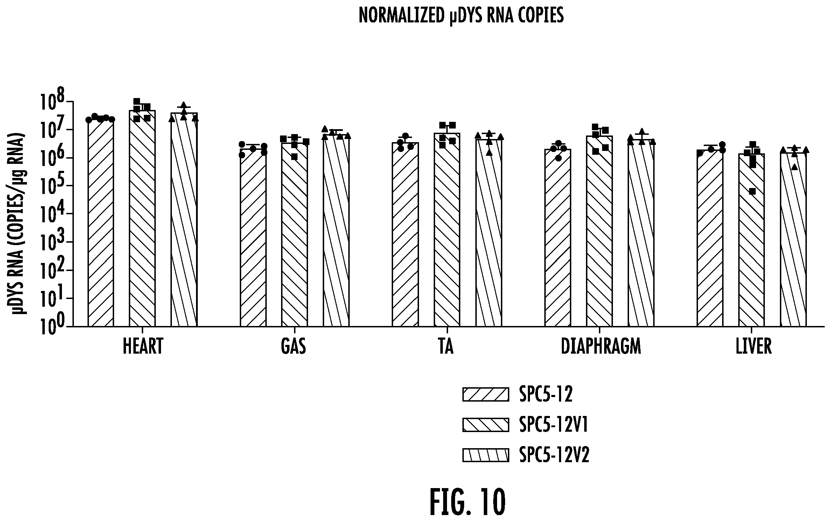

FIG. 10 shows an alternative representation of the normalized data (in copies/µg RNA) in heart, gastrocnemius, tibialis anterior, diaphragm and liver tissues from Fig. 9.

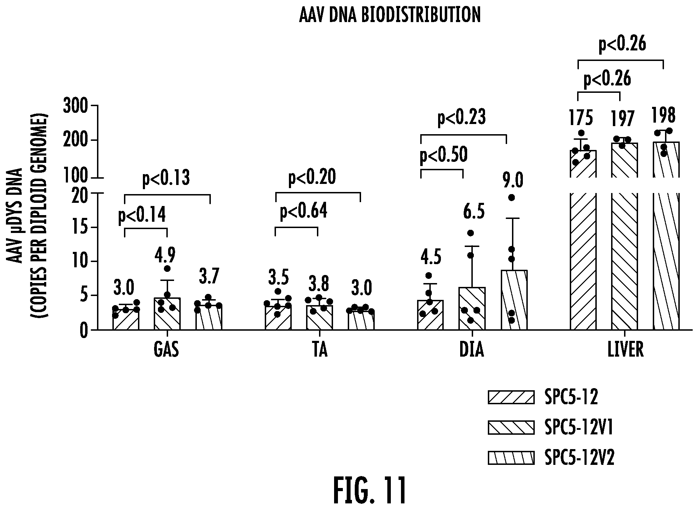

FIG. 11 shows AAV DNA biodistribution in tissues (gastrocnemius, tibialis anterior, diaphragm and liver) from mice administered recombinant AAV virions generated from constructs having a microdystrophin encoding transgene operably linked to indicated promoter sequences. Values normalized to diploid genomes. Gas = gastrocnemius, TA = tibialis anterior, DIA = diaphragm.

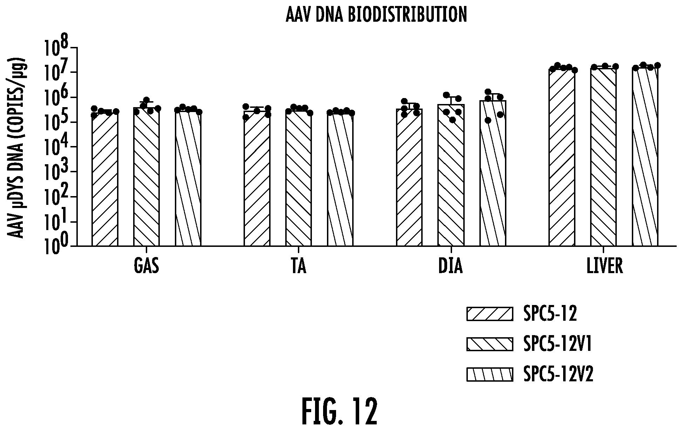

FIG. 12 shows AAV DNA biodistribution in mouse tissues (gastrocnemius, tibialis anterior, diaphragm and liver). Values normalized to micrograms DNA. Gas = gastrocnemius, TA = tibialis anterior, DIA = diaphragm.

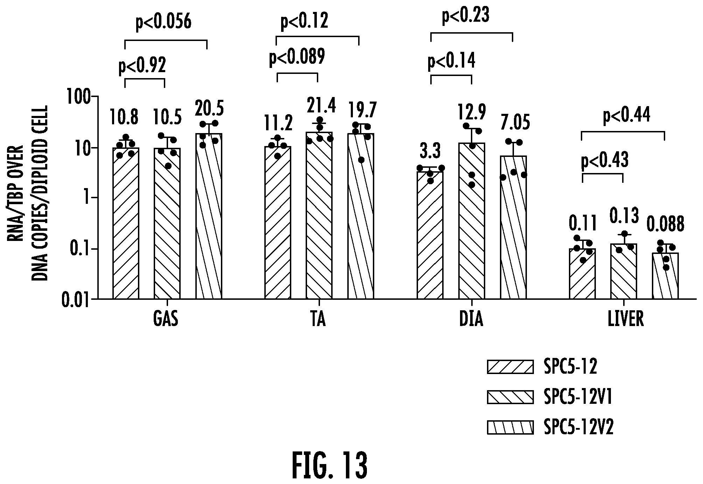

FIG. 13 shows the ratio of microdystrophin RNA to AAV DNA copies in a diploid cell in tissues (gastrocnemius, tibialis anterior, diaphragm and liver)of mice administered AAV virions generated from constructs having microdystrophin encoding transgene operably linked to the indicated promoter sequences. Gas = gastrocnemius, TA = tibialis anterior, DIA = diaphragm.

### DETAILED DESCRIPTION

The disclosed method and compositions may be understood more readily by reference to the following detailed description of particular embodiments and the Example included therein and to the Figures and their previous and following description.

The inventors have unexpectedly discovered that modifying regulatory elements to reduce the incidence of hairpin formation within muscle-specific promoter sequences can improve packaging of AAV vectors with full length AAV genomes and thus result in enhanced production and recovery of fully packaged viral (capsids) particles, which provides higher titers of the final product thus reducing overall cost of goods for the production of gene therapies for the treatment of muscle disorders.

It is to be understood that the disclosed method and compositions are not limited to specific synthetic methods, specific analytical techniques, or to particular reagents unless otherwise specified, and, as such, may vary. It is also to be understood that the terminology used herein is for the purpose of describing particular embodiments only and is not intended to be limiting.

### A. Definitions

It is understood that the disclosed method and compositions are not limited to the particular methodology, protocols, and reagents described as these may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "an", and "the" include plural reference unless the context clearly dictates otherwise. Thus, for example, reference to "a sequence" includes a plurality of such sequences, reference to "the promoter" is a reference to one or more promoters and equivalents thereof known to those skilled in the art, and so forth.

The term "regulatory element" or "nucleic acid regulatory element" are noncoding nucleic acid sequences that control the transcription of neighboring genes. Cis regulatory elements typically regulate gene transcription by binding to transcription factors. This includes "composite nucleic acid regulatory elements" comprising more than one enhancer or promoter elements as described herein. Also included are introns, polyadenylation sequences and other transcription enhancers.

The term "expression cassette" or "nucleic acid expression cassette" refers to nucleic acid molecules that include one or more transcriptional control elements including, but not limited to promoters, enhancers and/or regulatory elements, introns and polyadenylation sequences. The enhancers and promoters typically function to direct (trans)gene expression in one or more desired cell types, tissues or organs.

The term "operably linked" and "operably linked to" refers to nucleic acid sequences being linked and typically contiguous, or substantially contiguous, and, where necessary to join two protein coding regions, contiguous and in reading frame. However, since enhancers generally function when separated from the promoter by several kilobases and intronic sequences may be of variable lengths, some polynucleotide elements may be operably linked and still be functional while not directly contiguous with a downstream promoter and transgene.

The term "AAV" or "adeno-associated virus" refers to a Dependoparvovirus within the Parvoviridae genus of viruses. The AAV can be an AAV derived from a naturally occurring "wild-type" virus, an AAV derived from a rAAV genome packaged into a capsid comprising capsid proteins encoded by a naturally occurring cap gene and/or from a rAAV genome packaged into a capsid comprising capsid proteins encoded by a non-naturally occurring capsid cap gene. An example of the latter includes a rAAV having a capsid protein comprising a peptide insertion into or modification of the amino acid sequence of the naturally-occurring capsid.

The term "rAAV" refers to a "recombinant AAV." In some embodiments, a recombinant AAV has an AAV genome in which part or all of the rep and cap genes have been replaced with heterologous sequences.

The term "rep-cap helper plasmid" refers to a plasmid that provides the viral rep and cap gene function and aids the production of AAVs from rAAV genomes lacking functional rep and/or the cap gene sequences.

The term "cap gene" refers to the nucleic acid sequences that encode capsid proteins that form or help form the capsid coat of the virus. For AAV, the capsid protein may be VP1, VP2, or VP3.

The term "rep gene" refers to the nucleic acid sequences that encode the non-structural protein needed for replication and production of virus.

The terms "nucleic acids" and "nucleotide sequences" include DNA molecules (e.g., cDNA or genomic DNA), RNA molecules (e.g., mRNA), combinations of DNA and RNA molecules or hybrid DNA/RNA molecules, and analogs of DNA or RNA molecules. Such analogs can be generated using, for example, nucleotide analogs, which include, but are not limited to, inosine or tritylated bases. Such analogs can also comprise DNA or RNA molecules comprising modified backbones that lend beneficial attributes to the molecules such as, for example, nuclease resistance or an increased ability to cross cellular membranes. The nucleic acids or nucleotide sequences can be single-stranded, double-stranded, may contain both single-stranded and double-stranded portions, and may contain triple-stranded portions, but preferably is double-stranded DNA.

The terms "subject", "host", and "patient" are used interchangeably. As used herein, a subject is preferably a mammal such as a non-primate (e.g., cows, pigs, horses, cats, dogs, rats etc.) or a primate (e.g., monkey and human), most preferably a human.

The terms "therapeutic agent," "biotherapeutic agent," or "therapeutic," refer to any agent which can be used in treating, managing, or ameliorating symptoms associated with a disease or disorder, where the disease or disorder is associated with a function to be provided by a transgene. As used herein, a "therapeutically effective amount" refers to the amount of agent, (e.g., an amount of product expressed by the transgene) that provides at least one therapeutic benefit in the treatment or management of the target disease or disorder, when administered to a subject suffering therefrom. Further, a therapeutically effective amount with respect to an agent of the invention means that amount of agent alone, or when in combination with other therapies, that provides at least one therapeutic benefit in the treatment or management of the disease or disorder.

The phrase "muscle-specific" or "muscle-directed" refers to nucleic acid elements that have adapted their activity in muscle cells or tissue due to the interaction of such elements with the intracellular environment of the muscle cells. Muscle cells include skeletal muscle as well as cardiac muscle. Secretion of transgene product into the muscle, and/or bloodstream may also be enhanced following various routes of administration, such as intravenous or intramuscular administration, due to intramuscular expression where muscle-specific promoters are present. Various therapeutics benefit from muscle-specific expression of the transgene, or from both muscle-specific and liver-specific expression of the transgene (such as in combining the new synthetic muscle-specific promoter with a liver-specific promoter in a tandem promoter). Muscle production of a biotherapeutic agent (such as produced by the delivered transgene) may provide also provide the host with increased immunotolerance to the agent, as compared to direct injection of an equivalent protein agent to the host. Muscle specific or muscle directed activity may be assessed, for example, by administering to an animal model, such as a mouse or NHP, a recombinant AAV in which the muscle specific promoter is operably linked to a transgene, after a period of time sufficient for the transgene in the recombinant AAV to be expressed, harvesting muscle tissue and assaying for levels of transgene mRNA and/or protein product, including relative to the number of vector genomes in the muscle tissue.

A "variant" or "variant thereof" can mean a difference in some way from the reference sequence. For example, a variant can be different from the reference sequence due to a substitution, deletion or modification to a nucleic acid or amino acid. Where the variant includes a substitution of an amino acid residue or nucleic acid base, the substitution can be considered conservative or non-conservative. Conservative substitutions are those within the following groups: Ser, Thr, and Cys; Leu, Ile, and Val; Glu and Asp; Lys and Arg; Phe, Tyr, and Trp; and Gln, Asn, Glu, Asp, and His. Variants can include at least one substitution and/or at least one addition, there may also be at least one deletion. Variants can also include one or more non-naturally occurring residues. For example, they may include selenocysteine (e.g., seleno-L- cysteine) at any position, including in the place of cysteine. Many other "unnatural" amino acid substitutes are known in the art and are available from commercial sources. Examples of non-naturally occurring amino acids include D-amino acids, amino acid residues having an acetylaminomethyl group attached to a sulfur atom of a cysteine, a pegylated amino acid, and omega amino acids of the formula NH₂(CH₂)ₙCOOH wherein n is 2-6 neutral, nonpolar amino acids, such as sarcosine, t-butyl alanine, t-butyl glycine, N-methyl isoleucine, and norleucine. Phenylglycine may substitute for Trp, Tyr, or Phe; citrulline and methionine sulfoxide are neutral nonpolar, cysteic acid is acidic, and ornithine is basic. Proline may be substituted with hydroxyproline and retain the conformation conferring properties of proline.

### B. Modified Muscle-Specific Promoters and other Regulatory Elements

As detailed in the Examples, single stranded versions of the Spc5-12 promoter may form hairpin structures which, although not intended to be bound by any theory, may inhibit the production of full length AAV genomes in the production and packaging of recombinant AAV virions from cis plasmids encoding a transgene operably linked to the Spc5-12 promoter, resulting in an increase in the production of AAV vectors with fragmented genomes and non-transgene-related DNA contaminants, referred to as "partially-filled capsids" to be removed by purification processes. MEF-2, TEF and SRE elements are shown in FIGs. 1 and 2. Spc5-12 (having a nucleotide sequence of SEQ ID NO: 3) comprises a specific order to the MEF-2, TEF, and SRE elements. In some aspects, the promoter of SEQ ID NO:1 has the MEF-2 in stem1 and the SRE of stem2 switched in position (see Figure 1). In some aspects, the promoter of SEQ ID NO:2 has loop1 (MEF1) and stem1 (MEF2) missing (see Figure 2). Modification of the promoter sequence, including to change the positions of the MEF-2 and SRE elements (Mutant 1) or to reverse the orientations of the MEF-2 and SRE elements (Mutant 2), results in a new active promoter which reduces the formation of hairpin structures while maintaining the elements of the Spc5-12 promoter, and increases the production of full capsids containing the complete genome encoded by the *cis* plasmid while maintaining the muscle-specific promoter function. It was unexpected that such a modification to the promoter would result in an improved product by reducing the burden and cost of purification methods during the rAAV production process.

Accordingly, provided are regulatory elements comprising a modified Spc5-12 promoter having a nucleotide sequence of SEQ ID NO:1 (Spc5-12v1) or SEQ ID NO:2 (Spc5-12v2) or a composite nucleic acid sequence comprising a modified Spc5-12 promoter of SEQ ID NO:1 (Spc5-12v1) or SEQ ID NO:2 (Spc5-12v2), in combination with one or more other regulatory elements (e.g. enhancers). These modified promoters promote gene expression in muscle. The provided regulatory elements are included in *cis* plasmids in which the regulatory element is operably linked to the transgene for production of recombinant AAV virions.

Provided are nucleic acid regulatory elements for enhancing gene expression in the muscle comprising nucleic acid sequences SEQ ID NO:1 or SEQ ID NO:2. Also included are regulatory elements that enhance gene expression in the muscle which have 99%, 95%, 90%, 85% or 80% sequence identity with one of nucleic acid sequences SEQ ID NO:1 or SEQ ID NO:2, including wherein there is100% identity at nucleotides 121-129 and 197-209 to those positions of SEQ ID NO:1 or wherein there is 100% identity at nucleotides 113-131 and 191-212 to those positions of SEQ ID NO:2.

In some aspects, variants of SEQ ID NO: 1 are provided. In some aspects, the disclosed nucleic acids can comprise a nucleotide sequence having muscle specific promoter activity, at least 80% sequence identity to SEQ ID NO: 1, including with 100% sequence identity over nucleotides 121-129 and 197-209 of SEQ ID NO: 1, which generate a greater percentage of full capsids or yield of full capsids compared to a construct that is identical except for having the Spc5-12 promoter (SEQ ID NO:3). In some aspects, the disclosed nucleic acids can comprise a nucleotide sequence having muscle specific promoter activity, at least 85, 90, 95, 99 or 100% sequence identity to SEQ ID NO:1, including in some aspects with 100% sequence identity over nucleotides 121-129 and 197-209 of SEQ ID NO:1, which generate a greater percentage of full capsids or yield of full capsids compared to a comparable construct having the Spc5-12 promoter (SEQ ID NO:3) *(i.e.,* a construct identical except for the form of the Spc5-12 promoter). A variant of SEQ ID NO:1 can be the sequence of SEQ ID NO:1 having 1, 2, 3, 4, 5, 6, 7, 8, 9, 10, 15, 20, 25, 30, 35, 40, 45, 50, 55, 60, 65, or 70 nucleic acid substitutions. In some aspects, the variants have 100% sequence identity over nucleotides 121-129 and 197-209 of SEQ ID NO:1, have muscle specific promoter activity and generate a greater percentage of full capsids or yield of full capsids compared to a comparable construct identical except for having the Spc5-12 promoter (SEQ ID NO:3) *(i.e.,* a construct identical except for the form of the Spc5-12 promoter).

In some aspects, variants of SEQ ID NO:2 are provided. In some aspects, the disclosed nucleic acid can comprise a nucleotide sequence having muscle-specific promoter activity, at least 80% sequence identity to SEQ ID NO:2, including, in some aspects, 100% sequence identity over nucleotides 113-131 and 191-212 of SEQ ID NO:2, and which generate a greater percentage of full capsids or yield of full capsids compared to a construct identical except for having the Spc5-12 promoter (SEQ ID NO: 3). In some aspects, the disclosed nucleic acid can comprise a nucleotide sequence having muscle-specific promoter activity, at least 85, 90, 95, 99 or 100% sequence identity to SEQ ID NO:2, including, in some aspects,100% sequence identity over nucleotides 113-131 and 191-212 of SEQ ID NO:2, and which generate a greater percentage of full capsids or yield of full capsids compared to a construct that is identical except for having the Spc5-12 promoter (SEQ ID NO:3) *(i.e.,* a construct identical except for the form of the Spc5-12 promoter). A variant of SEQ ID NO:2 can be the sequence of SEQ ID NO:2 having 1, 2, 3, 4, 5, 6, 7, 8, 9, 10, 15, 20, 25, 30, 35, 40, 45, 50, 55, 60, 65, or 70 nucleic acid substitutions. In some aspects, the variants have 100% sequence identity over nucleotides 113-131 and 191-212 of SEQ ID NO:2 and retain muscle specific promoter activity and generate a greater percentage of full capsids or yield of full capsids compared to a construct having the Spc5-12 promoter (SEQ ID NO: 3) *(i.e.,* a construct identical except for the form of the Spc5-12 promoter).

Also provided are nucleic acid regulatory elements that are chimeric with respect to arrangements of elements in tandem in the expression cassette where at least one of the elements is a modified Spc5-12 promoter, including either Spc5-12v1 (SEQ ID NO:1) or Spc5-12v2 (SEQ ID NO: 2). Regulatory elements, in general, have multiple functions such as acting as recognition sites for transcription initiation or regulation, coordinating with cell-specific machinery to drive expression upon signaling, and enhancing expression of downstream gene(s).

In some aspects, disclosed are expression cassettes comprising regulatory elements designed to confer or enhance muscle-specific expression (including skeletal or cardiac muscle specific expression) comprising solely a promoter or the combination of a promoter (e.g. SEQ ID NO:1 or SEQ ID NO:2) and one or more other regulatory elements, such as enhancers.

In some aspects, the promoters of SEQ ID NO: 1 or SEQ ID NO:2 can be combined with, or operably linked to, one or more of the regulatory elements provided in PCT Application Publication No. WO 2021/021661. The combination of the promoters of SEQ ID NO:1 or SEQ ID NO:2 with one or more of the disclosed regulatory elements can result in a muscle-liver specific composite regulatory element.

Provided are arrangements of combinations of nucleic acid regulatory elements that promote transgene expression in muscle (skeletal and/or cardiac) tissue that include the modified Spc5-12 promoters, Spc5-12v1 (mutant 1) and Spc5-12v2 (mutant 2). Exemplary nucleotide sequences of the individual promoter and enhancer elements are provided in Table 1. In certain embodiments, the disclosed promoters have fewer repeat sequences prone to hairpin loop formations compared to the unmodified version of the same promoter. Muscle specific enhancers include the Mck Enhancer (MckE) and MhcE enhancers and tandem arrangements thereof arranged with a modified Spc5-12 promoter are described herein. Accordingly, provided are nucleic acid regulatory elements that comprise or consist of promoters and/or other nucleic acid elements, such as enhancers, that promote muscle expression such as one or more copies, for example two copies, of the MckE element, which may be arranged as two or more copies in tandem or an MckE and MhcE elements arranged in tandem arranged in combination with a modified Spc5-12 promoter (e.g., SEQ ID NO:1 or 2). These may be present as single copies or with two or more copies in tandem.

In other embodiments, provided are arrangements of combinations of nucleic acid regulatory elements that promote transgene expression in muscle (skeletal and/or cardiac) tissue. In particular, certain elements are arranged with two or more copies of the individual enhancer and promoter elements arranged in tandem and operably linked to a transgene to promote expression, particularly muscle specific expression. Exemplary nucleotide sequences of the individual promoter and enhancer elements are provided in Table 1. In certain embodiments the downstream promoter is a modified Spc5-12 (version 1 or version 2) promoter and the other promoter is a hAAT promoter or is a TBG promoter or vice versa.

Accordingly, with respect to expression in both the muscle and liver expression, provided are nucleic acid regulatory elements that comprise or consist of promoters and/or other nucleic acid elements, such as one or more elements that promote muscle specific expression (including skeletal and/or cardiac muscle), including a modified Spc5-12 promoter (SEQ ID NO:1 or SEQ ID NO:2), and may, for example, also include one or more copies, for example two copies, of the MckE element, which may be arranged as two or more copies in tandem or an MckE and MhcE elements arranged in tandemand liver specific regulatory elements such as ApoE enhancers, Mic/BiKE elements or hAAT promoters . These may be present as single copies or with two or more copies in tandem. In certain embodiments where there are 2 promoter elements (one of which is a modified Spc5-12 promoter), one promoter element is deleted for the initiation codon to prevent translation initiation at that site, and preferably, the promoter with the modified start codon is at the 3' end or the downstream end of the nucleic acid regulatory element. In certain embodiments, the composite nucleic acid regulatory element comprises a hAAT promoter, in embodiments a hAAT promoter which is start-codon modified (ΔATG) as the downstream promoter, and a second promoter in tandem with the hAAT promoter, which a modified Spc5-12 promoter (version 1 or version 2). In certain embodiments, the composite nucleic acid regulatory element comprises a modified Spc5-12 promoter (version 1 or version 2) which is start-codon modified (ΔATG) as the downstream promoter, and a second promoter in tandem with the modified Spc5-12 promoter, which a hAAT promoter. The nucleic acid regulatory element may also comprise, in addition to the modified Spc5-12 promoter sequences, one or more elements that promote liver specific expression, for example, such as ApoE enhancers, or Mic/BiKE elements. The recombinant expression cassettes provided herein comprise i) a nucleic acid regulatory element comprising a) one copy of ApoE, two or three copies of MckE arranged in tandem, one copy of each MckE, MhcE, and ApoE arrange in tandem, or two or three copies of MckE arranged in tandem with one copy of ApoE, b) a promoter comprising at least one copy of a modified Spc5-12 promoter, and ii) a transgene. In certain embodiments, the promoter is a tandem promoter with at least 2 promoter elements, one of which is a modified Spc5-12 promoter, which may be start-codon modified (ΔATG) if it is the downstream promoter and a second and upstream promoter is a CK8 promoter, a hAAT promoter or a TBG promoter.

The unique promoter sequences provided herein maintain tissue specificity and increase full capsid yield. The novel regulatory element nucleic acids were generated by modifying the tandem repeats and/or the likelihood of stem-loop-stem formation. This approach was employed to ameliorate potential genome instability that may interfere with rAAV vector replication. Ultimately, these designs improved the packaging efficiency to optimize the yield of full capsids containing an rAAV genome incorporating the regulatory elements operably linked to a nucleotide sequence encoding a therapeutic protein of interest.

In some aspects, rAAV vectors containing these mutant promoters have yield and/or efficiency of packaging full capsids with complete genomes, such as about 2-fold to 5-fold higher than comparable constructs (having the same transgene and other regulatory elements as well as ITR sequences) comprising the control Spc5-12 promoter. In some aspects, AAV constructs comprising the disclosed mutant promoters have an increased packaging efficiency as seen by a decrease in partial capsids relative to the number of full capsids as compared to AAV constructs comprising the Spc5-12 promoter.

**Table 1: Examples of regulatory elements.**

| SEQ ID NO: | Description | Sequence |
|---|---|---|
| 1 | Spc5-12 Version 1 ((Spc5-12 v1) mutant of Spc5-12) | GGCCGTCCGCCCTCGGCACCATCCTCACGACACCCAAATATGGCGACGG GTGAGGAATGGTGGGGAGTTATTTTTAGAGCGGTGAGGAAGGTGGGCAG GCAGCAGGTGTTGGCGCTCCATATTTGGCGGGAGTTATTTTTAGAGCGG AGGAATGGTGGACACCCAAATATGGCGACGGTTCCTCACCCGTCGCTAA AAATAACTCCGTGTCCGCCCTCGGCCGGGGCCGCATTCCTGGGGGCCGG GCGGTGCTCCCGCCCGCCTCGATAAAAGGCTCCGGGGCCGGCGGCGGCC CACGAGCTACCCGGAGGAGCGGGAGGCGCCAAGC |
| 2 | Spc5-12 Version 2 ((Spc5-12 v2) mutant of Spc5-12) | GGCCGTCCGCCCTCGGCACCATCCTCACGACACCCAAATATGGCGACGG GTGAGGAATGGTGGGGAGTTATTTTTAGAGCGGTGAGGAAGGTGGGCAG GCAGCAGGTGTTGGGGGAGTTATTTTTAGAGCGGGGAGTTATTTTTAGA GCGGAGGAATGGTGGACACCCAAATATGGCGACGGTTCCTCACGGACAC CCAAATATGGCGACGGGCCCTCGGCCGGGGCCGCATTCCTGGGGGCCGG GCGGTGCTCCCGCCCGCCTCGATAAAAGGCTCCGGGGCCGGCGGCGGCC CACGAGCTACCCGGAGGAGCGGGAGGCGCCAAGC |
| 3 | Spc5-12 promoter (Li, et al 1999) | GGCCGTCCGCCCTCGGCACCATCCTCACGACACCCAAATATGGCGACGG GTGAGGAATGGTGGGGAGTTATTTTTAGAGCGGTGAGGAAGGTGGGCAG GCAGCAGGTGTTGGCGCTCTAAAAATAACTCCCGGGAGTTATTTTTAGA GCGGAGGAATGGTGGACACCCAAATATGGCGACGGTTCCTCACCCGTCG CCATATTTGGGTGTCCGCCCTCGGCCGGGGCCGCATTCCTGGGGGCCGG GCGGTGCTCCCGCCCGCCTCGATAAAAGGCTCCGGGGCCGGCGGCGGCC CACGAGCTACCCGGAGGAGCGGGAGGCGCCAAGC |
| 4 | MEF-2 tandem sequence | CGCTCTAAAAAATAACTCCCGGGAGTTATTTTTAGAGCG |
| 5 | SRE/TEF-1 tandem sequence | GGACACCCAAATATGGCGACGGTTCCTCACCCGTCGCCATATTTGGGTG TCC |
| 6 | Mck Enhancer (MckE) | CCACTACGGGTTTAGGCTGCCCATGTAAGGAGGCAAGGCCTGGGGACAC CCGAGATGCCTGGTTATAATTAACCCAGACATGTGGCTGCCCCCCCCCC CCCCAACACCTGCTGCCTCTAAAAATAACCCTGTCCCTGGTGGATC |
| 7 | Tandem (2) Mck Enhancers (2 MckE) | CCACTACGGGTTTAGGCTGCCCATGTAAGGAGGCAAGGCCTGGGGACAC CCGAGATGCCTGGTTATAATTAACCCAGACATGTGGCTGCCCCCCCCCC CCCCAACACCTGCTGCCTCTAAAAATAACCCTGTCCCTGGTGGATCCCA CTACGGGTTTAGGCTGCCCATGTAAGGAGGCAAGGCCTGGGGACACCCG AGATGCCTGGTTATAATTAACCCAGACATGTGGCTGCCCCCCCCCCCCC |
| | | CAACACCTGCTGCCTCTAAAAATAACCCTGTCCCTGGTGGATC |
| 8 | Tandem Mck (3) | |
| | Enhancers (3 MckE) | CCACTACGGGTTTAGGCTGCCCATGTAAGGAGGCAAGGCCTGGGGACAC CCGAGATGCCTGGTTATAATTAACCCAGACATGTGGCTGCCCCCCCCCC CCCCAACACCTGCTGCCTCTAAAAATAACCCTGTCCCTGGTGGATCCCA CTACGGGTTTAGGCTGCCCATGTAAGGAGGCAAGGCCTGGGGACACCCG AGATGCCTGGTTATAATTAACCCAGACATGTGGCTGCCCCCCCCCCCCC CAACACCTGCTGCCTCTAAAAATAACCCTGTCCCTGGTGGATCCCACTA CGGGTTTAGGCTGCCCATGTAAGGAGGCAAGGCCTGGGGACACCCGAGA TGCCTGGTTATAATTAACCCAGACATGTGGCTGCCCCCCCCCCCCCCAA CACCTGCTGCCTCTAAAAATAACCCTGTCCCTGGTGGATC |
| 9 | Myosin heavy chain enhancer (MhcE) | CCCTTCAGATTAAAAATAACTGAGGTAAGGGCCTGGGTAGGGGAGGTGG TGTGAGACGCTCCTGTCTCTCCTCTATCTGCCCATCGGCCCTTTGGGGA GGAGGAATGTGCCCAAGGACTAAAAAAAGGCCATGGAGCCAGAGGGGCG AGGGCAACAGACCTTTCATGGGCAAACCTTGGGGCCCTGCTGAAGCTTT GGC |
| 44 | Alpha-Mic/Bik Enhancer (Mic/BikE) | AGGTTAATTTTTAAAAAGCAGTCAAAAGTCCAAGTGGCCCTTGGCAGCA TTTACTCTCTCTGTTTGCTCTGGTTAATAATCTCAGGAGCACAAACATT CC |
| 45 | Tandem (2) alpha-Mic/Bik Enhancers (2 Mic/BikE) | AGGTTAATTTTTAAAAAGCAGTCAAAAGTCCAAGTGGCCCTTGGCAGCA TTTACTCTCTCTGTTTGCTCTGGTTAATAATCTCAGGAGCACAAACATT CCAGGTTAATTTTTAAAAAGCAGTCAAAAGTCCAAGTGGCCCTTGGCAG CATTTACTCTCTCTGTTTGCTCTGGTTAATAATCTCAGGAGCACAAACA TTCC |
| 46 | ApoE Hepatic Control Region containing ApoE Enhancer | AGGCTCAGAGGCACACAGGAGTTTCTGGGCTCACCCTGCCCCCTTCCAA CCCCTCAGTTCCCATCCTCCAGCAGCTGTTTGTGTGCTGCCTCTGAAGT CCACACTGAACAAACTTCAGCCTACTCATGTCCCTAAAATGGGCAAACA TTGCAAGCAGCAAACAGCAAACACACAGCCCTCCCTGCCTGCTGACCTT GGAGCTGGGGCAGAGGTCAGAGACCTCTCTGGGCCCATGCCACCTCCAA CATCCACTCGACCCCTTGGAATTTCGGTGGAGAGGAGCAGAGGTTGTCC TGGCGTGGTTTAGGTAGTGTGAGAGGG |
| 47 | Tandem (2) ApoE Enhancers | AGGCTCAGAGGCACACAGGAGTTTCTGGGCTCACCCTGCCCCCTTCCAA CCCCTCAGTTCCCATCCTCCAGCAGCTGTTTGTGTGCTGCCTCTGAAGT CCACACTGAACAAACTTCAGCCTACTCATGTCCCTAAAATGGGCAAACA TTGCAAGCAGCAAACAGCAAACACACAGCCCTCCCTGCCTGCTGACCTT GGAGCTGGGGCAGAGGTCAGAGACCTCTCTGGGCCCATGCCACCTCCAA CATCCACTCGACCCCTTGGAATTTCGGTGGAGAGGAGCAGAGGTTGTCC TGGCGTGGTTTAGGTAGTGTGAGAGGGTCTAGAAGGCTCAGAGGCACAC AGGAGTTTCTGGGCTCACCCTGCCCCCTTCCAACCCCTCAGTTCCCATC CTCCAGCAGCTGTTTGTGTGCTGCCTCTGAAGTCCACACTGAACAAACT TCAGCCTACTCATGTCCCTAAAATGGGCAAACATTGCAAGCAGCAAACA GCAAACACACAGCCCTCCCTGCCTGCTGACCTTGGAGCTGGGGCAGAGG TCAGAGACCTCTCTG |
| 48 | TBG Promoter | GGGCTGGAAGCTACCTTTGACATCATTTCCTCTGCGAATGCATGTATAATTTCT ACAGAACCTATTAGAAAGGATCACCCAGCCTCTGCTTTTGTACAACTTTCCCTT AAAAAACTGCCAATTCCACTGCTGTTTGGCCCAATAGTGAGAACTTTTTCCTGC TGCCTCTTGGTGCTTTTGCCTATGGCCCCTATTCTGCCTGCTGAAGACACTCTT GCCAGCATGGACTTAAACCCCTCCAGCTCTGACAATCCTCTTTCTCTTTTGTTT TACATGAAGGGTCTGGCAGCCAAAGCAATCACTCAAAGTTCAAACCTTATCATT TTTTGCTTTGTTCCTCTTGGCCTTGGTTTTGTACATCAGCTTTGAAAATACCAT CCCAGGGTTAATGCTGGGGTTAATTTATAACTAAGAGTGCTCTAGTTTTGCAAT ACAGGACATGCTATAAAAATGGAAAGAT |
| 49 | hAAT Promoter | GATCTTGCTACCAGTGGAACAGCCACTAAGGATTCTGCAGTGAGAGCAGAGGGC |
| | | CAGCTAAGTGGTACTCTCCCAGAGACTGTCTGACTCACGCCACCCCCTCCACCT TGGACACAGGACGCTGTGGTTTCTGAGCCAGGTACAATGACTCCTTTCGGTAAG TGCAGTGGAAGCTGTACACTGCCCAGGCAAAGCGTCCGGGCAGCGTAGGCGGGC GACTCAGATCCCAGCCAGTGGACTTAGCCCCTGTTTGCTCCTCCGATAACTGGG GTGACCTTGGTTAATATTCACCAGCAGCCTCCCCCGTTGCCCCTCTGGATCCAC TGCTTAAATACGGACGAGGACAGGGCCCTGTCTCCTCAGCTTCAGGCACCACCA CTGACCTGGGACAGT |
| 50 | hAAT(ΔAT G) Promoter | GATCTTGCTACCAGTGGAACAGCCACTAAGGATTCTGCAGTGAGAGCAGAGGGC CAGCTAAGTGGTACTCTCCCAGAGACTGTCTGACTCACGCCACCCCCTCCACCT TGGACACAGGACGCTGTGGTTTCTGAGCCAGGTACAGTGACTCCTTTCGGTAAG TGCAGTGGAAGCTGTACACTGCCCAGGCAAAGCGTCCGGGCAGCGTAGGCGGGC GACTCAGATCCCAGCCAGTGGACTTAGCCCCTGTTTGCTCCTCCGATAACTGGG GTGACCTTGGTTAATATTCACCAGCAGCCTCCCCCGTTGCCCCTCTGGATCCAC TGCTTAAATACGGACGAGGACAGGGCCCTGTCTCCTCAGCTTCAGGCACCACCA CTGACCTGGGACAGT |
| 51 | ApoE.hAAT | AGGCTCAGAGGCACACAGGAGTTTCTGGGCTCACCCTGCCCCCTTCCAACCCCT CAGTTCCCATCCTCCAGCAGCTGTTTGTGTGCTGCCTCTGAAGTCCACACTGAA CAAACTTCAGCCTACTCATGTCCCTAAAATGGGCAAACATTGCAAGCAGCAAAC AGCAAACACACAGCCCTCCCTGCCTGCTGACCTTGGAGCTGGGGCAGAGGTCAG AGACCTCTCTGGGCCCATGCCACCTCCAACATCCACTCGACCCCTTGGAATTTC GGTGGAGAGGAGCAGAGGTTGTCCTGGCGTGGTTTAGGTAGTGTGAGAGGGGTA CCCGGGGATCTTGCTACCAGTGGAACAGCCACTAAGGATTCTGCAGTGAGAGCA GAGGGCCAGCTAAGTGGTACTCTCCCAGAGACTGTCTGACTCACGCCACCCCCT CCACCTTGGACACAGGACGCTGTGGTTTCTGAGCCAGGTACAATGACTCCTTTC GGTAAGTGCAGTGGAAGCTGTACACTGCCCAGGCAAAGCGTCCGGGCAGCGTAG GCGGGCGACTCAGATCCCAGCCAGTGGACTTAGCCCCTGTTTGCTCCTCCGATA ACTGGGGTGACCTTGGTTAATATTCACCAGCAGCCTCCCCCGTTGCCCCTCTGG ATCCACTGCTTAAATACGGACGAGGACAGGGCCCTGTCTCCTCAGCTTCAGGCA CCACCACTGACCTGGGACAGT |
| 52 | CK8 Promoter | CCCTGCATGCGAAGATCTTCGAACAAGGCTGTGGGGGACTGAGGGCAGGCTGTA ACAGGCTTGGGGGCCAGGGCTTATACGTGCCTGGGACTCCCAAAGTATTACTGT TCCATGTTCCCGGCGAAGGGCCAGCTGTCCCCCGCCAGCTAGACTCAGCACTTA GTTTAGGAACCAGTGAGCAAGTCAGCCCTTGGGGCAGCCCATACAAGGCCATGG GGCTGGGCAAGCTGCACGCCTGGGTCCGGGGTGGGCACGGTGCCCGGGCAACGA GCTGAAAGCTCATCTGCTCTCAGGGGCCCCTCCCTGGGGACAGCCCCTCCTGGC TAGTCACACCCTGTAGGCTCCTCTATATAACCCAGGGGCACAGGGGCTGCCCTC ATTCTACCACCACCTCCACAGCACAGACAGACACTCAGGAGCCAGCCAGCGTCG A |
| 53 | MCK7 | CTAGAAGCTGCATGTCTAAGCTAGACCCTTCAGATTAAAAATAACTGAG GTAAGGGCCTGGGTAGGGGAGGTGGTGTGAGACGCTCCTGTCTCTCCTC TATCTGCCCATCGGCCCTTTGGGGAGGAGGAATGTGCCCAAGGACTAAA AAAAGGCCATGGAGCCAGAGGGGCGAGGGCAACAGACCTTTCATGGGCA AACCTTGGGGCCCTGCTGTCTAGCATGCCCCACTACGGGTCTAGGCTGC CCATGTAAGGAGGCAAGGCCTGGGGACACCCGAGATGCCTGGTTATAAT TAACCCAGACATGTGGCTGCCCCCCCCCCCCCAACACCTGCTGCCTCTA AAAATAACCCTGTCCCTGGTGGATCCCCTGCATGCGAAGATCTTCGAAC AAGGCTGTGGGGGACTGAGGGCAGGCTGTAACAGGCTTGGGGGCCAGGG CTTATACGTGCCTGGGACTCCCAAAGTATTACTGTTCCATGTTCCCGGC GAAGGGCCAGCTGTCCCCCGCCAGCTAGACTCAGCACTTAGTTTAGGAA CCAGTGAGCAAGTCAGCCCTTGGGGCAGCCCATACAAGGCCATGGGGCT GGGCAAGCTGCACGCCTGGGTCCGGGGTGGGCACGGTGCCCGGGCAACG AGCTGAAAGCTCATCTGCTCTCAGGGGCCCCTCCCTGGGGACAGCCCCT CCTGGCTAGTCACACCCTGTAGGCTCCTCTATATAACCCAGGGGCACAG GGGCTGCCCTCATTCTACCACCACCTCCACAGCAC |
| 54 | Syn100 promoter | CGGCCGTCCGCCCTCGGCACCATTCCTCACGACACCGAAATATGGCGAC GGGTGAGGAATGGTGGGGAGTTATTTTTAGAGCGGTGAGGAATGGTGGG CAGGCAGCAGGTGTTGGGGGAGTTATTTTTAGAGCGGGGAGTTATTTTT |
| | | AGAGCGGTGAGGAATGGTGGACACCGAAATATGGCGACGGGTGAGGAAT GGTGCCGTCGCCATATTTGGGTGTCCCGTCCGCCCTCGGCCGGGGCCGC ATTCCTGGGGGCCGGGCGGTGCTCCCGCCCGCCTCGATAAAAGGCTCCG GGGCCGGCGGCGGCCCACGAGCTACCCGGAGGAGCGGGAGGCGTCTCTG CCAGCGGTCCGACGCGCAGTCAGCACCAGGTAGGTGGGCACCGCGCCGT GCCGTGCC |
| 55 | CK7 promoter | CCACTACGGGTTTAGGCTGCCCATGTAAGGAGGCAAGGCCTGGGGACAC CCGAGATGCCTGGTTATAATTAACCCAGACATGTGGCTGCCCCCCCCCC CCCCAACACCTGCTGCCTCTAAAAATAACCCTGTCCCTGGTGGATCCCC TGCATGCGAAGATCTTCGAACAAGGCTGTGGGGGACTGAGGGCAGGCTG TAACAGGCTTGGGGGCCAGGGCTTATACGTGCCTGGGACTCCCAAAGTA TTACTGTTCCATGTTCCCGGCGAAGGGCCAGCTGTCCCCCGCCAGCTAG ACTCAGCACTTAGTTTAGGAACCAGTGAGCAAGTCAGCCCTTGGGGCAG CCCATACAAGGCCATGGGGCTGGGCAAGCTGCACGCCTGGGTCCGGGGT GGGCACGGTGCCCGGGCAACGAGCTGAAAGCTCATCTGCTCTCAGGGGC CCCTCCCTGGGGACAGCCCCTCCTGGCTAGTCACACCCTGTAGGCTCCT CTATATAACCCAGGGGCACAGGGGCTGCCCTCATTCTACCACCACCTCC ACAGCACAGACAGACACTCAGGAGCCAGCCAGCGTCGA |
| 61 | Spc5-12v1 Stem1-Stem1' | CGCTCCATATTTGGCGGGAGTTATTTTTAGAGCG |
| 62 | Spc5-12v1 Stem2-Loop2-Stem2' | GGACACCCAAATATGGCGACGGTTCCTCACCCGTCGCTAAAAATAACTC CGTGTCC |
| 63 | Spc5-12v2 Stem1'-Stem1' | GGGAGTTATTTTTAGAGCGGGGAGTTATTTTTAGAGCG |
| 64 | Spc5-12v2 Stem2-Loop2-Stem2 | GGACACCCAAATATGGCGACGGTTCCTCACGGACACCCAAATATGGCGA CGG |
| 65 | Spc5-12 1^{st} Hairpin | CGCTCTAAAAATAACTCCCGGGAGTTATTTTTAGAGCG |
| 66 | Spc5-12 2^{nd} Hairpin | GGACACCCAAATATGGCGACGGTTCCTCACCCGTCGGCATATTTGGGTG TCG |

In some embodiments, the disclosed regulatory elements can be a part of the recombinant expression cassettes provided herein, examples of which are provided in Table 6.

The recombinant expression cassettes provided herein comprise a nucleic acid regulatory element operably linked to a transgene, wherein the regulatory element comprises a promoter comprising the nucleic acid sequence of SEQ ID NO:1 or SEQ ID NO:2 or variants thereof. The transgene may be any one of the genes or nucleic acids encoding the therapeutic proteins listed in, but not limited to, Tables 7A-7B, including microdystrophin which is made up of domains of dystrophin that are listed in Table 4. Several examples of microdystrophin proteins that may be incorporated (encoded for) as transgenes are listed in Table 5. In certain embodiments, the transgene is a therapeutic antibody, including a full length antibody or antigen binding fragment thereof, such as, a Fab fragment. In embodiments, the transgenes encode therapeutic proteins for treatment, amelioration or prevention of a muscle disease. In embodiments, the transgene encodes a microdystrophin. In embodiments, the transgene encodes Dys 1 (having an amino acid sequence of SEQ ID NO:35), Dys3 (having an amino acid sequence of SEQ ID NO: 36), Dys5 (having an amino acid sequence of SEQ ID NO: 37), MD1 (having an amino acid sequence of SEQ ID NO: 56), human microdystrophin (having an amino acid sequence of SEQ ID NO: 57), Dys3978 (having an amino acid sequence of SEQ ID NO: 58), MD3 (having an amino acid sequence of SEQ ID NO: 59), MD4 (having an amino acid sequence of SEQ ID NO: 60), or MD5 (having an amino acid sequence of SEQ ID NO: 72). In embodiments, the microdystrophin transgene is encoded by a nucleotide sequence comprising SEQ ID NO: 67, SEQ ID NO: 68, SEQ ID NO: 69, SEQ ID NO: 70 or SEQ ID NO: 71, or a nucleotide sequence at least about 90%, at least about 91%, at least about 92%, at least about 93%, at least about 94%, at least about 95%, at least about 96%, at least about 97%, at least about 98% or at least about 99% identical to SEQ ID NO: 67, SEQ ID NO: 68, SEQ ID NO: 69, SEQ ID NO: 70 or SEQ ID NO: 71. Accordingly, with respect to muscle specific expression, provided are nucleic acid regulatory elements that comprise or consist of promoters and/or other nucleic acid elements, such as enhancers that promote muscle expression, such as MckE enhancers. These may be present as single copies or with two or more copies in tandem.

### 1. Enhancers

In some aspects, the disclosed regulatory elements can comprise, in addition to the modified, muscle specific promoter, one or more enhancers. Specifically, muscle-specific enhancers can be used in combination with one or more of the muscle-specific promoters disclosed herein.

Accordingly, provided are muscle-specific enhancer (MckE) nucleic acid elements as a single copy, or two or three copies arranged in tandem (SEQ ID NOs: 6, 7 and 8, respectively, in Table 1) from the mouse muscle creatine kinase gene (Jaynes, J.B., Johnson, J.E., Buskin, J.N., Gartside, C.L., and Hauschka, S.D that can be arranged in tandem with the modified Spc5-12 promoter. The muscle creatine kinase gene is regulated by multiple upstream elements, including a muscle-specific enhancer. Mol. Cell. Biol., 8: 62-70, 1988; and GenBank Accn. No. AF188002.1). The 206-bp fragment from this region acts as a skeletal muscle enhancer and confers orientation-dependent activity in myocardiocytes. A 110-bp enhancer subfragment of this sequence confers high-level expression in skeletal myocytes but is inactive in myocardiocytes (Amacher, et al. 1993 Molecular and Cellular Biology 13(5):2753-64).

Also provided are Myosin heavy chain enhancer (MhcE) nucleic acids (SEQ ID NO: 9, in Table 1) placed in tandem with additional regulatory elements, including a modified Spc5-12 promoter element. Myosin is the most abundant protein in muscle, which is the most abundant tissue in the body. Enhancement of muscle production of transgene, including skeletal and cardiac muscle expression, would greatly benefit the biotherapeutic effect of many transgenes.

Other enhancers are well known to the skilled person in the art.

### 2. Introns

Another aspect of the present invention relates to nucleic acid expression cassettes comprising an intron within the regulatory cassette. In some embodiments, the intron nucleic acid is a chimeric intron derived from human β-globin and Ig heavy chain (also known as β-globin splice donor/immunoglobulin heavy chain splice acceptor intron, or β-globin/IgG chimeric intron, Reed, R., et al. Genes and Development, 1989). Use of an intron may further induce efficient splicing in eukaryotic cells. Although use of an intron may not indicate increases in expression to an already strong promoter, the presence of an intron may increase the expression level of transgene and can also increase the duration of expression in vivo.

In some embodiments, the intron is a VH4 intron. The VH4 intron nucleic acid can comprise SEQ ID NO: 11 as shown in Table 2 below. The VH4 intron 5' of the coding sequence may enhance proper splicing and, thus, transgene expression. Accordingly, in some embodiments, an intron is coupled to the 5' end of a transgene sequence, including positioned between the coding sequence and the promoter sequence. In other embodiments, the intron is less than 100 nucleotides in length.

**Table 2: Nucleotide sequences for different introns**

| Structure | SEQ ID | Sequence |
|---|---|---|
| Chimeric intron (β-globin/Ig Intron) | 10 | GTAAGTATCAAGGTTACAAGACAGGTTTAAGGAGACCAATAGAA ACTGGGCTTGTCGAGACAGAGAAGACTCTTGCGTTTCTGATAGG CACCTATTGGTCTTACTGACATCCACTTTGCCTTTCTCTCCACA G |
| VH4 intron | 11 | GTGAGTATCTCAGGGATCCAGACATGGGGATATGGGAGGTGCCT CTGATCCCAGGGCTCACTGTGGGTCTCTCTGTTCACAG |
| SV40 intron | 12 | GTAAGTTTAGTCTTTTTGTCTTTTATTTCAGGTCCCGGATCCGG TGGTGGTGCAAATCAAAGAACTGCTCCTCAGTGGATGTTGCCTT TACTTCTAG |

In other embodiments, the intron is a chimeric intron derived from human β-globin and Ig heavy chain (also known as β-globin splice donor/immunoglobulin heavy chain splice acceptor intron, or β-globin/IgG chimeric intron) (Table 2, SEQ ID NO: 10). Other introns well known to the skilled person may be employed, such as the chicken β-actin intron, minute virus of mice (MVM) intron, human factor IX intron (e.g., FIX truncated intron 1), β-globin splice donor/immunoglobulin heavy chain splice acceptor intron, adenovirus splice donor /immunoglobulin splice acceptor intron, and/or SV40 late splice donor /splice acceptor (19S/16S) intron (Table 2, SEQ ID NO: 12).

Other introns well known to the skilled person may be employed.

### 3. Other regulatory elements

### i. polyA

Another aspect of the present disclosure relates to expression cassettes comprising a polyadenylation (polyA) site downstream of the coding region of the transgene. Any polyA site that signals termination of transcription and directs the synthesis of a polyA tail is suitable for use in AAV vectors of the present disclosure. Exemplary polyA signals are derived from, but not limited to, the following: the SV40 late gene, the rabbit β-globin gene (SEQ ID NO:14), the bovine growth hormone (BPH) gene, the human growth hormone (hGH) gene, the synthetic polyA (SPA) site, and the bovine growth hormone (bGH) gene. *See, e.g.,* Powell and Rivera-Soto, 2015, Discov. Med., 19(102):49-57. In one embodiment, the polyA signal comprises SEQ ID NO: 14 as shown in **Table 3.**

**Table 3: Nucleotide Sequence of the PolyA Signal**

| Structure | SEQ ID NO: | Sequence |
|---|---|---|
| β-globin PolyA signal | 13 | ATAAAGGAAATTTATTTTCATTGCAATAGTGTGTTGGAATTTTTTGT GTCTCTCA |
| Rabbit β-globin polyA | 14 | GATCTTTTTCCCTCTGCCAAAAATTATGGGGACATCATGAAGCCCCT TGAGCATCTGACTTCTGGCTAATAAAGGAAATTTATTTTCATTGCAA TAGTGTGTTGGAATTTTTTGTGTCTCTCACTCG |

### C. Recombinant Expression Cassettes

Disclosed are recombinant expression cassettes comprising a regulatory element comprising a modified Spc5-12 promoter disclosed herein operably linked to a transgene. In some aspects, the regulatory element can be any one or more of the regulatory elements disclosed herein. For example, the nucleic acid regulatory element comprises one of the promoters of Table 1, for example a promoter having a nucleotide sequence of SEQ ID NO:1 or SEQ ID NO:2. Thus, in some aspects, disclosed are recombinant expression cassettes comprising a transgene operably linked to a promoter comprising the nucleotide sequence of SEQ ID NO:1 or SEQ ID NO:2.

In some embodiments, the expression cassette can comprise one or more of the disclosed nucleic acid regulatory elements operably linked to one or more of the disclosed transgenes. For example, the transgene can be any one of the genes or nucleic acids encoding the therapeutic proteins listed in, but not limited to, Tables 7A-7B. Thus, in some aspects, the transgene can encode a therapeutic antibody, including a full length antibody or an antigen binding fragment, such as a Fab fragment.

In some aspects, the disclosed recombinant expression cassettes can comprise one or more of the disclosed nucleic acid regulatory elements operably linked to a transgene, wherein the transgene is a muscle-specific disease therapeutic. For example, a muscle-specific disease therapeutic can be, but is not limited to, a microdystrophin (see also Table 5), growth and development factor 8 (GDF-8) inhibitor, SMN, huFollistatin344, hSGCA, human-alpha-sarcoglycan, GALGT2, hSGCB, or ravulizumab.

Encoded by the transgenes provided herein for the methods of the invention are microdystrophins that consist of dystrophin domains arranged amino-terminus to the carboxy terminus: ABD-H1-R1-R2-R3-H3-R24-H4-CR-CT, wherein ABD is an actin-binding domain of dystrophin, H1 is a hinge 1 region of dystrophin, R1 is a spectrin 1 region of dystrophin, R2 is a spectrin 2 region of dystrophin, R3 is a spectrin 3 region of dystrophin, H3 is a hinge 3 region of dystrophin, R24 is a spectrin 24 region of dystrophin, H4 is a hinge 4 region of dystrophin, CR is a cysteine-rich region of dystrophin and CT is the C terminal domain (and comprises at least the portion of the CT domain containing the α1-syntrophin binding site, including SEQ ID NO:33). Table 4 below has the amino acid sequences for these components, in particular from the full length human DMD protein (UniProtDB-11532).

To overcome the packaging limitation that is typical of AAV vectors, many of the microdystrophin genes developed for clinical use are lacking the CT domain. Several researchers have indicated that the DAPC does not even require the C-terminal domain in order to assemble or that the C-terminus is non-essential [Crawford, et al., J Cell Biol, 2000, 150(6): 1399-1409; and Ramos, J.N, et al. Molecular Therapy 2019, 27(3):1-13]. However, overexpression of a microdystrophin gene containing helix 1 of the coiled-coil motif of the CT domain in skeletal muscle of *mdx* mice increased the recruitment α1-syntrophin and α-dystrobrevin, which are members of DAP complex, serving as modular adaptors for signaling proteins recruited to the sarcolemma membrane [Koo, T., et al., Delivery of AAV2/9-microdystrophin genes incorporating helix 1 of the coiled-coil motif in the C-terminal domain of dystrophin improves muscle pathology and restores the level of α1-syntrophin and α-dystrobrevin in skeletal muscles of mdx mice. Hum Gene Ther, 2011. 22(11): p. 1379-88]. Overexpression of the longer version of microdystrophin also improved the muscle resistance to lengthening contraction-induced muscle damage in the mdx mice as compared with the shorter version [Koo, T., et al. 2011, *supra*]. The CT domain assists in the formation of the Dystrophin Associated Protein Complex (DAPC).

The CT domain of dystrophin contains two polypeptide stretches that are predicted to form α-helical coiled coils similar to those in the rod domain (see H1 indicated by single underlining and H2 indicated by double underlining in SEQ ID NO:29 in Table 4 below). Each coiled coil has a conserved repeating heptad (a,b,c,d,e,f,g)ₙ similar to those found in leucine zippers where leucine predominates at the "d" position. This domain has been named the CC (coiled coil) domain. The CC region of dystrophin forms the binding site for dystrobrevin and may modulate the interaction between α1-syntrophin and other dystrophin-associated proteins.

Both syntrophin isoforms, α1-syntrophin and β1-syntrophin are thought to interact directly with dystrophin through more than one binding site in dystrophin exons 73 and 74 (Yang et al, JBC 270(10):4975-8 (1995)). α1- and β1-syntrophin bind separately to the dystrophin C-terminal domain, and the binding site for α1-syntrophin reportedly resides at least within the amino acid residues 3447 to 3481, while that for β1-syntrophin has been reported to reside within the amino acid residues 3495 to 3535 (as numbered in the DMD protein of UniProtDB-11532 (SEQ ID NO:34), see also Table 4, SEQ ID NO:29, italic). Alpha1- (α1-) syntrophin and alpha-syntrophin are used interchangeably throughout.

Microdystrophins disclosed herein were found to bind to and recruit nNOS, as well as alpha-syntrophin, alpha-dystrobrevin and beta-dystroglycan. Binding to nNOS, in the context of a microdystrophin including a C-terminal domain of dystrophin binding to nNOS, means that the microdystrophin expressed in muscle tissue was determined by immunostaining with appropriate antibodies to identify each of alpha-syntrophin, alpha-dystrobrevin, and nNOS in or near the sarcolemma in a section of the transduced muscle tissue. In certain embodiments, the microdystrophin protein has a C-terminal domain that "increases binding" to α1-syntrophin, β-syntrophin and/or dystrobrevin compared to a comparable microdystrophin that does not contain the C-terminal domain (but has the same amino acid sequence otherwise, that is a "reference microdystrophin protein"). Increased binding of microdystrophin to α1-syntrophin, β-syntrophin and/or dystrobrevin means that the DAPC is stabilized or anchored to the sarcolemma, to a greater extent than a reference microdystrophin protein , as determined by greater levels of one or more DAPC components (including α1-syntrophin, β-syntrophin, α-dystrobrevin, β-dystroglycan or nNOS) in the muscle membrane as measured by immunostaining of muscle sections or in muscle tissue lysates or muscle membrane preparations as measured by western blot analysis.

In some embodiments, the microdystrophin including a C-terminal domain of dystrophin comprises an α1-syntrophin binding site and/or a dystrobrevin binding site in the C-terminal domain. In some embodiments, the C-terminal domain comprising an α1-syntrophin binding site is a truncated C-terminal domain. The α1-syntrophin binding site functions in part to recruit and anchor nNOS to the sarcolemma through α1-syntrophin.

The microdystrophins described herein can comprise all or a portion of the CT domain comprising the Helix 1 of the coiled-coil motif. The C Terminal sequence may be defined by the coding sequence of the exons of the *DMD* gene, in particular exons 70 to 74, and a portion of exon 75 (in particular, the nucleotide sequence encoding the first 36 amino acids of the amino acid sequence encoded by exon 75, or by the sequence of the human DMD protein, for example, the sequence of UniProtKB-P11532 (SEQ ID NO:34) (the CT is amino acids 3361 to 3554 of the UniProtKB-P11532 sequence), or comprising or consisting of binding sites for dystrobrevin and/or α1-syntrophin (indicated in Table 4, SEQ ID NO:29). In certain embodiments, the CT domain consists or comprises the 194 C-terminal amino acids of the DMD protein, for example, residues 3361 to 3554 of the amino acid sequence of UniProtKB-P11532 (SEQ ID NO:34), the amino acids encoded by exons 70 to 74, and the nucleotide sequence encoding the first 36 nucleotides of the nucleotide sequence of exon 75 of the *DMD* gene, or the amino acid sequence of SEQ ID NO:29 (see Table 4). For example, DYS1 (SEQ ID NO: 35) has the 194 amino acid CT sequence of SEQ ID NO:29. In other embodiments, the amino acid sequence of the C-terminal domain is truncated and comprises at least the binding sites for dystrobrevin and/or α1-syntrophin. In certain embodiments, the truncated C-terminal domain comprises the amino acid sequence MENSNGSYLNDSISPNESIDDEHLLIQHYCQSLNQ (α1-syntrophin binding site) (SEQ ID NO:33). In certain embodiments, the truncated C-terminal domain comprises an α1-syntrophin binding site, wherein the binding site has amino acid sequence MENSNGSYLNDSISPNESIDDEHLLIQHYCQSLNQ (SEQ ID NO:33). In particular embodiments, the CT domain sequence has the amino acid sequence of SEQ ID NO:30 or amino acids 3361 to 3500 of the UniProtKB-P11532 human DMD sequence. For example, RGX-DYS5 has a CT domain having the amino acid sequence of SEQ ID NO:30. In alternative embodiments, the microdystrophin lacks a CT domain, and may have the domains arranged as follows: ABD1-L1-H1-L2-R1-R2-L3-R3-H3-L4-R24-H4-CR, for example RGX-DYS3 (SEQ ID NO:36).

The NH₂ terminus and a region in the rod domain of dystrophin bind directly to but do not cross-link cytoskeletal actin. The rod domain of wild type dystrophin is composed of 24 repeating units that are similar to the triple helical repeats of spectrin. This repeating unit accounts for the majority of the dystrophin protein and is thought to give the molecule a flexible rod-like structure similar to β-spectrin. These α-helical coiled-coil repeats are interrupted by four proline-rich hinge regions. At the end of the 24th repeat is the fourth hinge region that is immediately followed by the WW domain [Blake, D. et al, Function and Genetics of Dystrophin and Dystrophin-Related Proteins in Muscle. Physiol. Rev. 82: 291-329, 2002]. Microdystrophins disclosed herein do not include R4 to R23, and only include 3 of the 4 hinge regions or portions thereof. In some embodiments, no new amino acid residues or linkers are introduced into the microdystrophin.

In some embodiments, microdystrophin comprises H3 *(e.g.,* SEQ ID NOS: 35, 36, or 37). In embodiments, H3 can be a full endogenous H3 domain from N-terminal to C-terminal, e.g., SEQ ID NO:23. Stated another way, some microdystrophin embodiments do not contain a fragment of the H3 domain but contain the entire H3 domain. In some embodiments, the C-terminal amino acid of the R3 domain is coupled directly (or covalently bonded to) the N-terminal amino acid of the H3 domain. In some embodiments, the C-terminal amino acid of the R3 domain coupled to the N-terminal amino acid of the H3 domain is Q. In some embodiments, the 5' amino acid of the H3 domain coupled to the R3 domain is Q.

Without being bound by any one theory, a full hinge domain may be appropriate in any microdystrophin in order to convey full activity upon the derived microdystrophin protein. Hinge segments of dystrophin have been recognized as being proline-rich in nature and may therefore confer flexibility to the protein product (Koenig and Kunkel, 265(6):4560-4566, 1990). Any deletion of a portion of the hinge, especially removal of one or more proline residues, may reduce its flexibility and therefore reduce its efficacy by hindering its interaction with other proteins in the DAP complex.

Microdystrophins disclosed herein comprise the wild-type dystrophin H4 sequence (which contains the WW domain) to and including the CR domain (which contains the ZZ domain, represented by a single underline (UniProtKB-P11532 aa 3307-3354) in SEQ ID NO:27). The WW domain is a protein-binding module found in several signaling and regulatory molecules. The WW domain binds to proline-rich substrates in an analogous manner to the src homology-3 (SH3) domain. This region mediates the interaction between β-dystroglycan and dystrophin, since the cytoplasmic domain of β-dystroglycan is proline rich. The WW domain is in the Hinge 4 (H4 region). The CR domain contains two EF-hand motifs that are similar to those in α-actinin and that could bind intracellular Ca²⁺. The ZZ domain contains a number of conserved cysteine residues that are predicted to form the coordination sites for divalent metal cations such as Zn²⁺. The ZZ domain is similar to many types of zinc finger and is found both in nuclear and cytoplasmic proteins. The ZZ domain of dystrophin binds to calmodulin in a Ca²⁺-dependent manner. Thus, the ZZ domain may represent a functional calmodulin-binding site and may have implications for calmodulin binding to other dystrophin-related proteins.

Microdystrophin embodiments can further comprise linkers (L1, L2, L3, L4, L4.1 and/or L4.2) or portions thereof connected the domains as shown as follows: ABD1-L1-H1-L2-R1-R2-L3-R3-H3-L4-R24-H4-CR-CT (e.g., SEQ ID NOs: 35, 37, or 91) or ABD1-L1-H1-L2-R1-R2-L3-R3-H3-L4-R24-H4-CR (e.g., SEQ ID NO: 36) L1 can be an endogenous linker L1 (e.g., SEQ ID NO: 16) that can couple ABD1 to H1. L2 can be an endogenous linker L2 (e.g., SEQ ID NO: 18) that can couple H1 to R1. L3 can be an endogenous linker L3 (e.g., SEQ ID NO:21) that can couple R2 to R3.

L4 can also be an endogenous linker that can couple H3 and R24. In some embodiments, L4 is 3 amino acids, *e.g.* TLE (SEQ ID NO:24) that precede R24 in the native dystrophin sequence. In other embodiments, L4 can be the 4 amino acids that precede R24 in the native dystrophin sequence (SEQ ID NO:31) or the 2 amino acids that precede R24 (SEQ ID NO:32). In other embodiments, there is no linker, L4 or otherwise, in between H3 and R24. On the 5' end of H3, as mentioned above, no linker is present, but rather R3 is directly coupled to H3, or alternatively H2.

The above described components of microdystrophin other domains not specifically described can have the amino acid sequences as provided in Table 4 below. The amino acid sequences for the domains provided herein correspond to the dystrophin isoform of UniProtKB-P1 1532 (DMD_HUMAN) (SEQ ID NO 34). Other embodiments can comprise the domains from naturally-occurring functional dystrophin isoforms known in the art, such as UniProtKB-A0A075B6G3 (A0A075B6G3_HUMAN), wherein, for example, R24 has an R substituted for the Q at amino acid 3 of SEQ ID NO:25.

Additional embodiments are disclosed in International Application PCT/US2020/062484, filed November 27, 2020.

**Table 4: Microdystrophin segment amino acid sequences**

| Structure | SEQ ID | Sequence |
|---|---|---|
| ABD1 | 15 | MLWWEEVEDCYEREDVQKKTFTKWVNAQFSKFGKQHIENLFSDLQD GRRLLDLLEGLTGQKLPKEKGSTRVHALNNVNKALRVLQNNNVDLV NIGSTDIVDGNHKLTLGLIWNIILHWQVKNVMKNIMAGLQQTNSEK ILLSWVRQSTRNYPQVNVINFTTSWSDGLALNALIHSHRPDLFDWN SVVCQQSATQRLEHAFNIARYQLGIEKLLDPEDVDTTYPDKKSILM YITSLFQVLP |
| L1 | 16 | QQVSIEAIQEVE |
| H1 | 17 | MLPRPPKVTKEEIIFQLIIIQMIIYSQQITVSLAQGYERTSSPKPRFKS YAYTQAAYVTTSDPTRSPFPSQHLEAPED |
| L2 | 18 | KSFGSSLME |
| R1 | 19 | SEVNLDRYQTALEEVLSWLLSAEDTLQAQGEISNDVEVVKDQFHTH EGYMMDLTAHQGRVGNILQLGSKLIGTGKLSEDEETEVQEQMNLLN SRWECLRVASMEKQSNLHR |
| R2 | 20 | SEVNLDRYQTALEEVLSWLLSAEDTLQAQGEISNDVEVVKDQFHTH EGYMMDLTAHQGRVGNILQLGSKLIGTGKLSEDEETEVQEQMNLLN SRWECLRVASMEKQSNLHR VLMDLQNQKLKELNDWLTKTEERTRKMEEEPLGPDLEDLKRQVQQH KVLQEDLEQEQVRVNSLTHMVVVVDESSGDHATAALEEQLKVLGDR WANICRWTEDRWVLLQD |
| L3 | 21 | IL |
| R3 | 22 | LKWQRLTEEQCLFSAWLSEKEDAVNKIHTTGFKDQNEMLSSLQKLA VLKADLEKKKQSMGKLYSLKQDLLSTLKNKSVTQKTEAWLDNFARC WDNLVQKLEKSTAQISQ |
| H3 | 23 | LKWQRLTEEQCLFSAWLSEKEDAVNKIHTTGFKDQNEMLSSLQKLA VLKADLEKKKQSMGKLYSLKQDLLSTLKNKSVTQKTEAWLDNFARC WDNLVQKLEKSTAQISQ QPDLAPGLTTIGASPTQTVTLVTQPVVTKETAISKLEMPSSLMLEV P |
| L4 | 24 | TLE |
| R24 | 25 | RLQELQEATDELDLKLRQAEVIKGSWQPVGDLLIDSLQDHLEKVKA LRGEIAPLKENVSHVNDLARQLTTLGIQLSPYNLSTLEDLNTRWKL LQVAVEDRVRQLHE AHRDFGPASQHFLSTSVQGPWERAISPNKVPYYINHETQTTCWDHP KMTELYQSLADLNNVRFSAYRTAMKL |
| H4 | 26 | AHRDFGPASQHFLSTSVQGPWERAISPNKVPYYINHETQTTCWDHP KMTELYQSLADLNNVRFSAYRTAMKL WW domain is represented by a single underline (UniProtKB- P11532 aa 3055-3088) |
| Cysteine-rich domain (CR) | 27 | RRLQKALCLDLLSLSAACDALDQHNLKQNDQPMDILQIINCLTTIY DRLEQEHNNLVNVPLCVDMCLNWLLNVYDTGRTGRIRVLSFKTGII SLCKAHLEDKYRYLFKQVASSTGFCDQRRLGLLLHDSIQIPRQLGE VASFGGSNIEPSVRSCFQFANNKPEIEAALFLDWMRLEPQSMVWLP VLHRVAAAETAKHQAKCNICKECPIIGFRYRSLKHFNYDICQSCFF SGRVAKGHKMHYPMVEYC ZZ domain is represented by a single underline (UniProtKB-P11532 aa 3307-3354) |
| CR short | 28 | AKHQAKCNICKECPIIGFRYRSLKHFNYDICQSCFFSGRVAKGHKM HYPMVEYC |
| C-terminal Domain (CT) | 29 | TPTTSGEDVRDFAKVLKNKFRTKRYFAKHPRMGYLPVQTVLEGDNM ETPVTLINFWPVDSAPASSPQLSHDDTHSRIEHYASRLAEMENSNG SYLNDSISPNESIDDEHLLIQHYCQSLNQDSPLSQPRSPAQILISL ESEERGELERILADLEEENRNLQAEYDRLKQQHEHKGLSPLPSPPE MMPTSPQSPR Coiled-coil motif H1 is represented by a single underline; motif H2 is represented by a double underline; dystrobrevin-binding side is in italics. |
| Minimal/truncate d C-terminal Domain (CT1.5) | 30 | TPTTSGEDVRDFAKVLKNKFRTKRYFAKHPRMGYLPVQTVLEGDNM ETPVTLINFWPVDSAPASSPQLSHDDTHSRIEHYASRLAEMENSNG SYLNDSISPNESIDDEHLLIQHYCQSLNQDSPLSQPRSPAQILISL ES α1-syntrophin-binding site is in italics. |
| L4 | 31 | ETLE |
| L4 | 32 | LE |
| Minimal alpha-syntrophin binding site | 33 | MENSNGSYLNDSISPNESIDDEHLLIQHYCQSLNQ |
| Human dystrophin (UniProtKB-P11532) | 34 | MLWWEEVEDC YEREDVQKKT FTKWVNAQFS KFGKQHIENL FSDLQDGRRL LDLLEGLTGQ KLPKEKGSTR VHALNNVNKA LRVLQNNNVD LVNIGSTDIV DGNHKLTLGL IWNIILHWQV KNVMKNIMAG LQQTNSEKIL LSWVRQSTRN YPQVNVINFT TSWSDGLALN ALIHSHRPDL FDWNSVVCQQ SATQRLEHAF NIARYQLGIE KLLDPEDVDT TYPDKKSILM YITSLFQVLP QQVSIEAIQE VEMLPRPPKV TKEEHFQLHH QMHYSQQITV SLAQGYERTS SPKPRFKSYA YTQAAYVTTS DPTRSPFPSQ HLEAPEDKSF GSSLMESEVN LDRYQTALEE VLSWLLSAED TLQAQGEISN DVEVVKDQFH THEGYMMDLT AHQGRVGNIL QLGSKLIGTG KLSEDEETEV QEQMNLLNSR WECLRVASME KQSNLHRVLM DLQNQKLKEL NDWLTKTEER TRKMEEEPLG PDLEDLKRQV QQHKVLQEDL EQEQVRVNSL THMVVVVDES SGDHATAALE EQLKVLGDRW ANICRWTEDR WVLLQDILLK WQRLTEEQCL FSAWLSEKED AVNKIHTTGF KDQNEMLSSL QKLAVLKADL EKKKQSMGKL YSLKQDLLST LKNKSVTQKT EAWLDNFARC WDNLVQKLEK STAQISQAVT TTQPSLTQTT VMETVTTVTT REQILVKHAQ EELPPPPPQK KRQITVDSEI RKRLDVDITE LHSWITRSEA VLQSPEFAIF RKEGNFSDLK EKVNAIEREK AEKFRKLQDA SRSAQALVEQ MVNEGVNADS IKQASEQLNS RWIEFCQLLS ERLNWLEYQN NIIAFYNQLQ QLEQMTTTAE NWLKIQPTTP SEPTAIKSQL KICKDEVNRL SDLQPQIERL KIQSIALKEK GQGPMFLDAD FVAFTNHFKQ VFSDVQAREK ELQTIFDTLP PMRYQETMSA IRTWVQQSET KLSIPQLSVT DYEIMEQRLG ELQALQSSLQ EQQSGLYYLS TTVKEMSKKA PSEISRKYQS EFEEIEGRWK KLSSQLVEHC |
| | | QKLEEQMNKL RKIQNHIQTL KKWMAEVDVF LKEEWPALGD SEILKKQLKQ CRLLVSDIQT IQPSLNSVNE GGQKIKNEAE PEFASRLETE LKELNTQWDH MCQQVYARKE ALKGGLEKTV SLQKDLSEMH EWMTQAEEEY LERDFEYKTP DELQKAVEEM KRAKEEAQQK EAKVKLLTES VNSVIAQAPP VAQEALKKEL ETLTTNYQWL CTRLNGKCKT LEEVWACWHE LLSYLEKANK WLNEVEFKLK TTENIPGGAE EISEVLDSLE NLMRHSEDNP NQIRILAQTL TDGGVMDELI NEELETFNSR WRELHEEAVR RQKLLEQSIQ SAQETEKSLH LIQESLTFID KQLAAYIADK VDAAQMPQEA QKIQSDLTSH EISLEEMKKH NQGKEAAQRV LSQIDVAQKK LQDVSMKFRL FQKPANFEQR LQESKMILDE VKMHLPALET KSVEQEVVQS QLNHCVNLYK SLSEVKSEVE MVIKTGRQIV QKKQTENPKE LDERVTALKL HYNELGAKVT ERKQQLEKCL KLSRKMRKEM NVLTEWLAAT DMELTKRSAV EGMPSNLDSE VAWGKATQKE IEKQKVHLKS ITEVGEALKT VLGKKETLVE DKLSLLNSNW IAVTSRAEEW LNLLLEYQKH METFDQNVDH ITKWIIQADT LLDESEKKKP QQKEDVLKRL KAELNDIRPK VDSTRDQAAN LMANRGDHCR KLVEPQISEL NHRFAAISHR IKTGKASIPL KELEQFNSDI QKLLEPLEAE IQQGVNLKEE DFNKDMNEDN EGTVKELLQR GDNLQQRITD ERKREEIKIK QQLLQTKHNA LKDLRSQRRK KALEISHQWY QYKRQADDLL KCLDDIEKKL ASLPEPRDER KIKEIDRELQ KKKEELNAVR RQAEGLSEDG AAMAVEPTQI QLSKRWREIE SKFAQFRRLN FAQIHTVREE TMMVMTEDMP LEISYVPSTY LTEITHVSQA LLEVEQLLNA PDLCAKDFED LFKQEESLKN IKDSLQQSSG RIDIIHSKKT AALQSATPVE RVKLQEALSQ LDFQWEKVNK MYKDRQGRFD RSVEKWRRFH YDIKIFNQWL TEAEQFLRKT QIPENWEHAK YKWYLKELQD GIGQRQTVVR TLNATGEEII QQSSKTDASI LQEKLGSLNL RWQEVCKQLS DRKKRLEEQK NILSEFQRDL NEFVLWLEEA DNIASIPLEP GKEQQLKEKL EQVKLLVEEL PLRQGILKQL NETGGPVLVS APISPEEQDK LENKLKQTNL QWIKVSRALP EKQGEIEAQI KDLGQLEKKL EDLEEQLNHL LLWLSPIRNQ LEIYNQPNQE GPFDVKETEI AVQAKQPDVE EILSKGQHLY KEKPATQPVK RKLEDLSSEW KAVNRLLQEL RAKQPDLAPG LTTIGASPTQ TVTLVTQPVV TKETAISKLE MPSSLMLEVP ALADFNRAWT ELTDWLSLLD QVIKSQRVMV GDLEDINEMI IKQKATMQDL EQRRPQLEEL ITAAQNLKNK TSNQEARTII TDRIERIQNQ WDEVQEHLQN RRQQLNEMLK DSTQWLEAKE EAEQVLGQAR AKLESWKEGP YTVDAIQKKI TETKQLAKDL RQWQTNVDVA NDLALKLLRD YSADDTRKVH MITENINASW RSIHKRVSER EAALEETHRL LQQFPLDLEK FLAWLTEAET TANVLQDATR KERLLEDSKG VKELMKQWQD LQGEIEAHTD VYHNLDENSQ KILRSLEGSD DAVLLQRRLD NMNFKWSELR KKSLNIRSHL EASSDQWKRL HLSLQELLVW LQLKDDELSR QAPIGGDFPA VQKQNDVHRA FKRELKTKEP VIMSTLETVR IFLTEQPLEG LEKLYQEPRE LPPEERAQNV TRLLRKQAEE VNTEWEKLNL HSADWQRKID ETLERLQELQ EATDELDLKL RQAEVIKGSW QPVGDLLIDS LQDHLEKVKA LRGEIAPLKE NVSHVNDLAR QLTTLGIQLS PYNLSTLEDL NTRWKLLQVA VEDRVRQLHE AHRDFGPASQ HFLSTSVQGP WERAISPNKV PYYINHETQT TCWDHPKMTE LYQSLADLNN VRFSAYRTAM KLRRLQKALC LDLLSLSAAC DALDQHNLKQ NDQPMDILQI INCLTTIYDR |
| | | LEQEHNNLVN VPLCVDMCLN WLLNVYDTGR TGRIRVLSFK TGIISLCKAH LEDKYRYLFK QVASSTGFCD QRRLGLLLHD SIQIPRQLGE VASFGGSNIE PSVRSCFQFA NNKPEIEAAL FLDWMRLEPQ SMVWLPVLHR VAAAETAKHQ AKCNICKECP IIGFRYRSLK HFNYDICQSC FFSGRVAKGH KMHYPMVEYC TPTTSGEDVR DFAKVLKNKF RTKRYFAKHP RMGYLPVQTV LEGDNMETPV TLINFWPVDS APASSPQLSH DDTHSRIEHY ASRLAEMENS NGSYLNDSIS PNESIDDEHL LIQHYCQSLN QDSPLSQPRS PAQILISLES EERGELERIL ADLEEENRNL QAEYDRLKQQ HEHKGLSPLP SPPEMMPTSP QSPRDAELIA EAKLLRQHKG RLEARMQILE DHNKQLESQL HRLRQLLEQP QAEAKVNGTT VSSPSTSLQR SDSSQPMLLR VVGSQTSDSM GEEDLLSPPQ DTSTGLEEVM EQLNNSFPSS RGRNTPGKPM REDTM |

The present disclosure also contemplates variants of these sequences so long as the function of each domain and linker is substantially maintained and/or the therapeutic efficacy of microdystrophin comprising such variants is substantially maintained. Functional activity includes (1) binding to one of, a combination of, or all of actin, β-dystroglycan, α1-syntrophin, α-dystrobrevin, and nNOS; (2) improved muscle function in an animal model (for example, in the *mdx* mouse model described herein) or in human subjects; and/or (3) cardioprotective or improvement in cardiac muscle function in animal models or human patients. In particular, microdystrophin can comprise ABD consisting of SEQ ID NO: 15 or an amino acid sequence with at least 80%, at least 85%, at least 90%, at least 95%, at least 96%, at least 97%, at least 98%, or at least 99% sequence identity to SEQ ID NO:15; H1 consisting of SEQ ID NO: 17 or an amino acid sequence with at least 80%, at least 85%, at least 90%, at least 95%, at least 96%, at least 97%, at least 98%, or at least 99% sequence identity to SEQ ID NO: 17; R1 consisting of SEQ ID NO: 19 or an amino acid sequence with at least 80%, at least 85%, at least 90%, at least 95%, at least 96%, at least 97%, at least 98%, or at least 99% sequence identity to SEQ ID NO: 19; R2 consisting of SEQ ID NO:8 or an amino acid sequence with at least 80%, at least 85%, at least 90%, at least 95%, at least 96%, at least 97%, at least 98%, or at least 99% sequence identity to SEQ ID NO:20; H3 consisting of SEQ ID NO:11 or an amino acid sequence with at least 80%, at least 85%, at least 90%, at least 95%, at least 96%, at least 97%, at least 98%, or at least 99% sequence identity to SEQ ID NO:23; R24 consisting of SEQ ID NO:25 or an amino acid sequence with at least 80%, at least 85%, at least 90%, at least 95%, at least 96%, at least 97%, at least 98%, or at least 99% sequence identity to SEQ ID NO:25; H4 consisting of SEQ ID NO:26 or an amino acid sequence with at least 80%, at least 85%, at least 90%, at least 95%, at least 96%, at least 97%, at least 98%, or at least 99% sequence identity to SEQ ID NO:26; CR consisting of SEQ ID NOs:27 or 28 or an amino acid sequence with at least 80%, at least 85%, at least 90%, at least 95%, at least 96%, at least 97%, at least 98%, or at least 99% sequence identity to SEQ ID NO:27 or 28; CT consisting of SEQ ID NOs:29 or 30 or an amino acid sequence with at least 80%, at least 85%, at least 90%, at least 95%, at least 96%, at least 97%, at least 98%, or at least 99% sequence identity to SEQ ID NOs:29 or 30, or CT comprising SEQ ID NO:33. In addition to the foregoing, microdystrophin can comprise linkers in the locations described above that comprise or consist of sequences as follows: L1 consisting of SEQ ID NO:16 or an amino acid sequence with at least 80%, at least 85%, at least 90%, at least 95%, at least 98%, or at least 99% sequence identity to SEQ ID NO:16; L2 consisting of SEQ ID NO:18 or an amino acid sequence with at least 80%, at least 85%, at least 90%, at least 95%, at least 98%, or at least 99% sequence identity to SEQ ID NO:18; L3 consisting of SEQ ID NO:21 or an amino acid sequence with at least 50% identity to SEQ ID NO:21 or a variant with conservative substitutions for both L3 residues; and L4 consisting of SEQ ID NOs:24, 31, or 32 or an amino acid sequence with at least 50%, at least 75% sequence identity to SEQ ID NOs:24, 31, or 32.

Table 5 includes a representative list of microdystrophin proteins (by their amino acid sequences). It is also contemplated that other embodiments are provided, for example substituted variants of microdystrophin, such as defined by SEQ ID NOs:35 (RGX-DYS1), 36 (RGX-DYS3), or 37 (RGX-DYS5). For example, conservative substitutions can be made to SEQ ID NOs:35, 36, or 37 and substantially maintain its functional activity. In embodiments, microdystrophin may have at least 60%, at least 70%, at least 80%, at least 85%, at least 90%, at least 95%, at least 96%, at least 97%, at least 98%, or at least 99% sequence identity to the amino acid sequence of SEQ ID NOs:35, 36, or 37 and maintain functional microdystrophin activity.

**Table 5: Amino acid sequences of microdystrophin proteins**

| Structure | SEQ ID NO: | Amino Acid Sequence |
|---|---|---|
| DYS1 | 35 | MLWWEEVEDCYEREDVQKKTFTKWVNAQFSKFGKQHIENLFSDLQDGR RLLDLLEGLTGQKLPKEKGSTRVHALNNVNKALRVLQNNNVDLVNIGS TDIVDGNHKLTLGLIWNIILHWQVKNVMKNIMAGLQQTNSEKILLSWV RQSTRNYPQVNVINFTTSWSDGLALNALIHSHRPDLFDWNSVVCQQSA TQRLEHAFNIARYQLGIEKLLDPEDVDTTYPDKKSILMYITSLFQVLP QQVSIEAIQEVEMLPRPPKVTKEEHFQLHHQMHYSQQITVSLAQGYER TSSPKPRFKSYAYTQAAYVTTSDPTRSPFPSQHLEAPEDKSFGSSLME SEVNLDRYQTALEEVLSWLLSAEDTLQAQGEISNDVEVVKDQFHTHEG YMMDLTAHQGRVGNILQLGSKLIGTGKLSEDEETEVQEQMNLLNSRWE CLRVASMEKQSNLHRVLMDLQNQKLKELNDWLTKTEERTRKMEEEPLG |
| | | PDLEDLKRQVQQHKVLQEDLEQEQVRVNSLTHMVVVVDESSGDHATAA LEEQLKVLGDRWANICRWTEDRWVLLQDILLKWQRLTEEQCLFSAWLS EKEDAVNKIHTTGFKDQNEMLSSLQKLAVLKADLEKKKQSMGKLYSLK QDLLSTLKNKSVTQKTEAWLDNFARCWDNLVQKLEKSTAQISQQPDLA PGLTTIGASPTQTVTLVTQPVVTKETAISKLEMPSSLMLEVPTLERLQ ELQEATDELDLKLRQAEVIKGSWQPVGDLLIDSLQDHLEKVKALRGEI APLKENVSHVNDLARQLTTLGIQLSPYNLSTLEDLNTRWKLLQVAVED RVRQLHEAHRDFGPASQHFLSTSVQGPWERAISPNKVPYYINHETQTT CWDHPKMTELYQSLADLNNVRFSAYRTAMKLRRLQKALCLDLLSLSAA CDALDQHNLKQNDQPMDILQIINCLTTIYDRLEQEHNNLVNVPLCVDM CLNWLLNVYDTGRTGRIRVLSFKTGIISLCKAHLEDKYRYLFKQVASS TGFCDQRRLGLLLHDSIQIPRQLGEVASFGGSNIEPSVRSCFQFANNK PEIEAALFLDWMRLEPQSMVWLPVLHRVAAAETAKHQAKCNICKECPI IGFRYRSLKHFNYDICQSCFFSGRVAKGHKMHYPMVEYCTPTTSGEDV RDFAKVLKNKFRTKRYFAKHPRMGYLPVQTVLEGDNMETPVTLINFWP VDSAPASSPQLSHDDTHSRIEHYASRLAEMENSNGSYLNDSISPNESI DDEHLLIQHYCQSLNQDSPLSQPRSPAQILISLESEERGELERILADL EEENRNLQAEYDRLKQQHEHKGLSPLPSPPEMMPTSPQSPR |
| DYS3 | 36 | MLWWEEVEDCYEREDVQKKTFTKWVNAQFSKFGKQHIENLFSDLQDGR RLLDLLEGLTGQKLPKEKGSTRVHALNNVNKALRVLQNNNVDLVNIGS TDIVDGNHKLTLGLIWNIILHWQVKNVMKNIMAGLQQTNSEKILLSWV RQSTRNYPQVNVINFTTSWSDGLALNALIHSHRPDLFDWNSVVCQQSA TQRLEHAFNIARYQLGIEKLLDPEDVDTTYPDKKSILMYITSLFQVLP QQVSIEAIQEVEMLPRPPKVTKEEHFQLHHQMHYSQQITVSLAQGYER TSSPKPRFKSYAYTQAAYVTTSDPTRSPFPSQHLEAPEDKSFGSSLME SEVNLDRYQTALEEVLSWLLSAEDTLQAQGEISNDVEVVKDQFHTHEG YMMDLTAHQGRVGNILQLGSKLIGTGKLSEDEETEVQEQMNLLNSRWE CLRVASMEKQSNLHRVLMDLQNQKLKELNDWLTKTEERTRKMEEEPLG PDLEDLKRQVQQHKVLQEDLEQEQVRVNSLTHMVVVVDESSGDHATAA LEEQLKVLGDRWANICRWTEDRWVLLQDILLKWQRLTEEQCLFSAWLS EKEDAVNKIHTTGFKDQNEMLSSLQKLAVLKADLEKKKQSMGKLYSLK QDLLSTLKNKSVTQKTEAWLDNFARCWDNLVQKLEKSTAQISQQPDLA PGLTTIGASPTQTVTLVTQPVVTKETAISKLEMPSSLMLEVPTLERLQ ELQEATDELDLKLRQAEVIKGSWQPVGDLLIDSLQDHLEKVKALRGEI APLKENVSHVNDLARQLTTLGIQLSPYNLSTLEDLNTRWKLLQVAVED RVRQLHEAHRDFGPASQHFLSTSVQGPWERAISPNKVPYYINHETQTT CWDHPKMTELYQSLADLNNVRFSAYRTAMKLRRLQKALCLDLLSLSAA CDALDQHNLKQNDQPMDILQIINCLTTIYDRLEQEHNNLVNVPLCVDM CLNWLLNVYDTGRTGRIRVLSFKTGIISLCKAHLEDKYRYLFKQVASS TGFCDQRRLGLLLHDSIQIPRQLGEVASFGGSNIEPSVRSCFQFANNK PEIEAALFLDWMRLEPQSMVWLPVLHRVAAAETAKHQAKCNICKECPI IGFRYRSLKHFNYDICQSCFFSGRVAKGHKMHYPMVEYCTPTTSGEDV RDFAKVLKNKFRTKRYFAKHPRMGYLPVQTVLEGDNMET |
| DYS5 | 37 | MLWWEEVEDCYEREDVQKKTFTKWVNAQFSKFGKQHIENLFSDLQDGR RLLDLLEGLTGQKLPKEKGSTRVHALNNVNKALRVLQNNNVDLVNIGS TDIVDGNHKLTLGLIWNIILHWQVKNVMKNIMAGLQQTNSEKILLSWV RQSTRNYPQVNVINFTTSWSDGLALNALIHSHRPDLFDWNSVVCQQSA TQRLEHAFNIARYQLGIEKLLDPEDVDTTYPDKKSILMYITSLFQVLP QQVSIEAIQEVEMLPRPPKVTKEEHFQLHHQMHYSQQITVSLAQGYER TSSPKPRFKSYAYTQAAYVTTSDPTRSPFPSQHLEAPEDKSFGSSLME SEVNLDRYQTALEEVLSWLLSAEDTLQAQGEISNDVEVVKDQFHTHEG YMMDLTAHQGRVGNILQLGSKLIGTGKLSEDEETEVQEQMNLLNSRWE CLRVASMEKQSNLHRVLMDLQNQKLKELNDWLTKTEERTRKMEEEPLG PDLEDLKRQVQQHKVLQEDLEQEQVRVNSLTHMVVVVDESSGDHATAA |
| | | LEEQLKVLGDRWANICRWTEDRWVLLQDILLKWQRLTEEQCLFSAWLS EKEDAVNKIHTTGFKDQNEMLSSLQKLAVLKADLEKKKQSMGKLYSLK QDLLSTLKNKSVTQKTEAWLDNFARCWDNLVQKLEKSTAQISQQPDLA PGLTTIGASPTQTVTLVTQPVVTKETAISKLEMPSSLMLEVPTLERLQ ELQEATDELDLKLRQAEVIKGSWQPVGDLLIDSLQDHLEKVKALRGEI APLKENVSHVNDLARQLTTLGIQLSPYNLSTLEDLNTRWKLLQVAVED RVRQLHEAHRDFGPASQHFLSTSVQGPWERAISPNKVPYYINHETQTT CWDHPKMTELYQSLADLNNVRFSAYRTAMKLRRLQKALCLDLLSLSAA CDALDQHNLKQNDQPMDILQIINCLTTIYDRLEQEHNNLVNVPLCVDM CLNWLLNVYDTGRTGRIRVLSFKTGIISLCKAHLEDKYRYLFKQVASS TGFCDQRRLGLLLHDSIQIPRQLGEVASFGGSNIEPSVRSCFQFANNK PEIEAALFLDWMRLEPQSMVWLPVLHRVAAAETAKHQAKCNICKECPI IGFRYRSLKHFNYDICQSCFFSGRVAKGHKMHYPMVEYCTPTTSGEDV RDFAKVLKNKFRTKRYFAKHPRMGYLPVQTVLEGDNMETPVTLINFWP VDSAPASSPQLSHDDTHSRIEHYASRLAEMENSNGSYLNDSISPNESI DDEHLLIQHYCQSLNQDSPLSQPRSPAQILISLES |
| **human** MD1 (R4-R23/ΔCT ) | 56 | MLWWEEVEDCYEREDVQKKTFTKWVNAQFSKFGKQHIENLFSDLQDGR RLLDLLEGLTGQKLPKEKGSTRVHALNNVNKALRVLQNNNVDLVNIGS TDIVDGNHKLTLGLIWNIILHWQVKNVMKNIMAGLQQTNSEKILLSWV RQSTRNYPQVNVINFTTSWSDGLALNALIHSHRPDLFDWNSVVCQQSA TQRLEHAFNIARYQLGIEKLLDPEDVDTTYPDKKSILMYITSLFQVLP QQVSIEAIQEVEMLPRPPKVTKEEHFQLHHQMHYSQQITVSLAQGYER TSSPKPRFKSYAYTQAAYVTTSDPTRSPFPSQHLEAPEDKSFGSSLME SEVNLDRYQTALEEVLSWLLSAEDTLQAQGEISNDVEVVKDQFHTHEG YMMDLTAHQGRVGNILQLGSKLIGTGKLSEDEETEVQEQMNLLNSRWE CLRVASMEKQSNLHRVLMDLQNQKLKELNDWLTKTEERTRKMEEEPLG PDLEDLKRQVQQHKVLQEDLEQEQVRVNSLTHMVVVVDESSGDHATAA LEEQLKVLGDRWANICRWTEDRWVLLQDILLKWQRLTEEQCLFSAWLS EKEDAVNKIHTTGFKDQNEMLSSLQKLAVLKADLEKKKQSMGKLYSLK QDLLSTLKNKSVTQKTEAWLDNFARCWDNLVQKLEKSTAQISQAVTTT QPSLTQTTVMETVTTVTTREQILVKHAQEELPPPPPQKKRQITVDTLE RLQELQEATDELDLKLRQAEVIKGSWQPVGDLLIDSLQDHLEKVKALR GEIAPLKENVSHVNDLARQLTTLGIQLSPYNLSTLEDLNTRWKLLQVA VEDRVRQLHEAHRDFGPASQHFLSTSVQGPWERAISPNKVPYYINHET QTTCWDHPKMTELYQSLADLNNVRFSAYRTAMKLRRLQKALCLDLLSL SAACDALDQHNLKQNDQPMDILQIINCLTTIYDRLEQEHNNLVNVPLC VDMCLNWLLNVYDTGRTGRIRVLSFKTGIISLCKAHLEDKYRYLFKQV ASSTGFCDQRRLGLLLHDSIQIPRQLGEVASFGGSNIEPSVRSCFQFA NNKPEIEAALFLDWMRLEPQSMVWLPVLHRVAAAETAKHQAKCNICKE CPIIGFRYRSLKHFNYDICQSCFFSGRVAKGHKMHYPMVEYCTPTTSG EDVRDFAKVLKNKFRTKRYFAKHPRMGYLPVQTVLEGDNMETDTM |
| **Human** microdys trophin | 57 | MLWWEEVEDCYEREDVQKKTFTKWVNAQFSKFGKQHIENLFSDLQDGR RLLDLLEGLTGQKLPKEKGSTRVHALNNVNKALRVLQNNNVDLVNIGS TDIVDGNHKLTLGLIWNIILHWQVKNVMKNIMAGLQQTNSEKILLSWV RQSTRNYPQVNVINFTTSWSDGLALNALIHSHRPDLFDWNSVVCQQSA TQRLEHAFNIARYQLGIEKLLDPEDVDTTYPDKKSILMYITSLFQVLP QQVSIEAIQEVEMLPRPPKVTKEEHFQLHHQMHYSQQITVSLAQGYER TSSPKPRFKSYAYTQAAYVTTSDPTRSPFPSQHLEAPEDKSFGSSLME SEVNLDRYQTALEEVLSWLLSAEDTLQAQGEISNDVEVVKDQFHTHEG YMMDLTAHQGRVGNILQLGSKLIGTGKLSEDEETEVQEQMNLLNSRWE CLRVASMEKQSNLHRVLMDLQNQKLKELNDWLTKTEERTRKMEEEPLG PDLEDLKRQVQQHKVLQEDLEQEQVRVNSLTHMVVVVDESSGDHATAA LEEQLKVLGDRWANICRWTEDRWVLLQDILLKWQRLTEEQCLFSAWLS EKEDAVNKIHTTGFKDQNEMLSSLQKLAVLKADLEKKKQSMGKLYSLK |
| | | QDLLSTLKNKSVTQKTEAWLDNFARCWDNLVQKLEKSTAQISQAVTTT QPSLTQTTVMETVTTVTTREQILVKHAQEELPPPPPQKKRTLERLQEL QEATDELDLKLRQAEVIKGSWQPVGDLLIDSLQDHLEKVKALRGEIAP LKENVSHVNDLARQLTTLGIQLSPYNLSTLEDLNTRWKLLQVAVEDRV RQLHEAHRDFGPASQHFLSTSVQGPWERAISPNKVPYYINHETQTTCW DHPKMTELYQSLADLNNVRFSAYRTAMKLRRLQKALCLDLLSLSAACD ALDQHNLKQNDQPMDILQIINCLTTIYDRLEQEHNNLVNVPLCVDMCL NWLLNVYDTGRTGRIRVLSFKTGIISLCKAHLEDKYRYLFKQVASSTG FCDQRRLGLLLHDSIQIPRQLGEVASFGGSNIEPSVRSCFQFANNKPE IEAALFLDWMRLEPQSMVWLPVLHRVAAAETAKHQAKCNICKECPIIG FRYRSLKHFNYDICQSCFFSGRVAKGHKMHYPMVEYCTPTTSGEDVRD FAKVLKNKFRTKRYFAKHPRMGYLPVQTVLEGDNMETDTM |
| Dys3978 | 58 | MLWWEEVEDCYEREDVQKKTFTKWVNAQFSKFGKQHIENLFSDLQDGR RLLDLLEGLTGQKLPKEKGSTRVHALNNVNKALRVLQNNNVDLVNIGS TDIVDGNHKLTLGLIWNIILHWQVKNVMKNIMAGLQQTNSEKILLSWV RQSTRNYPQVNVINFTTSWSDGLALNALIHSHRPDLFDWNSVVCQQSA TQRLEHAFNIARYQLGIEKLLDPEDVDTTYPDKKSILMYITSLFQVLP QQVSIEAIQEVEMLPRPPKVTKEEHFQLHHQMHYSQQITVSLAQGYER TSSPKPRFKSYAYTQAAYVTTSDPTRSPFPSQHLEAPEDKSFGSSLME SEVNLDRYQTALEEVLSWLLSAEDTLQAQGEISNDVEVVKDQFHTHEG YMMDLTAHQGRVGNILQLGSKLIGTGKLSEDEETEVQEQMNLLNSRWE CLRVASMEKQSNLHRVLMDLQNQKLKELNDWLTKTEERTRKMEEEPLG PDLEDLKRQVQQHKVLQEDLEQEQVRVNSLTHMVVVVDESSGDHATAA LEEQLKVLGDRWANICRWTEDRWVLLQDQPDLAPGLTTIGASPTQTVT LVTQPVVTKETAISKLEMPSSLMLEVPTHRLLQQFPLDLEKFLAWLTE AETTANVLQDATRKERLLEDSKGVKELMKQWQDLQGEIEAHTDVYHNL DENSQKILRSLEGSDDAVLLQRRLDNMNFKWSELRKKSLNIRSHLEAS SDQWKRLHLSLQELLVWLQLKDDELSRQAPIGGDFPAVQKQNDVHRAF KRELKTKEPVIMSTLETVRIFLTEQPLEGLEKLYQEPRELPPEERAQN VTRLLRKQAEEVNTEWEKLNLHSADWQRKIDETLERLQELQEATDELD LKLRQAEVIKGSWQPVGDLLIDSLQDHLEKVKALRGEIAPLKENVSHV NDLARQLTTLGIQLSPYNLSTLEDLNTRWKLLQVAVEDRVRQLHEAHR DFGPASQHFLSTSVQGPWERAISPNKVPYYINHETQTTCWDHPKMTEL YQSLADLNNVRFSAYRTAMKLRRLQKALCLDLLSLSAACDALDQHNLK QNDQPMDILQIINCLTTIYDRLEQEHNNLVNVPLCVDMCLNWLLNVYD TGRTGRIRVLSFKTGIISLCKAHLEDKYRYLFKQVASSTGFCDQRRLG LLLHDSIQIPRQLGEVASFGGSNIEPSVRSCFQFANNKPEIEAALFLD WMRLEPQSMVWLPVLHRVAAAETAKHQAKCNICKECPIIGFRYRSLKH FNYDICQSCFFSGRVAKGHKMHYPMVEYCTPTTSGEDVRDFAKVLKNK FRTKRYFAKHPRMGYLPVQTVLEGDNMET |
| Human MD3 | 59 | MLWWEEVEDCYEREDVQKKTFTKWVNAQFSKFGKQHIENLFSDLQDGR RLLDLLEGLTGQKLPKEKGSTRVHALNNVNKALRVLQNNNVDLVNIGS TDIVDGNHKLTLGLIWNIILHWQVKNVMKNIMAGLQQTNSEKILLSWV RQSTRNYPQVNVINFTTSWSDGLALNALIHSHRPDLFDWNSVVCQQSA TQRLEHAFNIARYQLGIEKLLDPEDVDTTYPDKKSILMYITSLFQVLP QQVSIEAIQEVEMLPRPPKVTKEEHFQLHHQMHYSQQITVSLAQGYER TSSPKPRFKSYAYTQAAYVTTSDPTRSPFPSQHLEAPEDKSFGSSLME SEVNLDRYQTALEEVLSWLLSAEDTLQAQGEISNDVEVVKDQFHTHEG YMMDLTAHQGRVGNILQLGSKLIGTGKLSEDEETEVQEQMNLLNSRWE CLRVASMEKQSNLHRVLMDLQNQKLKELNDWLTKTEERTRKMEEEPLG PDLEDLKRQVQQHKVLQEDLEQEQVRVNSLTHMVVVVDESSGDHATAA LEEQLKVLGDRWANICRWTEDRWVLLQDILLKWQRLTEEQCLFSAWLS EKEDAVNKIHTTGFKDQNEMLSSLQKLAVLKADLEKKKQSMGKLYSLK QDLLSTLKNKSVTQKTEAWLDNFARCWDNLVQKLEK |
| | | STAQISQAVTTTQPSLTQTTVMETVTTVTTREQILVKHAQEELPPPPP QKKRQITVDTLERLQELQEATDELDLKLRQAEVIKGSWQPVGDLLIDS LQDHLEKVKALRGEIAPLKENVSHVNDLARQLTTLGIQLSPYNLSTLE DLNTRWKLLQVAVEDRVRQLHEAHRDFGPASQHFLSTSVQGPWERAIS PNKVPYYINHETQTTCWDHPKMTELYQSLADLNNVRFSAYRTAMKLRR LQKALCLDLLSLSAACDALDQHNLKQNDQPMDILQIINCLTTIYDRLE QEHNNLVNVPLCVDMCLNWLLNVYDTGRTGRIRVLSFKTGIISLCKAH LEDKYRYLFKQVASSTGFCDQRRLGLLLHDSIQIPRQLGEVASFGGSN IEPSVRSCFQFANNKPEIEAALFLDWMRLEPQSMVWLPVLHRVAAAET AKHQAKCNICKECPIIGFRYRSLKHFNYDICQSCFFSGRVAKGHKMHY PMVEYCTPTTSGEDVRDFAKVLKNKFRTKRYFAKHPRMGYLPVQTVLE GDNMETPVTLINFWPVDSAPASSPQLSHDDTHSRIEHYASRLAEMENS NGSYLNDSISPNESIDDEHLLIQHYCQSLNQDSPLSQPRSPAQILISL ESEERGELERILADLEEENRNLQAEYDRLKQQHEHKGLSPLPSPPEMM PTSPQSPRDAELIAEAKLLRQHKGRLEARMQILEDHNKQLESQLHRLR QLLEQPQAEDTM |
| Human MD4 | 60 | MLWWEEVEDCYEREDVQKKTFTKWVNAQFSKFGKQHIENLFSDLQDGR RLLDLLEGLTGQKLPKEKGSTRVHALNNVNKALRVLQNNNVDLVNIGS TDIVDGNHKLTLGLIWNIILHWQVKNVMKNIMAGLQQTNSEKILLSWV RQSTRNYPQVNVINFTTSWSDGLALNALIHSHRPDLFDWNSVVCQQSA TQRLEHAFNIARYQLGIEKLLDPEDVDTTYPDKKSILMYITSLFQVLP QQVSIEAIQEVEMLPRPPKVTKEEHFQLHHQMHYSQQITVSLAQGYER TSSPKPRFKSYAYTQAAYVTTSDPTRSPFPSQHLEAPEDKSFGSSLME SEVNLDRYQTALEEVLSWLLSAEDTLQAQGEISNDVEVVKDQFHTHEG YMMDLTAHQGRVGNILQLGSKLIGTGKLSEDEETEVQEQMNLLNSRWE CLRVASMEKQSNLHRVLMDLQNQKLKELNDWLTKTEERTRKMEEEPLG PDLEDLKRQVQQHKVLQEDLEQEQVRVNSLTHMVVVVDESSGDHATAA LEEQLKVLGDRWANICRWTEDRWVLLQDILLKWQRLTEEQCLFSAWLS EKEDAVNKIHTTGFKDQNEMLSSLQKLAVLKADLEKKKQSMGKLYSLK QDLLSTLKNKSVTQKTEAWLDNFARCWDNLVQKLEKSTAQISQAVTTT QPSLTQTTVMETVTTVTTREQILVKHAQEELPPPPPQKKRQITVDTLE RLQELQEATDELDLKLRQAEVIKGSWQPVGDLLIDSLQDHLEKVKALR GEIAPLKENVSHVNDLARQLTTLGIQLSPYNLSTLEDLNTRWKLLQVA VEDRVRQLHEAHRDFGPASQHFLSTSVQGPWERAISPNKVPYYINHET QTTCWDHPKMTELYQSLADLNNVRFSAYRTAMKLRRLQKALCLDLLSL SAACDALDQHNLKQNDQPMDILQIINCLTTIYDRLEQEHNNLVNVPLC VDMCLNWLLNVYDTGRTGRIRVLSFKTGIISLCKAHLEDKYRYLFKQV ASSTGFCDQRRLGLLLHDSIQIPRQLGEVASFGGSNIEPSVRSCFQFA NNKPEIEAALFLDWMRLEPQSMVWLPVLHRVAAAETAKHQAKCNICKE CPIIGFRYRSLKHFNYDICQSCFFSGRVAKGHKMHYPMVEYCTPTTSG EDVRDFAKVLKNKFRTKRYFAKHPRMGYLPVQTVLEGDNMETPVTLIN FWPVDSAPASSPQLSHDDTHSRIEHYASRLAEMENSNGSYLNDSISPN ESIDDEHLLIQHYCQSLNQDSPLSQPRSPAQILISLESEERGELERIL ADLEEENRNLQAEYDRLKQQHEHKGLSPLPSPPEMMPTSPQSPRDAEL IAEAKLLRQHKGRLEARMQILEDHNKQLESQLHRLRQLLEQPQAEAKV NGTTVSSPSTSLQRSDSSQPMLLRVVGSQTSDSMGEEDLLSPPQDTST GLEEVMEQLNNSFPSSRGRNTPGKPMREDTM |
| MD5 | 72 | MLWWEEVEDCYEREDVQKKTFTKWVNAQFSKFGKQHIENLFSDLQDGR RLLDLLEGLTGQKLPKEKGSTRVHALNNVNKALRVLQNNNVDLVNIGS TDIVDGNHKLTLGLIWNIILHWQVKNVMKNIMAGLQQTNSEKILLSWV RQSTRNYPQVNVINFTTSWSDGLALNALIHSHRPDLFDWNSVVCQQSA TQRLEHAFNIARYQLGIEKLLDPEDVDTTYPDKKSILMYITSLFQVLP QQVSIEAIQEVEMLPRPPKVTKEEHFQLHHQMHYSQQITVSLAQGYER TSSPKPRFKSYAYTQAAYVTTSDPTRSPFPSQHLEAPEDKSFGSSLME |
| | | SEVNLDRYQTALEEVLSWLLSAEDTLQAQGEISNDVEVVKDQFHTHEG YMMDLTAHQGRVGNILQLGSKLIGTGKLSEDEETEVQEQMNLLNSRWE CLRVASMEKQSNLHSYVPSTYLTEITHVSQALLEVEQLLNAPDLCAKD FEDLFKQEESLKNIKDSLQQSSGRIDIIHSKKTAALQSATPVERVKLQ EALSQLDFQWEKVNKMYKDRQGRFDRSVEKWRRFHYDIKIFNQWLTEA EQFLRKTQIPENWEHAKYKWYLKELQDGIGQRQTWRTLNATGEEIIQQ SSKTDASILQEKLGSLNLRWQEVCKQLSDRKKRLEEQSDQWKRLHLSL QELLVWLQLKDDELSRQAPIGGDFPAVQKQNDVHRAFKRELKTKEPVI MSTLETVRIFLTEQPLEGLEKLYQEPRELPPEERAQNVTRLLRKQAEE VNTEWEKLNLHSADWQRKIDETLERLQELQEATDELDLKLRQAEVIKG SWQPVGDLLIDSLQDHLEKVKALRGEIAPLKENVSHVNDLARQLTTLG IQLSPYNLSTLEDLNTRWKLLQVAVEDRVRQLHEAHRDFGPASQHFLS TSVQGPWERAISPNKVPYYINHETQTTCWDHPKMTELYQSLADLNNVR FSAYRTAMKLRRLQKALCLDLLSLSAACDALDQHNLKQNDQPMDILQI INCLTTIYDRLEQEHNNLVNVPLCVDMCLNWLLNVYDTGRTGRIRVLS FKTGIISLCKAHLEDKYRYLFKQVASSTGFCLHRVAAAETAKHQAKCN ICKECPIIGFRYRSLKHFNYDICQSCFFSGRVAKGHKMHYPMVEYCTP TTSGEDVRDFAKVLKNKFRTKRYFAKHPRMGYLPVQTVLEGDNMETDT M |

Examples of a recombinant expression cassettes and portions of a recombinant expression cassette having a promoter operably linked to a transgene are shown in Table 6, including the genome sequences with 5' and 3' ITR sequences. Also provided are recombinant expression cassettes comprising a nucleotide sequence coding for any of the microdystrophins provided in Table 5 operably linked to either Spc5-12 V1 or Spc5-12 V2 and any other regulatory elements, such as polyA signal sequences.

**Table 6**

| SEQ ID NO: | Description | Sequence |
|---|---|---|
| 38 | Spc5-12 V1-Microdystrophin (µDys1) nucleotide | AGAGGCCGTCCGCCCTCGGCACCATCCTCACGACACCCAAATATGGCGACGG GTGAGGAATGGTGGGGAGTTATTTTTAGAGCGGTGAGGAAGGTGGGCAGGCA GCAGGTGTTGGCGCTCCATATTTGGCGGGAGTTATTTTTAGAGCGGAGGAAT GGTGGACACCCAAATATGGCGACGGTTCCTCACCCGTCGCTAAAAATAACTC CGTGTCCGCCCTCGGCCGGGGCCGCATTCCTGGGGGCCGGGCGGTGCTCCCG CCCGCCTCGATAAAAGGCTCCGGGGCCGGCGGCGGCCCACGAGCTACCCGGA GGAGCGGGAGGCGCCAAGCGgAATTCGCCACCATGCTTTGGTGGGAAGAGGT GGAAGATTGCTATGAGAGGGAAGATGTGCAGAAGAAAACCTTCACCAAATGG GTCAATGCCCAGTTCAGCAAGTTTGGCAAGCAGCACATTGAGAACCTGTTCA GTGACCTGCAGGATGGCAGAAGGCTGCTGGATCTGCTGGAAGGCCTGACAGG CCAGAAGCTGCCTAAAGAGAAGGGCAGCACAAGAGTGCATGCCCTGAACAAT GTGAACAAGGCCCTGAGAGTGCTGCAGAACAACAATGTGGACCTGGTCAATA TTGGCAGCACAGACATTGTGGATGGCAACCACAAGCTGACCCTGGGCCTGAT CTGGAACATCATCCTGCACTGGCAAGTGAAGAATGTGATGAAGAACATCATG GCTGGCCTGCAGCAGACCAACTCTGAGAAGATCCTGCTGAGCTGGGTCAGAC AGAGCACCAGAAACTACCCTCAAGTGAATGTGATCAACTTCACCACCTCTTG GAGTGATGGACTGGCCCTGAATGCCCTGATCCACAGCCACAGACCTGACCTG TTTGACTGGAACTCTGTTGTGTGCCAGCAGTCTGCCACACAGAGACTGGAAC ATGCCTTCAACATTGCCAGATACCAGCTGGGAATTGAGAAACTGCTGGACCC TGAGGATGTGGACACCACCTATCCTGACAAGAAATCCATCCTCATGTACATC |
| | | ACCAGCCTGTTCCAGGTGCTGCCCCAGCAAGTGTCCATTGAGGCCATTCAAG AGGTTGAGATGCTGCCCAGACCTCCTAAAGTGACCAAAGAGGAACACTTCCA GCTGCACCACCAGATGCACTACTCTCAGCAGATCACAGTGTCTCTGGCCCAG GGATATGAGAGAACAAGCAGCCCCAAGCCTAGGTTCAAGAGCTATGCCTACA CACAGGCTGCCTATGTGACCACATCTGACCCCACAAGAAGCCCATTTCCAAG CCAGCATCTGGAAGCCCCTGAGGACAAGAGCTTTGGCAGCAGCCTGATGGAA TCTGAAGTGAACCTGGATAGATACCAGACAGCCCTGGAAGAAGTGCTGTCCT GGCTGCTGTCTGCTGAGGATACACTGCAGGCTCAGGGTGAAATCAGCAATGA TGTGGAAGTGGTCAAGGACCAGTTTCACACCCATGAGGGCTACATGATGGAC CTGACAGCCCACCAGGGCAGAGTGGGAAATATCCTGCAGCTGGGCTCCAAGC TGATTGGCACAGGCAAGCTGTCTGAGGATGAAGAGACAGAGGTGCAAGAGCA GATGAACCTGCTGAACAGCAGATGGGAGTGTCTGAGAGTGGCCAGCATGGAA AAGCAGAGCAACCTGCACAGAGTGCTCATGGACCTGCAGAATCAGAAACTGA AAGAACTGAATGACTGGCTGACCAAGACAGAAGAAAGGACTAGGAAGATGGA AGAGGAACCTCTGGGACCAGACCTGGAAGATCTGAAAAGACAGGTGCAGCAG CATAAGGTGCTGCAAGAGGACCTTGAGCAAGAGCAAGTCAGAGTGAACAGCC TGACACACATGGTGGTGGTTGTGGATGAGTCCTCTGGGGATCATGCCACAGC TGCTCTGGAAGAACAGCTGAAGGTGCTGGGAGACAGATGGGCCAACATCTGT AGGTGGACAGAGGATAGATGGGTGCTGCTCCAGGACATTCTGCTGAAGTGGC AGAGACTGACAGAGGAACAGTGCCTGTTTTCTGCCTGGCTCTCTGAGAAAGA GGATGCTGTCAACAAGATCCATACCACAGGCTTCAAGGATCAGAATGAGATG CTCAGCTCCCTGCAGAAACTGGCTGTGCTGAAGGCTGACCTGGAAAAGAAAA AGCAGTCCATGGGCAAGCTCTACAGCCTGAAGCAGGACCTGCTGTCTACCCT GAAGAACAAGTCTGTGACCCAGAAAACTGAGGCCTGGCTGGACAACTTTGCT AGATGCTGGGACAACCTGGTGCAGAAGCTGGAAAAGTCTACAGCCCAGATCA GCCAGCAACCTGATCTTGCCCCTGGCCTGACCACAATTGGAGCCTCTCCAAC ACAGACTGTGACCCTGGTTACCCAGCCAGTGGTCACCAAAGAGACAGCCATC AGCAAACTGGAAATGCCCAGCTCTCTGATGCTGGAAGTCCCCACACTGGAAA GGCTGCAAGAACTTCAAGAGGCCACAGATGAGCTGGACCTGAAGCTGAGACA GGCTGAAGTGATCAAAGGCAGCTGGCAGCCAGTTGGGGACCTGCTCATTGAT AGCCTGCAGGACCATCTGGAAAAAGTGAAAGCCCTGAGGGGAGAGATTGCCC CTCTGAAAGAAAATGTGTCCCATGTGAATGACCTGGCCAGACAGCTGACCAC ACTGGGAATCCAGCTGAGCCCCTACAACCTGAGCACCCTTGAGGACCTGAAC ACCAGGTGGAAGCTCCTCCAGGTGGCAGTGGAAGATAGAGTCAGGCAGCTGC ATGAGGCCCACAGAGATTTTGGACCAGCCAGCCAGCACTTTCTGTCTACCTC TGTGCAAGGCCCCTGGGAGAGAGCTATCTCTCCTAACAAGGTGCCCTACTAC ATCAACCATGAGACACAGACCACCTGTTGGGATCACCCCAAGATGACAGAGC TGTACCAGAGTCTGGCAGACCTCAACAATGTCAGATTCAGTGCCTACAGGAC TGCCATGAAGCTCAGAAGGCTCCAGAAAGCTCTGTGCCTGGACCTGCTTTCC CTGAGTGCAGCTTGTGATGCCCTGGACCAGCACAATCTGAAGCAGAATGACC AGCCTATGGACATCCTCCAGATCATCAACTGCCTCACCACCATCTATGATAG GCTGGAACAAGAGCACAACAATCTGGTCAATGTGCCCCTGTGTGTGGACATG TGCCTGAATTGGCTGCTGAATGTGTATGACACAGGCAGAACAGGCAGGATCA GAGTCCTGTCCTTCAAGACAGGCATCATCTCCCTGTGCAAAGCCCACTTGGA GGACAAGTACAGATACCTGTTCAAGCAAGTGGCCTCCAGCACAGGCTTTTGT GACCAGAGAAGGCTGGGCCTGCTCCTGCATGACAGCATTCAGATCCCTAGAC AGCTGGGAGAAGTGGCTTCCTTTGGAGGCAGCAATATTGAGCCATCAGTCAG GTCCTGTTTTCAGTTTGCCAACAACAAGCCTGAGATTGAGGCTGCCCTGTTC CTGGACTGGATGAGACTTGAGCCTCAGAGCATGGTCTGGCTGCCTGTGCTTC ATAGAGTGGCTGCTGCTGAGACTGCCAAGCACCAGGCCAAGTGCAACATCTG CAAAGAGTGCCCCATCATTGGCTTCAGATACAGATCCCTGAAGCACTTCAAC TATGATATCTGCCAGAGCTGCTTCTTTAGTGGCAGGGTTGCCAAGGGCCACA AAATGCACTACCCCATGGTGGAATACTGCACCCCAACAACCTCTGGGGAAGA TGTTAGAGACTTTGCCAAGGTGCTGAAAAACAAGTTCAGGACCAAGAGATAC TTTGCTAAGCACCCCAGAATGGGCTACCTGCCTGTCCAGACAGTGCTTGAGG GTGACAACATGGAAACCCCTGTGACACTGATCAATTTCTGGCCAGTGGACTC TGCCCCTGCCTCAAGTCCACAGCTGTCCCATGATGACACCCACAGCAGAATT GAGCACTATGCCTCCAGACTGGCAGAGATGGAAAACAGCAATGGCAGCTACC |
| | | TGAATGATAGCATCAGCCCCAATGAGAGCATTGATGATGAGCATCTGCTGAT CCAGCACTACTGTCAGTCCCTGAACCAGGACTCTCCACTGAGCCAGCCTAGA AGCCCTGCTCAGATCCTGATCAGCCTTGAGTCTGAGGAAAGGGGAGAGCTGG AAAGAATCCTGGCAGATCTTGAGGAAGAGAACAGAAACCTGCAGGCAGAGTA TGACAGGCTCAAACAGCAGCATGAGCACAAGGGACTGAGCCCTCTGCCTTCT CCTCCTGAAATGATGCCCACCTCTCCACAGTCTCCAAGGTGATGA |
| 39 | Spc5-12 V1-µDys1 transgene cassette (ITR to ITR) | CTGCGCGCTCGCTCGCTCACTGAGGCCGCCCGGGCAAAGCCCGGGCGTCGGG CGACCTTTGGTCGCCCGGCCTCAGTGAGCGAGCGAGCGCGCAGAGAGGGAGT GGCCAACTCCATCACTAGGGGTTCCTCATATGCAGGGTAATGGGGATCCTCT AGAGGCCGTCCGCCCTCGGCACCATCCTCACGACACCCAAATATGGCGACGG GTGAGGAATGGTGGGGAGTTATTTTTAGAGCGGTGAGGAAGGTGGGCAGGCA GCAGGTGTTGGCGCTCCATATTTGGCGGGAGTTATTTTTAGAGCGGAGGAAT GGTGGACACCCAAATATGGCGACGGTTCCTCACCCGTCGCTAAAAATAACTC CGTGTCCGCCCTCGGCCGGGGCCGCATTCCTGGGGGCCGGGCGGTGCTCCCG CCCGCCTCGATAAAAGGCTCCGGGGCCGGCGGCGGCCCACGAGCTACCCGGA GGAGCGGGAGGCGCCAAGCGGAATTCGCCACCATGCTTTGGTGGGAAGAGGT GGAAGATTGCTATGAGAGGGAAGATGTGCAGAAGAAAACCTTCACCAAATGG GTCAATGCCCAGTTCAGCAAGTTTGGCAAGCAGCACATTGAGAACCTGTTCA GTGACCTGCAGGATGGCAGAAGGCTGCTGGATCTGCTGGAAGGCCTGACAGG CCAGAAGCTGCCTAAAGAGAAGGGCAGCACAAGAGTGCATGCCCTGAACAAT GTGAACAAGGCCCTGAGAGTGCTGCAGAACAACAATGTGGACCTGGTCAATA TTGGCAGCACAGACATTGTGGATGGCAACCACAAGCTGACCCTGGGCCTGAT CTGGAACATCATCCTGCACTGGCAAGTGAAGAATGTGATGAAGAACATCATG GCTGGCCTGCAGCAGACCAACTCTGAGAAGATCCTGCTGAGCTGGGTCAGAC AGAGCACCAGAAACTACCCTCAAGTGAATGTGATCAACTTCACCACCTCTTG GAGTGATGGACTGGCCCTGAATGCCCTGATCCACAGCCACAGACCTGACCTG TTTGACTGGAACTCTGTTGTGTGCCAGCAGTCTGCCACACAGAGACTGGAAC ATGCCTTCAACATTGCCAGATACCAGCTGGGAATTGAGAAACTGCTGGACCC TGAGGATGTGGACACCACCTATCCTGACAAGAAATCCATCCTCATGTACATC ACCAGCCTGTTCCAGGTGCTGCCCCAGCAAGTGTCCATTGAGGCCATTCAAG AGGTTGAGATGCTGCCCAGACCTCCTAAAGTGACCAAAGAGGAACACTTCCA GCTGCACCACCAGATGCACTACTCTCAGCAGATCACAGTGTCTCTGGCCCAG GGATATGAGAGAACAAGCAGCCCCAAGCCTAGGTTCAAGAGCTATGCCTACA CACAGGCTGCCTATGTGACCACATCTGACCCCACAAGAAGCCCATTTCCAAG CCAGCATCTGGAAGCCCCTGAGGACAAGAGCTTTGGCAGCAGCCTGATGGAA TCTGAAGTGAACCTGGATAGATACCAGACAGCCCTGGAAGAAGTGCTGTCCT GGCTGCTGTCTGCTGAGGATACACTGCAGGCTCAGGGTGAAATCAGCAATGA TGTGGAAGTGGTCAAGGACCAGTTTCACACCCATGAGGGCTACATGATGGAC CTGACAGCCCACCAGGGCAGAGTGGGAAATATCCTGCAGCTGGGCTCCAAGC TGATTGGCACAGGCAAGCTGTCTGAGGATGAAGAGACAGAGGTGCAAGAGCA GATGAACCTGCTGAACAGCAGATGGGAGTGTCTGAGAGTGGCCAGCATGGAA AAGCAGAGCAACCTGCACAGAGTGCTCATGGACCTGCAGAATCAGAAACTGA AAGAACTGAATGACTGGCTGACCAAGACAGAAGAAAGGACTAGGAAGATGGA AGAGGAACCTCTGGGACCAGACCTGGAAGATCTGAAAAGACAGGTGCAGCAG CATAAGGTGCTGCAAGAGGACCTTGAGCAAGAGCAAGTCAGAGTGAACAGCC TGACACACATGGTGGTGGTTGTGGATGAGTCCTCTGGGGATCATGCCACAGC TGCTCTGGAAGAACAGCTGAAGGTGCTGGGAGACAGATGGGCCAACATCTGT AGGTGGACAGAGGATAGATGGGTGCTGCTCCAGGACATTCTGCTGAAGTGGC AGAGACTGACAGAGGAACAGTGCCTGTTTTCTGCCTGGCTCTCTGAGAAAGA GGATGCTGTCAACAAGATCCATACCACAGGCTTCAAGGATCAGAATGAGATG CTCAGCTCCCTGCAGAAACTGGCTGTGCTGAAGGCTGACCTGGAAAAGAAAA AGCAGTCCATGGGCAAGCTCTACAGCCTGAAGCAGGACCTGCTGTCTACCCT GAAGAACAAGTCTGTGACCCAGAAAACTGAGGCCTGGCTGGACAACTTTGCT AGATGCTGGGACAACCTGGTGCAGAAGCTGGAAAAGTCTACAGCCCAGATCA GCCAGCAACCTGATCTTGCCCCTGGCCTGACCACAATTGGAGCCTCTCCAAC ACAGACTGTGACCCTGGTTACCCAGCCAGTGGTCACCAAAGAGACAGCCATC AGCAAACTGGAAATGCCCAGCTCTCTGATGCTGGAAGTCCCCACACTGGAAA GGCTGCAAGAACTTCAAGAGGCCACAGATGAGCTGGACCTGAAGCTGAGACA |
| | | GGCTGAAGTGATCAAAGGCAGCTGGCAGCCAGTTGGGGACCTGCTCATTGAT AGCCTGCAGGACCATCTGGAAAAAGTGAAAGCCCTGAGGGGAGAGATTGCCC CTCTGAAAGAAAATGTGTCCCATGTGAATGACCTGGCCAGACAGCTGACCAC ACTGGGAATCCAGCTGAGCCCCTACAACCTGAGCACCCTTGAGGACCTGAAC ACCAGGTGGAAGCTCCTCCAGGTGGCAGTGGAAGATAGAGTCAGGCAGCTGC ATGAGGCCCACAGAGATTTTGGACCAGCCAGCCAGCACTTTCTGTCTACCTC TGTGCAAGGCCCCTGGGAGAGAGCTATCTCTCCTAACAAGGTGCCCTACTAC ATCAACCATGAGACACAGACCACCTGTTGGGATCACCCCAAGATGACAGAGC TGTACCAGAGTCTGGCAGACCTCAACAATGTCAGATTCAGTGCCTACAGGAC TGCCATGAAGCTCAGAAGGCTCCAGAAAGCTCTGTGCCTGGACCTGCTTTCC CTGAGTGCAGCTTGTGATGCCCTGGACCAGCACAATCTGAAGCAGAATGACC AGCCTATGGACATCCTCCAGATCATCAACTGCCTCACCACCATCTATGATAG GCTGGAACAAGAGCACAACAATCTGGTCAATGTGCCCCTGTGTGTGGACATG TGCCTGAATTGGCTGCTGAATGTGTATGACACAGGCAGAACAGGCAGGATCA GAGTCCTGTCCTTCAAGACAGGCATCATCTCCCTGTGCAAAGCCCACTTGGA GGACAAGTACAGATACCTGTTCAAGCAAGTGGCCTCCAGCACAGGCTTTTGT GACCAGAGAAGGCTGGGCCTGCTCCTGCATGACAGCATTCAGATCCCTAGAC AGCTGGGAGAAGTGGCTTCCTTTGGAGGCAGCAATATTGAGCCATCAGTCAG GTCCTGTTTTCAGTTTGCCAACAACAAGCCTGAGATTGAGGCTGCCCTGTTC CTGGACTGGATGAGACTTGAGCCTCAGAGCATGGTCTGGCTGCCTGTGCTTC ATAGAGTGGCTGCTGCTGAGACTGCCAAGCACCAGGCCAAGTGCAACATCTG CAAAGAGTGCCCCATCATTGGCTTCAGATACAGATCCCTGAAGCACTTCAAC TATGATATCTGCCAGAGCTGCTTCTTTAGTGGCAGGGTTGCCAAGGGCCACA AAATGCACTACCCCATGGTGGAATACTGCACCCCAACAACCTCTGGGGAAGA TGTTAGAGACTTTGCCAAGGTGCTGAAAAACAAGTTCAGGACCAAGAGATAC TTTGCTAAGCACCCCAGAATGGGCTACCTGCCTGTCCAGACAGTGCTTGAGG GTGACAACATGGAAACCCCTGTGACACTGATCAATTTCTGGCCAGTGGACTC TGCCCCTGCCTCAAGTCCACAGCTGTCCCATGATGACACCCACAGCAGAATT GAGCACTATGCCTCCAGACTGGCAGAGATGGAAAACAGCAATGGCAGCTACC TGAATGATAGCATCAGCCCCAATGAGAGCATTGATGATGAGCATCTGCTGAT CCAGCACTACTGTCAGTCCCTGAACCAGGACTCTCCACTGAGCCAGCCTAGA AGCCCTGCTCAGATCCTGATCAGCCTTGAGTCTGAGGAAAGGGGAGAGCTGG AAAGAATCCTGGCAGATCTTGAGGAAGAGAACAGAAACCTGCAGGCAGAGTA TGACAGGCTCAAACAGCAGCATGAGCACAAGGGACTGAGCCCTCTGCCTTCT CCTCCTGAAATGATGCCCACCTCTCCACAGTCTCCAAGGTGATGACTCGAGA GGCCTAATAAAGAGCTCAGATGCATCGATCAGAGTGTGTTGGTTTTTTGTGT GCCAGGGTAATGGGCTAGCTGCGGCCGCAGGAACCCCTAGTGATGGAGTTGG CCACTCCCTCTCTGCGCGCTCGCTCGCTCACTGAGGCCGGGCGACCAAAGGT CGCCCGACGCCCGGGCTTTGCCCGGGCGGCCTCAGTGAGCGAGCGAGCGCGC AG |
| 40 | Spc5-12 V2-Microdystrophin (µDys1) nucleotide | GGCCGTCCGCCCTCGGCACCATCCTCACGACACCCAAATATGGCGACGGGTG AGGAATGGTGGGGAGTTATTTTTAGAGCGGTGAGGAAGGTGGGCAGGCAGCA GGTGTTGGGGGAGTTATTTTTAGAGCGGGGAGTTATTTTTAGAGCGGAGGAA TGGTGGACACCCAAATATGGCGACGGTTCCTCACGGACACCCAAATATGGCG ACGGGCCCTCGGCCGGGGCCGCATTCCTGGGGGCCGGGCGGTGCTCCCGCCC GCCTCGATAAAAGGCTCCGGGGCCGGCGGCGGCCCACGAGCTACCCGGAGGA GCGGGAGGCGCCAAGCGGAATTCGCCACCATGCTTTGGTGGGAAGAGGTGGA AGATTGCTATGAGAGGGAAGATGTGCAGAAGAAAACCTTCACCAAATGGGTC AATGCCCAGTTCAGCAAGTTTGGCAAGCAGCACATTGAGAACCTGTTCAGTG ACCTGCAGGATGGCAGAAGGCTGCTGGATCTGCTGGAAGGCCTGACAGGCCA GAAGCTGCCTAAAGAGAAGGGCAGCACAAGAGTGCATGCCCTGAACAATGTG AACAAGGCCCTGAGAGTGCTGCAGAACAACAATGTGGACCTGGTCAATATTG GCAGCACAGACATTGTGGATGGCAACCACAAGCTGACCCTGGGCCTGATCTG GAACATCATCCTGCACTGGCAAGTGAAGAATGTGATGAAGAACATCATGGCT GGCCTGCAGCAGACCAACTCTGAGAAGATCCTGCTGAGCTGGGTCAGACAGA GCACCAGAAACTACCCTCAAGTGAATGTGATCAACTTCACCACCTCTTGGAG TGATGGACTGGCCCTGAATGCCCTGATCCACAGCCACAGACCTGACCTGTTT GACTGGAACTCTGTTGTGTGCCAGCAGTCTGCCACACAGAGACTGGAACATG |
| | | CCTTCAACATTGCCAGATACCAGCTGGGAATTGAGAAACTGCTGGACCCTGA GGATGTGGACACCACCTATCCTGACAAGAAATCCATCCTCATGTACATCACC AGCCTGTTCCAGGTGCTGCCCCAGCAAGTGTCCATTGAGGCCATTCAAGAGG TTGAGATGCTGCCCAGACCTCCTAAAGTGACCAAAGAGGAACACTTCCAGCT GCACCACCAGATGCACTACTCTCAGCAGATCACAGTGTCTCTGGCCCAGGGA TATGAGAGAACAAGCAGCCCCAAGCCTAGGTTCAAGAGCTATGCCTACACAC AGGCTGCCTATGTGACCACATCTGACCCCACAAGAAGCCCATTTCCAAGCCA GCATCTGGAAGCCCCTGAGGACAAGAGCTTTGGCAGCAGCCTGATGGAATCT GAAGTGAACCTGGATAGATACCAGACAGCCCTGGAAGAAGTGCTGTCCTGGC TGCTGTCTGCTGAGGATACACTGCAGGCTCAGGGTGAAATCAGCAATGATGT GGAAGTGGTCAAGGACCAGTTTCACACCCATGAGGGCTACATGATGGACCTG ACAGCCCACCAGGGCAGAGTGGGAAATATCCTGCAGCTGGGCTCCAAGCTGA TTGGCACAGGCAAGCTGTCTGAGGATGAAGAGACAGAGGTGCAAGAGCAGAT GAACCTGCTGAACAGCAGATGGGAGTGTCTGAGAGTGGCCAGCATGGAAAAG CAGAGCAACCTGCACAGAGTGCTCATGGACCTGCAGAATCAGAAACTGAAAG AACTGAATGACTGGCTGACCAAGACAGAAGAAAGGACTAGGAAGATGGAAGA GGAACCTCTGGGACCAGACCTGGAAGATCTGAAAAGACAGGTGCAGCAGCAT AAGGTGCTGCAAGAGGACCTTGAGCAAGAGCAAGTCAGAGTGAACAGCCTGA CACACATGGTGGTGGTTGTGGATGAGTCCTCTGGGGATCATGCCACAGCTGC TCTGGAAGAACAGCTGAAGGTGCTGGGAGACAGATGGGCCAACATCTGTAGG TGGACAGAGGATAGATGGGTGCTGCTCCAGGACATTCTGCTGAAGTGGCAGA GACTGACAGAGGAACAGTGCCTGTTTTCTGCCTGGCTCTCTGAGAAAGAGGA TGCTGTCAACAAGATCCATACCACAGGCTTCAAGGATCAGAATGAGATGCTC AGCTCCCTGCAGAAACTGGCTGTGCTGAAGGCTGACCTGGAAAAGAAAAAGC AGTCCATGGGCAAGCTCTACAGCCTGAAGCAGGACCTGCTGTCTACCCTGAA GAACAAGTCTGTGACCCAGAAAACTGAGGCCTGGCTGGACAACTTTGCTAGA TGCTGGGACAACCTGGTGCAGAAGCTGGAAAAGTCTACAGCCCAGATCAGCC AGCAACCTGATCTTGCCCCTGGCCTGACCACAATTGGAGCCTCTCCAACACA GACTGTGACCCTGGTTACCCAGCCAGTGGTCACCAAAGAGACAGCCATCAGC AAACTGGAAATGCCCAGCTCTCTGATGCTGGAAGTCCCCACACTGGAAAGGC TGCAAGAACTTCAAGAGGCCACAGATGAGCTGGACCTGAAGCTGAGACAGGC TGAAGTGATCAAAGGCAGCTGGCAGCCAGTTGGGGACCTGCTCATTGATAGC CTGCAGGACCATCTGGAAAAAGTGAAAGCCCTGAGGGGAGAGATTGCCCCTC TGAAAGAAAATGTGTCCCATGTGAATGACCTGGCCAGACAGCTGACCACACT GGGAATCCAGCTGAGCCCCTACAACCTGAGCACCCTTGAGGACCTGAACACC AGGTGGAAGCTCCTCCAGGTGGCAGTGGAAGATAGAGTCAGGCAGCTGCATG AGGCCCACAGAGATTTTGGACCAGCCAGCCAGCACTTTCTGTCTACCTCTGT GCAAGGCCCCTGGGAGAGAGCTATCTCTCCTAACAAGGTGCCCTACTACATC AACCATGAGACACAGACCACCTGTTGGGATCACCCCAAGATGACAGAGCTGT ACCAGAGTCTGGCAGACCTCAACAATGTCAGATTCAGTGCCTACAGGACTGC CATGAAGCTCAGAAGGCTCCAGAAAGCTCTGTGCCTGGACCTGCTTTCCCTG AGTGCAGCTTGTGATGCCCTGGACCAGCACAATCTGAAGCAGAATGACCAGC CTATGGACATCCTCCAGATCATCAACTGCCTCACCACCATCTATGATAGGCT GGAACAAGAGCACAACAATCTGGTCAATGTGCCCCTGTGTGTGGACATGTGC CTGAATTGGCTGCTGAATGTGTATGACACAGGCAGAACAGGCAGGATCAGAG TCCTGTCCTTCAAGACAGGCATCATCTCCCTGTGCAAAGCCCACTTGGAGGA CAAGTACAGATACCTGTTCAAGCAAGTGGCCTCCAGCACAGGCTTTTGTGAC CAGAGAAGGCTGGGCCTGCTCCTGCATGACAGCATTCAGATCCCTAGACAGC TGGGAGAAGTGGCTTCCTTTGGAGGCAGCAATATTGAGCCATCAGTCAGGTC CTGTTTTCAGTTTGCCAACAACAAGCCTGAGATTGAGGCTGCCCTGTTCCTG GACTGGATGAGACTTGAGCCTCAGAGCATGGTCTGGCTGCCTGTGCTTCATA GAGTGGCTGCTGCTGAGACTGCCAAGCACCAGGCCAAGTGCAACATCTGCAA AGAGTGCCCCATCATTGGCTTCAGATACAGATCCCTGAAGCACTTCAACTAT GATATCTGCCAGAGCTGCTTCTTTAGTGGCAGGGTTGCCAAGGGCCACAAAA TGCACTACCCCATGGTGGAATACTGCACCCCAACAACCTCTGGGGAAGATGT TAGAGACTTTGCCAAGGTGCTGAAAAACAAGTTCAGGACCAAGAGATACTTT GCTAAGCACCCCAGAATGGGCTACCTGCCTGTCCAGACAGTGCTTGAGGGTG ACAACATGGAAACCTGATGA |
| 41 | Spc5-12 V2-µDys transgene cassette (ITR to ITR) | CTGCGCGCTCGCTCGCTCACTGAGGCCGCCCGGGCAAAGCCCGGGCGTCGGG CGACCTTTGGTCGCCCGGCCTCAGTGAGCGAGCGAGCGCGCAGAGAGGGAGT GGCCAACTCCATCACTAGGGGTTCCTCATATGCAGGGTAATGGGGATCCTCT AGAGGCCGTCCGCCCTCGGCACCATCCTCACGACACCCAAATATGGCGACGG GTGAGGAATGGTGGGGAGTTATTTTTAGAGCGGTGAGGAAGGTGGGCAGGCA GCAGGTGTTGGGGGAGTTATTTTTAGAGCGGGGAGTTATTTTTAGAGCGGAG GAATGGTGGACACCCAAATATGGCGACGGTTCCTCACGGACACCCAAATATG GCGACGGGCCCTCGGCCGGGGCCGCATTCCTGGGGGCCGGGCGGTGCTCCCG CCCGCCTCGATAAAAGGCTCCGGGGCCGGCGGCGGCCCACGAGCTACCCGGA GGAGCGGGAGGCGCCAAGCGGAATTCGCCACCATGCTTTGGTGGGAAGAGGT GGAAGATTGCTATGAGAGGGAAGATGTGCAGAAGAAAACCTTCACCAAATGG GTCAATGCCCAGTTCAGCAAGTTTGGCAAGCAGCACATTGAGAACCTGTTCA GTGACCTGCAGGATGGCAGAAGGCTGCTGGATCTGCTGGAAGGCCTGACAGG CCAGAAGCTGCCTAAAGAGAAGGGCAGCACAAGAGTGCATGCCCTGAACAAT GTGAACAAGGCCCTGAGAGTGCTGCAGAACAACAATGTGGACCTGGTCAATA TTGGCAGCACAGACATTGTGGATGGCAACCACAAGCTGACCCTGGGCCTGAT CTGGAACATCATCCTGCACTGGCAAGTGAAGAATGTGATGAAGAACATCATG GCTGGCCTGCAGCAGACCAACTCTGAGAAGATCCTGCTGAGCTGGGTCAGAC AGAGCACCAGAAACTACCCTCAAGTGAATGTGATCAACTTCACCACCTCTTG GAGTGATGGACTGGCCCTGAATGCCCTGATCCACAGCCACAGACCTGACCTG TTTGACTGGAACTCTGTTGTGTGCCAGCAGTCTGCCACACAGAGACTGGAAC ATGCCTTCAACATTGCCAGATACCAGCTGGGAATTGAGAAACTGCTGGACCC TGAGGATGTGGACACCACCTATCCTGACAAGAAATCCATCCTCATGTACATC ACCAGCCTGTTCCAGGTGCTGCCCCAGCAAGTGTCCATTGAGGCCATTCAAG AGGTTGAGATGCTGCCCAGACCTCCTAAAGTGACCAAAGAGGAACACTTCCA GCTGCACCACCAGATGCACTACTCTCAGCAGATCACAGTGTCTCTGGCCCAG GGATATGAGAGAACAAGCAGCCCCAAGCCTAGGTTCAAGAGCTATGCCTACA CACAGGCTGCCTATGTGACCACATCTGACCCCACAAGAAGCCCATTTCCAAG CCAGCATCTGGAAGCCCCTGAGGACAAGAGCTTTGGCAGCAGCCTGATGGAA TCTGAAGTGAACCTGGATAGATACCAGACAGCCCTGGAAGAAGTGCTGTCCT GGCTGCTGTCTGCTGAGGATACACTGCAGGCTCAGGGTGAAATCAGCAATGA TGTGGAAGTGGTCAAGGACCAGTTTCACACCCATGAGGGCTACATGATGGAC CTGACAGCCCACCAGGGCAGAGTGGGAAATATCCTGCAGCTGGGCTCCAAGC TGATTGGCACAGGCAAGCTGTCTGAGGATGAAGAGACAGAGGTGCAAGAGCA GATGAACCTGCTGAACAGCAGATGGGAGTGTCTGAGAGTGGCCAGCATGGAA AAGCAGAGCAACCTGCACAGAGTGCTCATGGACCTGCAGAATCAGAAACTGA AAGAACTGAATGACTGGCTGACCAAGACAGAAGAAAGGACTAGGAAGATGGA AGAGGAACCTCTGGGACCAGACCTGGAAGATCTGAAAAGACAGGTGCAGCAG CATAAGGTGCTGCAAGAGGACCTTGAGCAAGAGCAAGTCAGAGTGAACAGCC TGACACACATGGTGGTGGTTGTGGATGAGTCCTCTGGGGATCATGCCACAGC TGCTCTGGAAGAACAGCTGAAGGTGCTGGGAGACAGATGGGCCAACATCTGT AGGTGGACAGAGGATAGATGGGTGCTGCTCCAGGACATTCTGCTGAAGTGGC AGAGACTGACAGAGGAACAGTGCCTGTTTTCTGCCTGGCTCTCTGAGAAAGA GGATGCTGTCAACAAGATCCATACCACAGGCTTCAAGGATCAGAATGAGATG CTCAGCTCCCTGCAGAAACTGGCTGTGCTGAAGGCTGACCTGGAAAAGAAAA AGCAGTCCATGGGCAAGCTCTACAGCCTGAAGCAGGACCTGCTGTCTACCCT GAAGAACAAGTCTGTGACCCAGAAAACTGAGGCCTGGCTGGACAACTTTGCT AGATGCTGGGACAACCTGGTGCAGAAGCTGGAAAAGTCTACAGCCCAGATCA GCCAGCAACCTGATCTTGCCCCTGGCCTGACCACAATTGGAGCCTCTCCAAC ACAGACTGTGACCCTGGTTACCCAGCCAGTGGTCACCAAAGAGACAGCCATC AGCAAACTGGAAATGCCCAGCTCTCTGATGCTGGAAGTCCCCACACTGGAAA GGCTGCAAGAACTTCAAGAGGCCACAGATGAGCTGGACCTGAAGCTGAGACA GGCTGAAGTGATCAAAGGCAGCTGGCAGCCAGTTGGGGACCTGCTCATTGAT AGCCTGCAGGACCATCTGGAAAAAGTGAAAGCCCTGAGGGGAGAGATTGCCC CTCTGAAAGAAAATGTGTCCCATGTGAATGACCTGGCCAGACAGCTGACCAC ACTGGGAATCCAGCTGAGCCCCTACAACCTGAGCACCCTTGAGGACCTGAAC ACCAGGTGGAAGCTCCTCCAGGTGGCAGTGGAAGATAGAGTCAGGCAGCTGC ATGAGGCCCACAGAGATTTTGGACCAGCCAGCCAGCACTTTCTGTCTACCTC |
| | | TGTGCAAGGCCCCTGGGAGAGAGCTATCTCTCCTAACAAGGTGCCCTACTAC ATCAACCATGAGACACAGACCACCTGTTGGGATCACCCCAAGATGACAGAGC TGTACCAGAGTCTGGCAGACCTCAACAATGTCAGATTCAGTGCCTACAGGAC TGCCATGAAGCTCAGAAGGCTCCAGAAAGCTCTGTGCCTGGACCTGCTTTCC CTGAGTGCAGCTTGTGATGCCCTGGACCAGCACAATCTGAAGCAGAATGACC AGCCTATGGACATCCTCCAGATCATCAACTGCCTCACCACCATCTATGATAG GCTGGAACAAGAGCACAACAATCTGGTCAATGTGCCCCTGTGTGTGGACATG TGCCTGAATTGGCTGCTGAATGTGTATGACACAGGCAGAACAGGCAGGATCA GAGTCCTGTCCTTCAAGACAGGCATCATCTCCCTGTGCAAAGCCCACTTGGA GGACAAGTACAGATACCTGTTCAAGCAAGTGGCCTCCAGCACAGGCTTTTGT GACCAGAGAAGGCTGGGCCTGCTCCTGCATGACAGCATTCAGATCCCTAGAC AGCTGGGAGAAGTGGCTTCCTTTGGAGGCAGCAATATTGAGCCATCAGTCAG GTCCTGTTTTCAGTTTGCCAACAACAAGCCTGAGATTGAGGCTGCCCTGTTC CTGGACTGGATGAGACTTGAGCCTCAGAGCATGGTCTGGCTGCCTGTGCTTC ATAGAGTGGCTGCTGCTGAGACTGCCAAGCACCAGGCCAAGTGCAACATCTG CAAAGAGTGCCCCATCATTGGCTTCAGATACAGATCCCTGAAGCACTTCAAC TATGATATCTGCCAGAGCTGCTTCTTTAGTGGCAGGGTTGCCAAGGGCCACA AAATGCACTACCCCATGGTGGAATACTGCACCCCAACAACCTCTGGGGAAGA TGTTAGAGACTTTGCCAAGGTGCTGAAAAACAAGTTCAGGACCAAGAGATAC TTTGCTAAGCACCCCAGAATGGGCTACCTGCCTGTCCAGACAGTGCTTGAGG GTGACAACATGGAAACCTGATGAGTCGACAGGCCTAATAAAGAGCTCAGATG CATCGATCAGAGTGTGTTGGTTTTTTGTGTGGCTAGCTGCGGCCGCAGGAAC CCCTAGTGATGGAGTTGGCCACTCCCTCTCTGCGCGCTCGCTCGCTCACTGA GGCCGGGCGACCAAAGGTCGCCCGACGCCCGGGCTTTGCCCGGGCGGCCTCA GTGAGCGAGCGAGCGCGCAG |
| 67 | Dys1 Nucleotide Sequence | ATGCTTTGGTGGGAAGAGGTGGAAGATTGCTATGAGAGGGAAGATGTGCAGA AGAAAACCTTCACCAAATGGGTCAATGCCCAGTTCAGCAAGTTTGGCAAGCA GCACATTGAGAACCTGTTCAGTGACCTGCAGGATGGCAGAAGGCTGCTGGAT CTGCTGGAAGGCCTGACAGGCCAGAAGCTGCCTAAAGAGAAGGGCAGCACAA GAGTGCATGCCCTGAACAATGTGAACAAGGCCCTGAGAGTGCTGCAGAACAA CAATGTGGACCTGGTCAATATTGGCAGCACAGACATTGTGGATGGCAACCAC AAGCTGACCCTGGGCCTGATCTGGAACATCATCCTGCACTGGCAAGTGAAGA ATGTGATGAAGAACATCATGGCTGGCCTGCAGCAGACCAACTCTGAGAAGAT CCTGCTGAGCTGGGTCAGACAGAGCACCAGAAACTACCCTCAAGTGAATGTG ATCAACTTCACCACCTCTTGGAGTGATGGACTGGCCCTGAATGCCCTGATCC ACAGCCACAGACCTGACCTGTTTGACTGGAACTCTGTTGTGTGCCAGCAGTC TGCCACACAGAGACTGGAACATGCCTTCAACATTGCCAGATACCAGCTGGGA ATTGAGAAACTGCTGGACCCTGAGGATGTGGACACCACCTATCCTGACAAGA AATCCATCCTCATGTACATCACCAGCCTGTTCCAGGTGCTGCCCCAGCAAGT GTCCATTGAGGCCATTCAAGAGGTTGAGATGCTGCCCAGACCTCCTAAAGTG ACCAAAGAGGAACACTTCCAGCTGCACCACCAGATGCACTACTCTCAGCAGA TCACAGTGTCTCTGGCCCAGGGATATGAGAGAACAAGCAGCCCCAAGCCTAG GTTCAAGAGCTATGCCTACACACAGGCTGCCTATGTGACCACATCTGACCCC ACAAGAAGCCCATTTCCAAGCCAGCATCTGGAAGCCCCTGAGGACAAGAGCT TTGGCAGCAGCCTGATGGAATCTGAAGTGAACCTGGATAGATACCAGACAGC CCTGGAAGAAGTGCTGTCCTGGCTGCTGTCTGCTGAGGATACACTGCAGGCT CAGGGTGAAATCAGCAATGATGTGGAAGTGGTCAAGGACCAGTTTCACACCC ATGAGGGCTACATGATGGACCTGACAGCCCACCAGGGCAGAGTGGGAAATAT CCTGCAGCTGGGCTCCAAGCTGATTGGCACAGGCAAGCTGTCTGAGGATGAA GAGACAGAGGTGCAAGAGCAGATGAACCTGCTGAACAGCAGATGGGAGTGTC TGAGAGTGGCCAGCATGGAAAAGCAGAGCAACCTGCACAGAGTGCTCATGGA CCTGCAGAATCAGAAACTGAAAGAACTGAATGACTGGCTGACCAAGACAGAA GAAAGGACTAGGAAGATGGAAGAGGAACCTCTGGGACCAGACCTGGAAGATC TGAAAAGACAGGTGCAGCAGCATAAGGTGCTGCAAGAGGACCTTGAGCAAGA GCAAGTCAGAGTGAACAGCCTGACACACATGGTGGTGGTTGTGGATGAGTCC TCTGGGGATCATGCCACAGCTGCTCTGGAAGAACAGCTGAAGGTGCTGGGAG ACAGATGGGCCAACATCTGTAGGTGGACAGAGGATAGATGGGTGCTGCTCCA GGACATTCTGCTGAAGTGGCAGAGACTGACAGAGGAACAGTGCCTGTTTTCT |
| | | GCCTGGCTCTCTGAGAAAGAGGATGCTGTCAACAAGATCCATACCACAGGCT TCAAGGATCAGAATGAGATGCTCAGCTCCCTGCAGAAACTGGCTGTGCTGAA GGCTGACCTGGAAAAGAAAAAGCAGTCCATGGGCAAGCTCTACAGCCTGAAG CAGGACCTGCTGTCTACCCTGAAGAACAAGTCTGTGACCCAGAAAACTGAGG CCTGGCTGGACAACTTTGCTAGATGCTGGGACAACCTGGTGCAGAAGCTGGA AAAGTCTACAGCCCAGATCAGCCAGCAACCTGATCTTGCCCCTGGCCTGACC ACAATTGGAGCCTCTCCAACACAGACTGTGACCCTGGTTACCCAGCCAGTGG TCACCAAAGAGACAGCCATCAGCAAACTGGAAATGCCCAGCTCTCTGATGCT GGAAGTCCCCACACTGGAAAGGCTGCAAGAACTTCAAGAGGCCACAGATGAG CTGGACCTGAAGCTGAGACAGGCTGAAGTGATCAAAGGCAGCTGGCAGCCAG TTGGGGACCTGCTCATTGATAGCCTGCAGGACCATCTGGAAAAAGTGAAAGC CCTGAGGGGAGAGATTGCCCCTCTGAAAGAAAATGTGTCCCATGTGAATGAC CTGGCCAGACAGCTGACCACACTGGGAATCCAGCTGAGCCCCTACAACCTGA GCACCCTTGAGGACCTGAACACCAGGTGGAAGCTCCTCCAGGTGGCAGTGGA AGATAGAGTCAGGCAGCTGCATGAGGCCCACAGAGATTTTGGACCAGCCAGC CAGCACTTTCTGTCTACCTCTGTGCAAGGCCCCTGGGAGAGAGCTATCTCTC CTAACAAGGTGCCCTACTACATCAACCATGAGACACAGACCACCTGTTGGGA TCACCCCAAGATGACAGAGCTGTACCAGAGTCTGGCAGACCTCAACAATGTC AGATTCAGTGCCTACAGGACTGCCATGAAGCTCAGAAGGCTCCAGAAAGCTC TGTGCCTGGACCTGCTTTCCCTGAGTGCAGCTTGTGATGCCCTGGACCAGCA CAATCTGAAGCAGAATGACCAGCCTATGGACATCCTCCAGATCATCAACTGC CTCACCACCATCTATGATAGGCTGGAACAAGAGCACAACAATCTGGTCAATG TGCCCCTGTGTGTGGACATGTGCCTGAATTGGCTGCTGAATGTGTATGACAC AGGCAGAACAGGCAGGATCAGAGTCCTGTCCTTCAAGACAGGCATCATCTCC CTGTGCAAAGCCCACTTGGAGGACAAGTACAGATACCTGTTCAAGCAAGTGG CCTCCAGCACAGGCTTTTGTGACCAGAGAAGGCTGGGCCTGCTCCTGCATGA CAGCATTCAGATCCCTAGACAGCTGGGAGAAGTGGCTTCCTTTGGAGGCAGC AATATTGAGCCATCAGTCAGGTCCTGTTTTCAGTTTGCCAACAACAAGCCTG AGATTGAGGCTGCCCTGTTCCTGGACTGGATGAGACTTGAGCCTCAGAGCAT GGTCTGGCTGCCTGTGCTTCATAGAGTGGCTGCTGCTGAGACTGCCAAGCAC CAGGCCAAGTGCAACATCTGCAAAGAGTGCCCCATCATTGGCTTCAGATACA GATCCCTGAAGCACTTCAACTATGATATCTGCCAGAGCTGCTTCTTTAGTGG CAGGGTTGCCAAGGGCCACAAAATGCACTACCCCATGGTGGAATACTGCACC CCAACAACCTCTGGGGAAGATGTTAGAGACTTTGCCAAGGTGCTGAAAAACA AGTTCAGGACCAAGAGATACTTTGCTAAGCACCCCAGAATGGGCTACCTGCC TGTCCAGACAGTGCTTGAGGGTGACAACATGGAAACCCCTGTGACACTGATC AATTTCTGGCCAGTGGACTCTGCCCCTGCCTCAAGTCCACAGCTGTCCCATG ATGACACCCACAGCAGAATTGAGCACTATGCCTCCAGACTGGCAGAGATGGA AAACAGCAATGGCAGCTACCTGAATGATAGCATCAGCCCCAATGAGAGCATT GATGATGAGCATCTGCTGATCCAGCACTACTGTCAGTCCCTGAACCAGGACT CTCCACTGAGCCAGCCTAGAAGCCCTGCTCAGATCCTGATCAGCCTTGAGTC TGAGGAAAGGGGAGAGCTGGAAAGAATCCTGGCAGATCTTGAGGAAGAGAAC AGAAACCTGCAGGCAGAGTATGACAGGCTCAAACAGCAGCATGAGCACAAGG GACTGAGCCCTCTGCCTTCTCCTCCTGAAATGATGCCCACCTCTCCACAGTC TCCAAGGTGATGA |
| 68 | Dys3 Nucleotide Sequence | ATGCTTTGGTGGGAAGAGGTGGAAGATTGCTATGAGAGGGAAGATGTGCAGA AGAAAACCTTCACCAAATGGGTCAATGCCCAGTTCAGCAAGTTTGGCAAGCA GCACATTGAGAACCTGTTCAGTGACCTGCAGGATGGCAGAAGGCTGCTGGAT CTGCTGGAAGGCCTGACAGGCCAGAAGCTGCCTAAAGAGAAGGGCAGCACAA GAGTGCATGCCCTGAACAATGTGAACAAGGCCCTGAGAGTGCTGCAGAACAA CAATGTGGACCTGGTCAATATTGGCAGCACAGACATTGTGGATGGCAACCAC AAGCTGACCCTGGGCCTGATCTGGAACATCATCCTGCACTGGCAAGTGAAGA ATGTGATGAAGAACATCATGGCTGGCCTGCAGCAGACCAACTCTGAGAAGAT CCTGCTGAGCTGGGTCAGACAGAGCACCAGAAACTACCCTCAAGTGAATGTG ATCAACTTCACCACCTCTTGGAGTGATGGACTGGCCCTGAATGCCCTGATCC ACAGCCACAGACCTGACCTGTTTGACTGGAACTCTGTTGTGTGCCAGCAGTC TGCCACACAGAGACTGGAACATGCCTTCAACATTGCCAGATACCAGCTGGGA ATTGAGAAACTGCTGGACCCTGAGGATGTGGACACCACCTATCCTGACAAGA |
| | | AATCCATCCTCATGTACATCACCAGCCTGTTCCAGGTGCTGCCCCAGCAAGT GTCCATTGAGGCCATTCAAGAGGTTGAGATGCTGCCCAGACCTCCTAAAGTG ACCAAAGAGGAACACTTCCAGCTGCACCACCAGATGCACTACTCTCAGCAGA TCACAGTGTCTCTGGCCCAGGGATATGAGAGAACAAGCAGCCCCAAGCCTAG GTTCAAGAGCTATGCCTACACACAGGCTGCCTATGTGACCACATCTGACCCC ACAAGAAGCCCATTTCCAAGCCAGCATCTGGAAGCCCCTGAGGACAAGAGCT TTGGCAGCAGCCTGATGGAATCTGAAGTGAACCTGGATAGATACCAGACAGC CCTGGAAGAAGTGCTGTCCTGGCTGCTGTCTGCTGAGGATACACTGCAGGCT CAGGGTGAAATCAGCAATGATGTGGAAGTGGTCAAGGACCAGTTTCACACCC ATGAGGGCTACATGATGGACCTGACAGCCCACCAGGGCAGAGTGGGAAATAT CCTGCAGCTGGGCTCCAAGCTGATTGGCACAGGCAAGCTGTCTGAGGATGAA GAGACAGAGGTGCAAGAGCAGATGAACCTGCTGAACAGCAGATGGGAGTGTC TGAGAGTGGCCAGCATGGAAAAGCAGAGCAACCTGCACAGAGTGCTCATGGA CCTGCAGAATCAGAAACTGAAAGAACTGAATGACTGGCTGACCAAGACAGAA GAAAGGACTAGGAAGATGGAAGAGGAACCTCTGGGACCAGACCTGGAAGATC TGAAAAGACAGGTGCAGCAGCATAAGGTGCTGCAAGAGGACCTTGAGCAAGA GCAAGTCAGAGTGAACAGCCTGACACACATGGTGGTGGTTGTGGATGAGTCC TCTGGGGATCATGCCACAGCTGCTCTGGAAGAACAGCTGAAGGTGCTGGGAG ACAGATGGGCCAACATCTGTAGGTGGACAGAGGATAGATGGGTGCTGCTCCA GGACATTCTGCTGAAGTGGCAGAGACTGACAGAGGAACAGTGCCTGTTTTCT GCCTGGCTCTCTGAGAAAGAGGATGCTGTCAACAAGATCCATACCACAGGCT TCAAGGATCAGAATGAGATGCTCAGCTCCCTGCAGAAACTGGCTGTGCTGAA GGCTGACCTGGAAAAGAAAAAGCAGTCCATGGGCAAGCTCTACAGCCTGAAG CAGGACCTGCTGTCTACCCTGAAGAACAAGTCTGTGACCCAGAAAACTGAGG CCTGGCTGGACAACTTTGCTAGATGCTGGGACAACCTGGTGCAGAAGCTGGA AAAGTCTACAGCCCAGATCAGCCAGCAACCTGATCTTGCCCCTGGCCTGACC ACAATTGGAGCCTCTCCAACACAGACTGTGACCCTGGTTACCCAGCCAGTGG TCACCAAAGAGACAGCCATCAGCAAACTGGAAATGCCCAGCTCTCTGATGCT GGAAGTCCCCACACTGGAAAGGCTGCAAGAACTTCAAGAGGCCACAGATGAG CTGGACCTGAAGCTGAGACAGGCTGAAGTGATCAAAGGCAGCTGGCAGCCAG TTGGGGACCTGCTCATTGATAGCCTGCAGGACCATCTGGAAAAAGTGAAAGC CCTGAGGGGAGAGATTGCCCCTCTGAAAGAAAATGTGTCCCATGTGAATGAC CTGGCCAGACAGCTGACCACACTGGGAATCCAGCTGAGCCCCTACAACCTGA GCACCCTTGAGGACCTGAACACCAGGTGGAAGCTCCTCCAGGTGGCAGTGGA AGATAGAGTCAGGCAGCTGCATGAGGCCCACAGAGATTTTGGACCAGCCAGC CAGCACTTTCTGTCTACCTCTGTGCAAGGCCCCTGGGAGAGAGCTATCTCTC CTAACAAGGTGCCCTACTACATCAACCATGAGACACAGACCACCTGTTGGGA TCACCCCAAGATGACAGAGCTGTACCAGAGTCTGGCAGACCTCAACAATGTC AGATTCAGTGCCTACAGGACTGCCATGAAGCTCAGAAGGCTCCAGAAAGCTC TGTGCCTGGACCTGCTTTCCCTGAGTGCAGCTTGTGATGCCCTGGACCAGCA CAATCTGAAGCAGAATGACCAGCCTATGGACATCCTCCAGATCATCAACTGC CTCACCACCATCTATGATAGGCTGGAACAAGAGCACAACAATCTGGTCAATG TGCCCCTGTGTGTGGACATGTGCCTGAATTGGCTGCTGAATGTGTATGACAC AGGCAGAACAGGCAGGATCAGAGTCCTGTCCTTCAAGACAGGCATCATCTCC CTGTGCAAAGCCCACTTGGAGGACAAGTACAGATACCTGTTCAAGCAAGTGG CCTCCAGCACAGGCTTTTGTGACCAGAGAAGGCTGGGCCTGCTCCTGCATGA CAGCATTCAGATCCCTAGACAGCTGGGAGAAGTGGCTTCCTTTGGAGGCAGC AATATTGAGCCATCAGTCAGGTCCTGTTTTCAGTTTGCCAACAACAAGCCTG AGATTGAGGCTGCCCTGTTCCTGGACTGGATGAGACTTGAGCCTCAGAGCAT GGTCTGGCTGCCTGTGCTTCATAGAGTGGCTGCTGCTGAGACTGCCAAGCAC CAGGCCAAGTGCAACATCTGCAAAGAGTGCCCCATCATTGGCTTCAGATACA GATCCCTGAAGCACTTCAACTATGATATCTGCCAGAGCTGCTTCTTTAGTGG CAGGGTTGCCAAGGGCCACAAAATGCACTACCCCATGGTGGAATACTGCACC CCAACAACCTCTGGGGAAGATGTTAGAGACTTTGCCAAGGTGCTGAAAAACA AGTTCAGGACCAAGAGATACTTTGCTAAGCACCCCAGAATGGGCTACCTGCC TGTCCAGACAGTGCTTGAGGGTGACAACATGGAAACC |
| 69 | Dys5 Nucleotide Sequence | ATGCTTTGGTGGGAAGAGGTGGAAGATTGCTATGAGAGGGAAGATGTGCAGA AGAAAACCTTCACCAAATGGGTCAATGCCCAGTTCAGCAAGTTTGGCAAGCA |
| | | GCACATTGAGAACCTGTTCAGTGACCTGCAGGATGGCAGAAGGCTGCTGGAT CTGCTGGAAGGCCTGACAGGCCAGAAGCTGCCTAAAGAGAAGGGCAGCACAA GAGTGCATGCCCTGAACAATGTGAACAAGGCCCTGAGAGTGCTGCAGAACAA CAATGTGGACCTGGTCAATATTGGCAGCACAGACATTGTGGATGGCAACCAC AAGCTGACCCTGGGCCTGATCTGGAACATCATCCTGCACTGGCAAGTGAAGA ATGTGATGAAGAACATCATGGCTGGCCTGCAGCAGACCAACTCTGAGAAGAT CCTGCTGAGCTGGGTCAGACAGAGCACCAGAAACTACCCTCAAGTGAATGTG ATCAACTTCACCACCTCTTGGAGTGATGGACTGGCCCTGAATGCCCTGATCC ACAGCCACAGACCTGACCTGTTTGACTGGAACTCTGTTGTGTGCCAGCAGTC TGCCACACAGAGACTGGAACATGCCTTCAACATTGCCAGATACCAGCTGGGA ATTGAGAAACTGCTGGACCCTGAGGATGTGGACACCACCTATCCTGACAAGA AATCCATCCTCATGTACATCACCAGCCTGTTCCAGGTGCTGCCCCAGCAAGT GTCCATTGAGGCCATTCAAGAGGTTGAGATGCTGCCCAGACCTCCTAAAGTG ACCAAAGAGGAACACTTCCAGCTGCACCACCAGATGCACTACTCTCAGCAGA TCACAGTGTCTCTGGCCCAGGGATATGAGAGAACAAGCAGCCCCAAGCCTAG GTTCAAGAGCTATGCCTACACACAGGCTGCCTATGTGACCACATCTGACCCC ACAAGAAGCCCATTTCCAAGCCAGCATCTGGAAGCCCCTGAGGACAAGAGCT TTGGCAGCAGCCTGATGGAATCTGAAGTGAACCTGGATAGATACCAGACAGC CCTGGAAGAAGTGCTGTCCTGGCTGCTGTCTGCTGAGGATACACTGCAGGCT CAGGGTGAAATCAGCAATGATGTGGAAGTGGTCAAGGACCAGTTTCACACCC ATGAGGGCTACATGATGGACCTGACAGCCCACCAGGGCAGAGTGGGAAATAT CCTGCAGCTGGGCTCCAAGCTGATTGGCACAGGCAAGCTGTCTGAGGATGAA GAGACAGAGGTGCAAGAGCAGATGAACCTGCTGAACAGCAGATGGGAGTGTC TGAGAGTGGCCAGCATGGAAAAGCAGAGCAACCTGCACAGAGTGCTCATGGA CCTGCAGAATCAGAAACTGAAAGAACTGAATGACTGGCTGACCAAGACAGAA GAAAGGACTAGGAAGATGGAAGAGGAACCTCTGGGACCAGACCTGGAAGATC TGAAAAGACAGGTGCAGCAGCATAAGGTGCTGCAAGAGGACCTTGAGCAAGA GCAAGTCAGAGTGAACAGCCTGACACACATGGTGGTGGTTGTGGATGAGTCC TCTGGGGATCATGCCACAGCTGCTCTGGAAGAACAGCTGAAGGTGCTGGGAG ACAGATGGGCCAACATCTGTAGGTGGACAGAGGATAGATGGGTGCTGCTCCA GGACATTCTGCTGAAGTGGCAGAGACTGACAGAGGAACAGTGCCTGTTTTCT GCCTGGCTCTCTGAGAAAGAGGATGCTGTCAACAAGATCCATACCACAGGCT TCAAGGATCAGAATGAGATGCTCAGCTCCCTGCAGAAACTGGCTGTGCTGAA GGCTGACCTGGAAAAGAAAAAGCAGTCCATGGGCAAGCTCTACAGCCTGAAG CAGGACCTGCTGTCTACCCTGAAGAACAAGTCTGTGACCCAGAAAACTGAGG CCTGGCTGGACAACTTTGCTAGATGCTGGGACAACCTGGTGCAGAAGCTGGA AAAGTCTACAGCCCAGATCAGCCAGCAACCTGATCTTGCCCCTGGCCTGACC ACAATTGGAGCCTCTCCAACACAGACTGTGACCCTGGTTACCCAGCCAGTGG TCACCAAAGAGACAGCCATCAGCAAACTGGAAATGCCCAGCTCTCTGATGCT GGAAGTCCCCACACTGGAAAGGCTGCAAGAACTTCAAGAGGCCACAGATGAG CTGGACCTGAAGCTGAGACAGGCTGAAGTGATCAAAGGCAGCTGGCAGCCAG TTGGGGACCTGCTCATTGATAGCCTGCAGGACCATCTGGAAAAAGTGAAAGC CCTGAGGGGAGAGATTGCCCCTCTGAAAGAAAATGTGTCCCATGTGAATGAC CTGGCCAGACAGCTGACCACACTGGGAATCCAGCTGAGCCCCTACAACCTGA GCACCCTTGAGGACCTGAACACCAGGTGGAAGCTCCTCCAGGTGGCAGTGGA AGATAGAGTCAGGCAGCTGCATGAGGCCCACAGAGATTTTGGACCAGCCAGC CAGCACTTTCTGTCTACCTCTGTGCAAGGCCCCTGGGAGAGAGCTATCTCTC CTAACAAGGTGCCCTACTACATCAACCATGAGACACAGACCACCTGTTGGGA TCACCCCAAGATGACAGAGCTGTACCAGAGTCTGGCAGACCTCAACAATGTC AGATTCAGTGCCTACAGGACTGCCATGAAGCTCAGAAGGCTCCAGAAAGCTC TGTGCCTGGACCTGCTTTCCCTGAGTGCAGCTTGTGATGCCCTGGACCAGCA CAATCTGAAGCAGAATGACCAGCCTATGGACATCCTCCAGATCATCAACTGC CTCACCACCATCTATGATAGGCTGGAACAAGAGCACAACAATCTGGTCAATG TGCCCCTGTGTGTGGACATGTGCCTGAATTGGCTGCTGAATGTGTATGACAC AGGCAGAACAGGCAGGATCAGAGTCCTGTCCTTCAAGACAGGCATCATCTCC CTGTGCAAAGCCCACTTGGAGGACAAGTACAGATACCTGTTCAAGCAAGTGG CCTCCAGCACAGGCTTTTGTGACCAGAGAAGGCTGGGCCTGCTCCTGCATGA CAGCATTCAGATCCCTAGACAGCTGGGAGAAGTGGCTTCCTTTGGAGGCAGC |
| | | AATATTGAGCCATCAGTCAGGTCCTGTTTTCAGTTTGCCAACAACAAGCCTG AGATTGAGGCTGCCCTGTTCCTGGACTGGATGAGACTTGAGCCTCAGAGCAT GGTCTGGCTGCCTGTGCTTCATAGAGTGGCTGCTGCTGAGACTGCCAAGCAC CAGGCCAAGTGCAACATCTGCAAAGAGTGCCCCATCATTGGCTTCAGATACA GATCCCTGAAGCACTTCAACTATGATATCTGCCAGAGCTGCTTCTTTAGTGG CAGGGTTGCCAAGGGCCACAAAATGCACTACCCCATGGTGGAATACTGCACC CCAACAACCTCTGGGGAAGATGTTAGAGACTTTGCCAAGGTGCTGAAAAACA AGTTCAGGACCAAGAGATACTTTGCTAAGCACCCCAGAATGGGCTACCTGCC TGTCCAGACAGTGCTTGAGGGTGACAACATGGAAACCCCTGTGACACTGATC AATTTCTGGCCAGTGGACTCTGCCCCTGCCTCAAGTCCACAGCTGTCCCATG ATGACACCCACAGCAGAATTGAGCACTATGCCTCCAGACTGGCAGAGATGGA AAACAGCAATGGCAGCTACCTGAATGATAGCATCAGCCCCAATGAGAGCATT GATGATGAGCATCTGCTGATCCAGCACTACTGTCAGTCCCTGAACCAGGACT CTCCACTGAGCCAGCCTAGAAGCCCTGCTCAGATCCTGATCAGCCTTGAGTC TTGATGA |
| 70 | Codon-Optimized Microdystrophin from WO 2023018854 (Solid Biosciences) | atgctgtggtgggaggaagtggaagattgctacgagcgcgaggacgtgcaga agaaaaccttcaccaaatgggtcaacgcccagttcagcaagttcggcaagca gcacatcgagaacctgttcagcgacctgcaggacggcagacggctgctggat ctgctggaaggcctgaccggacagaagctgcccaaagagaagggcagcacca gagtgcacgccctgaacaacgtgaacaaggccctgcgggtgctgcagaacaa caaTgtggacctGgtgaacatTggcagcacAgacatTgtggaTggcaaccac aagctgaccctgggcctgatctggaacatcatcctgcactggcaagtgaaga acgtgatgaagaacatcatggccggcctgcagcagaccaacagcgagaagat cctgctgagctgggtgcgccagagcaccagaaactacccccaagtgaacgtg atcaacttcaccacctcttggagcgacggcctggccctgaatgccctgatcc acagccacagacccgacctgttTgactggaacagTgtGgtgtgtcagcagag cgccacccagaggctggaacacgccttcaatatcgccagataccagctgggc atTgagaagctgctggaccccgaggatgtggacaccacctaccccgacaaga aatccatcctgatgtatatcaccagcctgttccaggtgctgcctcagcaggt gtccatcgaggccatccaggaagtggaaatgctgcccagacccccccaaagtg accaaagaggaacacttccagctgcaccaccagatgcactactctcagcaga tcaccgtgtccctggcccagggctacgagagaaccagcagccccaagccccg gttcaagagctacgcctatacccaggccgcctacgtgaccaccagcgaccct accagaagcccattccccagccagcatctggaagcccccgaggacaagagct tcggcagcagcctgatggaaagcgaagtgaacctggatagataccagaccgc cctggaagaggtgctgtcctggctgctgagcgccgaggatacactgcaggct cagggcgagatcagcaaTgaTgtggaagtGgtgaaggaccagttccacaccc acgagggctacatgatggacctgacagcccaccagggcagagtgggcaacat tctgcagctgggctccaagctgatcggcaccggcaagctgagcgaggacgaa gagacagaggtgcaggaacagatgaacctgctgaacagcagatggagtgcc tgagagtggccagcatggaaaagcagagcaacctgcacagctacgtgcccag cacctacctgcgccaaggacttcgaggatctgttcaagcaggaagagagcct gaagaatatcaaggactctctgcagcagtccagcggcagaatcgacatcatc cacagcaagaaaacagccgccctgcagtccgccacccccgtggaaagagtga agctgcaggaagccctgtcccagctggacttccagtgggagaaagtgaacaa gatgtacaaggaccggcagggcagattTgaccgcagTgtggaaaagtggAgg AggttccactacgacatcaagatcttcaaccagtggctgacAgaggccgagc agttcctgagaaagacccagatccccgagaactgggagcacgccaagtacaa gtggtatctgaaagaactgcaggatggcatTggccagagacagacAgtGgtg cggacactgaatgccaccggcgaggaaatcatccagcagagcagcaagaccg acgccagtattctgcaggaaaagctgggcagcctgaacctgagatggcagga agtgtgcaagcagctgtccgaccggaagaagagactggaagaacagagTgac cagtggaagcggctgcatctgtcactgcaggaactgctGgtgtggctgcagc tgaaggaTgaTgagctgagcagacaggcccctatTggcggcgattttcccgc AgtgcagaaacagaacgaTgtgcaccgggccttcaagagagagctgaaaaca aaagaaccAgtgatcatgagcaccctggaaacAgtgcggatctttctgaccg agcagcccctggaaggactggaaaaactgtaccaggaacccagagagctgcc ccctgaagaacgggcccagaacgtgaccagactgctgAggaagcaggccgag |
| | | gaagtgaacacAgaatggagaagctgaacctgcactcTgcTgactggcagA ggaagatTgaTgagacactggaacggctgcaggaactgcaggaggccacAga cgagctggacctgaaactgagacaggccgaagtgatcaagggcagctggcag ccagtgggcgacctgctgatcgacagcctgcaggatcacctggaaaaagtga aagccctgagaggcgagatTgccccccctgaaagaaaaTgtg[?]cccaTgtgaa cgacctggcccggcagctgacaacactgggcatccagctgagcccctacaac ctgtccacac[?]ggaagatctgaacacccggtggaaactgctgcaggtggccg tggaagatagagtgcggcagctgcacgaggcccacagagat[?][?]tggccctgc ctcccagcac[?][?]cctgagcacatctgtgcagggcccctgggagagagccatc tcccccaacaaggtgccctactacatcaaccacgagacacagaccacctgtt gggaccaccccaagatgacAgagctgtaccagagcctggccgacctgaacaa tgtgAggttcagTgcctacAggaccgccatgaagctgcggagactgcagaaa gctctgtgcc[?]ggacctgctgtccctgtccgccgcttgtga[?]gccctggacc agcacaacctgaagcagaacgaccagcccatggatatcctgcagatcatcaa ctgcctgaccaccatctacgaccgcctggaacaggaacacaacaacctGgtg aatgtgcccc[?]gtgTgtggacatgtgcctgaattggctgctgaatgtgtacg acaccggccggacaggccggatcagagtgctgagcttcaagaccggcatcat cagcctgtgcaaggcccacctggaagataagtaccgctacc[?]gttcaaacag gtggccagctccaccggcttttgcgaccagagaaggctgggcctgctgctgc acgacagcatccagatccctagacagctgggcgaggtggcc[?]ctttTggcgg cagcaatatTgagcctagTgtgcggagctgcttccagttTgccaacaacaag cccgagatTgaggccgccctgttcctggactggatgcggctggaaccccaga gcatggtgtggctgcctgtgctgcatagagtggccgctgccgagacagccaa gcaccaggccaagtgcaacatctgcaaagagtgccccatca[?]cggcttccgg tacagaagcc[?]gaagcacttcaactacgatatctgccagagctgctttttca gcggacgggtggccaagggccacaaaatgcactaccccatggtggaatactg cacccccaccacctccggggaggatgtgcgggattttgccaaggtgctgaaa aacaagttccggaccaagcgctactTgccaaacacccccggatgggctatc tgcccgtgcagacagtgctggaaggcgacaacatggaaaccgacaccatgta g |
| 71 | SEQ ID NO: 7 of WO 2017/181015 A1 (encodes SEQ ID NO: 57 of Table 5) | ATGCTGTGGTGGGAGGAGGTGGAGGATTGTTATGAAAGGGAGGACGTGCAGA AGAAGACTTTTACCAAGTGGGTGAACGCTCAGTTCAGCAAATTTGGGAAGCA GCACATCGAGAATCTGTTTTCCGACCTGCAGGATGGGAGACGGCTGCTGGAT CTGCTGGAAGGACTGACTGGCCAGAAGCTGCCCAAAGAGAAGGGGAGCACTA GGGTGCACGCCCTGAACAACGTGAACAAAGCTCTGAGAGTGCTGCAGAACAA CAACGTGGATCTGGTGAATATTGGCAGTACTGATATCGTGGACGGGAACCAC AAACTGACACTGGGCCTGATCTGGAACATTATTCTGCACTGGCAGGTGAAAA ATGTGATGAAGAACATCATGGCCGGGCTGCAGCAGACCAATTCCGAGAAGAT CCTGCTGTCTTGGGTGCGGCAGAGCACCCGCAACTATCCCCAGGTGAACGTG ATTAACTTCACTACATCCTGGAGCGACGGGCTGGCCCTGAATGCTCTGATTC ACAGCCACAGGCCTGATCTGTTCGACTGGAATAGCGTGGTGTGCCAGCAGTC TGCCACACAGCGCCTGGAACATGCCTTCAATATCGCTCGGTACCAGCTGGGG ATCGAAAAACTGCTGGACCCAGAGGATGTGGACACTACATACCCAGATAAAA AGTCTATTCTGATGTACATTACTAGCCTGTTCCAGGTGCTGCCACAGCAGGT GTCTATTGAAGCCATTCAGGAGGTGGAAATGCTGCCCCGCCCCCCCAAAGTG ACTAAAGAGGAGCATTTTCAGCTGCATCATCAGATGCATTACAGCCAGCAGA TTACCGTGAGCCTGGCTCAGGGATATGAGCGCACCAGTAGTCCAAAACCACG GTTCAAGTCCTACGCTTATACCCAGGCTGCCTACGTGACAACTAGCGACCCT ACTAGATCCCCCTTTCCATCCCAGCACCTGGAGGCCCCAGAGGACAAGAGCT TTGGGTCCAGCCTGATGGAAAGCGAGGTGAATCTGGATCGGTACCAGACAGC CCTGGAGGAGGTGCTGAGCTGGCTGCTGAGTGCTGAAGACACACTGCAGGCC CAGGGCGAAATTTCCAATGACGTGGAAGTGGTGAAGGATCAGTTCCACACAC ACGAGGGCTATATGATGGACCTGACAGCTCACCAGGGGCGCGTGGGCAATAT CCTGCAGCTGGGCTCTAAACTGATCGGCACCGGGAAACTGAGTGAGGACGAG GAAACAGAAGTGCAGGAGCAGATGAACCTGCTGAACAGCCGCTGGGAGTGTC TGAGAGTGGCTAGTATGGAGAAGCAGTCCAACCTGCACCGGGTGCTGATGGA CCTGCAGAACCAGAAACTGAAAGAGCTGAACGACTGGCTGACAAAGACTGAG GAACGCACAAGGAAGATGGAGGAGGAGCCACTGGGACCCGACCTGGAGGATC |
| | | TGAAGAGACAGGTGCAGCAGCATAAGGTGCTGCAGGAGGATCTGGAACAGGA GCAGGTGCGGGTGAACTCCCTGACACATATGGTGGTGGTGGTGGACGAATCT AGTGGAGATCACGCCACCGCCGCCCTGGAGGAACAGCTGAAGGTGCTGGGGG ACCGGTGGGCCAACATTTGCCGGTGGACCGAGGACAGGTGGGTGCTGCTGCA GGACATCCTGCTGAAATGGCAGAGGCTGACCGAGGAGCAGTGTCTGTTTAGT GCTTGGCTGAGCGAGAAAGAGGACGCCGTGAACAAGATCCACACAACCGGCT TTAAGGATCAGAACGAAATGCTGTCTAGCCTGCAGAAACTGGCTGTGCTGAA GGCCGATCTGGAGAAAAAGAAGCAGAGCATGGGCAAACTGTATAGCCTGAAA CAGGACCTGCTGAGCACCCTGAAGAACAAGAGCGTGACCCAGAAGACAGAAG CCTGGCTGGATAACTTTGCCCGCTGCTGGGACAACCTGGTGCAGAAACTGGA GAAAAGTACAGCTCAGATCTCTCAGGCTGTGACCACAACCCAGCCTAGCCTG ACCCAGACAACCGTGATGGAAACCGTGACCACCGTGACAACCCGCGAACAGA TCCTGGTGAAACATGCCCAGGAAGAGCTGCCACCTCCACCTCCCCAGAAGAA GAGAACCCTGGAGCGGCTGCAGGAGCTGCAGGAAGCCACTGACGAACTGGAC CTGAAGCTGAGGCAGGCCGAAGTGATTAAGGGGTCTTGGCAGCCTGTGGGCG ATCTGCTGATTGATTCCCTGCAGGACCACCTGGAAAAGGTGAAGGCTCTGAG AGGCGAAATTGCTCCACTGAAGGAGAACGTGAGTCATGTGAACGATCTGGCT AGACAGCTGACAACACTGGGCATCCAGCTGAGCCCATACAATCTGAGCACAC TGGAGGACCTGAATACCAGGTGGAAGCTGCTGCAGGTGGCTGTGGAAGACCG GGTGCGGCAGCTGCATGAGGCCCATCGCGACTTCGGACCAGCCAGCCAGCAC TTTCTGAGCACATCCGTGCAGGGGCCCTGGGAGAGGGCCATTTCTCCCAACA AGGTGCCCTACTATATTAATCACGAGACCCAGACCACTTGTTGGGACCATCC CAAGATGACAGAACTGTACCAGTCCCTGGCCGATCTGAACAACGTGAGGTTT AGCGCTTACAGAACCGCTATGAAGCTGAGACGGCTGCAGAAGGCCCTGTGCC TGGATCTGCTGTCCCTGTCCGCCGCCTGCGATGCCCTGGATCAGCATAATCT GAAGCAGAACGATCAGCCAATGGATATCCTGCAGATCATCAACTGCCTGACC ACTATCTACGACAGGCTGGAGCAGGAGCACAACAACCTGGTGAACGTGCCTC TGTGCGTGGATATGTGCCTGAACTGGCTGCTGAACGTGTATGACACTGGGCG CACCGGCCGGATCAGAGTGCTGAGTTTTAAAACTGGGATTATCTCCCTGTGT AAGGCCCACCTGGAGGACAAGTACAGGTACCTGTTCAAGCAGGTGGCTAGTA GCACTGGATTTTGTGACCAGCGCCGCCTGGGACTGCTGCTGCATGATAGTAT CCAGATTCCTAGACAGCTGGGAGAGGTGGCTAGTTTCGGAGGATCTAACATC GAACCCAGCGTGCGCAGCTGTTTCCAGTTTGCCAATAACAAACCTGAAATCG AGGCTGCTCTGTTCCTGGATTGGATGCGCCTGGAACCACAGAGCATGGTGTG GCTGCCTGTGCTGCACAGAGTGGCTGCCGCCGAAACTGCCAAGCACCAGGCT AAATGCAACATCTGCAAGGAATGTCCCATTATCGGCTTTCGCTACAGGAGTC TGAAACATTTTAACTACGATATTTGCCAGAGCTGCTTCTTTTCCGGAAGAGT GGCCAAAGGACACAAGATGCACTACCCTATGGTGGAATATTGCACCCCAACT ACATCTGGCGAAGATGTGCGCGATTTTGCCAAGGTGCTGAAGAATAAGTTTC GGACTAAGAGGTACTTCGCCAAGCACCCCCGCATGGGGTATCTGCCAGTGCA GACAGTGCTGGAAGGAGACAATATGGAGACCGATACAATGTGAGC |

### D. Vectors for Gene Delivery

Another aspect of the present invention relates to the genetic engineering of nucleic acid regulatory elements and incorporating these nucleic acid sequences in a vector expression system. In one embodiment, the vector is a viral vector, including but not limited to recombinant adeno-associated viral (rAAV) vectors (e.g. Gao G., et al 2003 Proc. Natl. Acad. Sci. U.S.A. 100(10):6081-6086), lentiviral vectors (e.g. Matrai, J, et al. 2011, Hepatology 53, 1696-707), retroviral vectors (e.g. Axelrod, JH, et al. 1990. Proc Natl Acad Sci USA; 87, 5173-7), adenoviral vectors (e.g. Brown et al., 2004 Blood 103, 804-10), herpes-simplex viral vectors (Marconi, P. et al. Proc Natl Acad Sci U S A. 1996 93(21): 11319-11320; Baez, MV, et al. Chapter 19 - Using Herpes Simplex Virus Type 1-Based Amplicon Vectors for Neuroscience Research and Gene Therapy of Neurologic Diseases, Ed.: Robert T. Gerlai, Molecular-Genetic and Statistical Techniques for Behavioral and Neural Research, Academic Press, 2018:Pages 445-477), and retrotransposon-based vector systems (e.g. Soifer, 2004, Current Gene Therapy 4(4):373-384). In another embodiment, the vector is a non-viral vector. rAAV vectors have limited packaging capacity of the vector particles (i.e. approximately 4.7 kb), constraining the size of the transgene expression cassette to obtain functional vectors (Jiang et al., 2006 Blood. 108:107-15). The length of the transgene and the length of the regulatory nucleic acid sequences comprising tandem enhancer(s) and promoter(s) are taken into consideration when selecting a regulatory region suitable for a particular transgene and target tissue.

Another aspect of the present invention relates to a viral vector comprising an expression cassette comprising a nucleic acid regulatory element of SEQ ID NO:1 or SEQ ID NO:2 operably linked to a transgene. In some embodiments, the expression cassette enhances expression of the transgene in muscle.

In another aspect, the expression cassettes are suitable for packaging in an AAV capsid, as such the cassette comprises (1) AAV inverted terminal repeats (ITRs) flank the expression cassette; (2) regulatory control elements, a) modified Spc5-12 promoter having a nucleotide sequence of SEQ ID NO:1 or SEQ ID NO:2 alone or in combination with an enhancer (such as those listed in Table 1), b) a poly A signal, and c) optionally an intron; and (3) a transgene providing (e.g., coding for) one or more RNA or protein products of interest, including a microdystrophin protein. In certain embodiments, the expression cassette comprises a nucleotide sequence comprising SEQ ID NO: 38. In certain embodiments, the expression cassette comprises a nucleotide sequence comprising SEQ ID NO: 40.

In certain embodiments, the transgene is from Tables 7A-7B. In embodiments for expressing an intact or substantially intact mAb, the constructs described herein comprise the following components: (1) AAV2 inverted terminal repeats that flank the expression cassette; (2) regulatory control elements comprising a) a promoter having a nucleotide sequence of SEQ ID NO:1 or SEQ ID NO:2 alone or in combination with an enhancer (such as those listed in Table 1), b) a poly A signal, and c) optionally an intron; and (3) nucleic acid sequences coding for any therapeutic protein used to treat muscle specific diseases such as muscular dystrophies. In certain embodiments, the therapeutic protein is Dys1, having an amino acid sequence of SEQ ID NO: 35.

In certain embodiments, the rAAV comprise an artificial genome comprising a nucleic acid sequence comprising SEQ ID NO: 39. In certain embodiments, the rAAV comprise an artificial genome comprising a nucleic acid sequence comprising SEQ ID NO: 41.

In the various embodiments, the target tissue may be muscle or heart. The transgenes expressed in muscle are considered systemic expression, since enhanced delivery of muscle-expressed protein may be sufficient to cross into other tissues including crossing the blood brain barrier to the CNS and delivering therapeutics for treating neurological disorders or neurological symptoms of a systemic disorder.

### 1. AAV

Another aspect of the present invention relates to expression cassettes suitable for packaging in an AAV capsid, as such the expression cassette comprises (1) AAV inverted terminal repeats (ITRs) flank the expression cassette; (2) regulatory control elements, comprising a) a promoter having a nucleotide sequence of SEQ ID NO:1 or SEQ ID NO:2 provided herein, b) a poly A signal, and c) optionally, an intron; and (3) a transgene providing (e.g., coding for) one or more RNA or protein products of interest, particularly a therapeutic for muscle-specific diseases or disorders, including a microdystrophin.

The provided nucleic acids and methods are suitable for use in the production of any isolated recombinant AAV particles, in the production of a composition comprising any isolated recombinant AAV particles, or in the method for treating a disease or disorder in a subject in need thereof comprising the administration of any isolated recombinant AAV particles. As such, the rAAV may be of any serotype, modification, or derivative, known in the art, or any combination thereof (e.g., a population of rAAV particles that comprises two or more serotypes, e.g., comprising two or more of rAAV2, rAAV8, and rAAV9 particles) known in the art. In some embodiments, the rAAV particles are AAV1, AAV2, rAAV3, AAV4, AAV5, AAV6, AAV7, AAV8, AAV9, AAV10, AAV-11, AAV-12, AAV-13, AAV-14, AAV-15 and AAV-16, AAV.rh8, AAV.rh10, AAV.rh20, AAV.rh39, AAV.Rh74, AAV.RHM4-1, AAVhu.32, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, or AAV.HSC16 or other rAAV particles, or combinations of two or more thereof.

In some embodiments, rAAV particles have a capsid protein from an AAV serotype selected from AAV1, AAV1, AAV2, rAAV3, AAV4, AAV5, AAV6, AAV7, AAV8, AAV9, AAV10, AAV-11, AAV-12, AAV-13, AAV-14, AAV-15 and AAV-16, AAV.rh8, AAV.rh10, AAV.rh20, AAV.rh39, AAV.Rh74, AAV.RHM4-1, AAVhu.32, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, or AAV.HSC16 or a derivative, modification, or pseudotype thereof. In some embodiments, rAAV particles comprise a capsid protein at least 80% or more identical, e.g., 85%, 86%, 87%, 88%, 89%, 90%, 91%, 92%, 93%, 94%, 95%, 96%, 97%, 98%, 99%, 99.5%, etc., i.e. up to 100% identical, to e.g., VP1, VP2 and/or VP3 sequence of an AAV capsid serotype selected from AAV1, AAV1, AAV2, AAV3, AAV4, AAV5, AAV6, AAV7, AAV8, AAV9, AAV10, AAV-11, AAV-12, AAV-13, AAV-14, AAV-15 and AAV-16, AAV.rh8, AAV.rh10, AAV.rh20, AAV.rh39, AAV.Rh74, AAV.RHM4-1, AAVhu.32, AAV.hu37, AAV.Anc80, rAAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16.

In some embodiments, rAAV particles comprise a capsid protein from an AAV capsid serotype selected from AAV1, AAV1, AAV2, rAAV3, AAV4, AAV5, AAV6, AAV7, AAV8, AAV9, AAV10, AAV-11, AAV-12, AAV-13, AAV-14, AAV-15 and AAV-16, AAV.rh8, AAV.rh10, AAV.rh20, AAV.rh39, AAV.Rh74, AAV.RHM4-1, AAVhu.32, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, or AAV.HSC16, or a derivative, modification, or pseudotype thereof. In some embodiments, rAAV particles comprise a capsid protein at least 80% or more identical, e.g., 85%, 86%, 87%, 88%, 89%, 90%, 91%, 92%, 93%, 94%, 95%, 96%, 97%, 98%, 99%, 99.5%, etc., i.e. up to 100% identical, to e.g., VP1, VP2 and/or VP3 sequence of an AAV capsid serotype selected from AAV1, AAV1, AAV2, AAV3, AAV4, AAV5, AAV6, AAV7, AAV8, AAV9, AAV10, AAV-11, AAV-12, AAV-13, AAV-14, AAV-15 and AAV-16, AAV.rh8, AAV.rh10, AAV.rh20, AAV.rh39, AAV.Rh74, AAV.RHM4-1, AAVhu32, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, or AAV.HSC16.

In some embodiments, rAAV particles comprise the capsid of Anc80 or Anc80L65, as described in Zinn et al., 2015, Cell Rep. 12(6): 1056-1068. In certain embodiments, the rAAV particles comprise the capsid with one of the following amino acid insertions: LGETTRP (SEQ ID NO:42) or LALGETTRP (SEQ ID NO:43), as described in United States Patent Nos. 9,193,956; 9458517; and 9,587,282 and US patent application publication no. 2016/0376323. In some embodiments, rAAV particles comprise the capsid of AAV.7m8, as described in United States Patent Nos. 9,193,956; 9,458,517; and 9,587,282 and US patent application publication no. 2016/0376323. In some embodiments, rAAV particles comprise any AAV capsid disclosed in United States Patent No. 9,585,971, such as AAV-PHP.B. In some embodiments, rAAV particles comprise any AAV capsid disclosed in United States Patent No. 9,840,719 and WO 2015/013313, such as AAV.Rh74 and RHM4-1. In some embodiments, rAAV particles comprise any AAV capsid disclosed in WO 2014/172669, such as AAV rh74. In some embodiments, rAAV particles comprise the capsid of AAV2/5, as described in Georgiadis et al., 2016, Gene Therapy 23: 857-862 and Georgiadis et al., 2018, Gene Therapy 25: 450. In some embodiments, rAAV particles comprise any AAV capsid disclosed in WO 2017/070491, such as AAV2tYF. In some embodiments, rAAV particles comprise the capsids of AAVLK03 or AAV3B, as described in Puzzo et al., 2017, Sci. Transl. Med. 29(9): 418. In some embodiments, rAAV particles comprise any AAV capsid disclosed in US Pat Nos. 8,628,966; US 8,927,514; US 9,923,120 and WO 2016/049230, such as HSC1, HSC2, HSC3, HSC4, HSC5, HSC6, HSC7, HSC8, HSC9, HSC10 , HSC11, HSC12, HSC13, HSC14, HSC15, or HSC16.

In some embodiments, rAAV particles comprise an AAV capsid disclosed in any of the following patents and patent applications: United States Patent Nos. 7,282,199; 7,906,111; 8,524,446: 8,999,678; 8,628,966; 8,927,514; 8,734,809; US 9,284,357; 9,409,953; 9,169,299; 9,193,956; 9458517; and 9,587,282; US patent application publication nos. 2015/0374803; 2015/0126588; 2017/0067908; 2013/0224836; 2016/0215024; 2017/0051257; and International Patent Application Nos. PCT/US2015/034799; PCT/EP2015/053335. In some embodiments, rAAV particles have a capsid protein at least 80% or more identical, e.g., 85%, 86%, 87%, 88%, 89%, 90%, 91%, 92%, 93%, 94%, 95%, 96%, 97%, 98%, 99%, 99.5%, etc., i.e. up to 100% identical, to the VP1, VP2 and/or VP3 sequence of an AAV capsid disclosed in any of the following patents and patent applications: United States Patent Nos. 7,282,199; 7,906,111; 8,524,446; 8,999,678; 8,628,966; 8,927,514; 8,734,809; US 9,284,357; 9,409,953; 9,169,299; 9,193,956; 9458517; and 9,587,282; US patent application publication nos. 2015/0374803; 2015/0126588; 2017/0067908; 2013/0224836; 2016/0215024; 2017/0051257; and International Patent Application Nos. PCT/US2015/034799; PCT/EP2015/053335.

In some embodiments, rAAV particles have a capsid protein disclosed in Intl. Appl. Publ. No. WO 2003/052051 (see, e.g., SEQ ID NO: 2 in '051 publication), WO 2005/033321 (see, e.g., SEQ ID NOs: 123 and 88 in '321 publication), WO 03/042397 (see, e.g., SEQ ID NOs: 2, 81, 85, and 97 in '397 publication), WO 2006/068888 (see, e.g., SEQ ID NOs: 1 and 3-6 in '888 publication), WO 2006/110689, (see, e.g., SEQ ID NOs: 5-38 in '689 publication) WO2009/104964 (see, e.g., SEQ ID NOs: 1-5, 7, 9, 20, 22, 24 and 31 in '964 publication), WO2010/127097 (see, e.g., SEQ ID NOs: 5-38 in '097 publication), and WO 2015/191508 (see, e.g., SEQ ID NOs: 80-294 in '508 publication), and U.S. Appl. Publ. No. 20150023924 (see, e.g., SEQ ID NOs: 1, 5-10 in '924 publication). In some embodiments, rAAV particles have a capsid protein at least 80% or more identical, e.g., 85%, 86%, 87%, 88%, 89%, 90%, 91%, 92%, 93%, 94%, 95%, 96%, 97%, 98%, 99%, 99.5%, etc., i.e. up to 100% identical, to the VP1, VP2 and/or VP3 sequence of an AAV capsid disclosed in Intl. Appl. Publ. No. WO 2003/052051 (see, e.g., SEQ ID NO: 2 in '051 publication), WO 2005/033321 (see, e.g., SEQ ID NOs: 123 and 88 in '321 publication), WO 03/042397 (see, e.g., SEQ ID NOs: 2, 81, 85, and 97 in '397 publication), WO 2006/068888 (see, e.g., SEQ ID NOs: 1 and 3-6 in '888 publication), WO 2006/110689 (see, e.g., SEQ ID NOs: 5-38 in '689 publication) WO2009/104964 (see, e.g., SEQ ID NOs: 1-5, 7, 9, 20, 22, 24 and 31 in '964 publication), WO 2010/127097 (see, e.g., SEQ ID NOs: 5-38 in '097 publication), and WO 2015/191508 (see, e.g., SEQ ID NOs: 80-294 of in '508 publication), and U.S. Appl. Publ. No. 20150023924 (see, e.g., SEQ ID NOs: 1, 5-10 in '924 publication).

Nucleic acid sequences of AAV based viral vectors and methods of making recombinant AAV and AAV capsids are taught, for example, in United States Patent Nos. 7,282,199; 7,906,111; 8,524,446; 8,999,678; 8,628,966; 8,927,514; 8,734,809; US 9,284,357; 9,409,953; 9,169,299; 9,193,956; 9458517; and 9,587,282; US patent application publication nos. 2015/0374803; 2015/0126588; 2017/0067908; 2013/0224836; 2016/0215024; 2017/0051257; International Patent Application Nos. PCT/US2015/034799; PCT/EP2015/053335; WO 2003/052051, WO 2005/033321, WO 03/042397, WO 2006/068888, WO 2006/110689, WO2009/104964, WO 2010/127097, and WO 2015/191508, and U.S. Appl. Publ. No. 20150023924.

In additional embodiments, rAAV particles comprise a pseudotyped AAV capsid. In some embodiments, the pseudotyped AAV capsids are rAAV2/8 or rAAV2/9 or rAAV2/hu.32 pseudotyped AAV capsids. Methods for producing and using pseudotyped rAAV particles are known in the art (see, e.g., Duan et al., J. Virol., 75:7662-7671 (2001); Halbert et al., J. Virol., 74:1524-1532 (2000); Zolotukhin et al., Methods 28:158-167 (2002); and Auricchio et al., Hum. Molec. Genet. 10:3075-3081, (2001).

In additional embodiments, rAAV particles comprise a capsid containing a capsid protein chimeric of two or more AAV capsid serotypes. In some embodiments, the capsid protein is a chimeric of 2 or more AAV capsid proteins from AAV serotypes selected from AAV1, AAV1, AAV2, AAV3, AAV4, AAV5, AAV6, AAV7, AAV8, AAV9, AAV10, AAV-11, AAV-12, AAV-2/13, AAV-14, AAV-15 and AAV-16, AAV.rh8, AAV.rh10, AAV.rh20, AAV.rh39, AAV.Rh74, AAV.RHM4-1, AAVhu32, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16.

In certain embodiments, a single-stranded AAV (ssAAV) can be used. In certain embodiments, a self-complementary vector, e.g., scAAV, can be used (see, e.g., Wu, 2007, Human Gene Therapy, 18(2):171-82, McCarty et al, 2001, Gene Therapy, Vol. 8, Number 16, Pages 1248-1254; and U.S. Patent Nos. 6,596,535; 7,125,717; and 7,456,683).

In some embodiments, rAAV particles comprise a capsid protein from an AAV capsid serotype selected from AAV-8 or AAV-9. In some embodiments, the rAAV particles have an AAV capsid serotype of AAV-1 or a derivative, modification, or pseudotype thereof. In some embodiments, the rAAV particles have an AAV capsid serotype of AAV-4 or a derivative, modification, or pseudotype thereof. In some embodiments, the rAAV particles have an AAV capsid serotype of AAV-5 or a derivative, modification, or pseudotype thereof. In some embodiments, the rAAV particles have an AAV capsid serotype of AAV-8 or a derivative, modification, or pseudotype thereof. In some embodiments, the rAAV particles have an AAV capsid serotype of AAV-9 or a derivative, modification, or pseudotype thereof.

In some embodiments, rAAV particles comprise a capsid protein that is a derivative, modification, or pseudotype of AAV-8 or AAV-9 capsid protein. In some embodiments, rAAV particles comprise a capsid protein that has an AAV-8 capsid protein at least 80% or more identical, e.g., 85%, 86%, 87%, 88%, 89%, 90%, 91%, 92%, 93%, 94%, 95%, 96%, 97%, 98%, 99%, 99.5%, etc., i.e. up to 100% identical, to the VP1, VP2 and/or VP3 sequence of AAV-8 capsid protein.

In some embodiments, rAAV particles comprise a capsid protein that is a derivative, modification, or pseudotype of AAV-9 capsid protein. In some embodiments, rAAV particles in the clarified feed comprise a capsid protein that has an AAV-8 capsid protein at least 80% or more identical, e.g., 85%, 86%, 87%, 88%, 89%, 90%, 91%, 92%, 93%, 94%, 95%, 96%, 97%, 98%, 99%, 99.5%, etc., i.e. up to 100% identical, to the VP1, VP2 and/or VP3 sequence of AAV-9 capsid protein.

In additional embodiments, rAAV particles comprise a mosaic capsid. Mosaic AAV particles are composed of a mixture of viral capsid proteins from different serotypes of AAV. In some embodiments, rAAV particles comprise a mosaic capsid containing capsid proteins of a serotype selected from AAV1, AAV2, AAV3, AAV4, AAV5, AAV6, AAV7, AAV8, AAV9, AAV10, AAV-11, AAV-12, AAV-13, AAV-14, AAV-15 and AAV-16, AAV.rh8, AAV.rh10, AAV.rh20, AAV.rh39, AAV.Rh74, AAV.RHM4-1, AAVhu32, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, and AAV.HSC16.

In some embodiments, rAAV particles comprise a mosaic capsid containing capsid proteins of a serotype selected from AAV-1, AAV-2, AAV-5, AAV-6, AAV-7, AAV-8, AAV-9, AAV-10, AAVrh.8, and AAVrh.10.In additional embodiments, rAAV particles comprise a pseudotyped rAAV particle. In some embodiments, the pseudotyped rAAV particle comprises (a) a nucleic acid vector comprising AAV ITRs and (b) a capsid comprised of capsid proteins derived from AAVx (e.g., AAV-1, AAV-3, AAV-4, AAV-5, AAV-6, AAV-7, AAV-8, AAV-9, AAV-10 AAV-11, AAV-12, AAV-13, AAV-14, AAV-15 and AAV-16). In additional embodiments, rAAV particles comprise a pseudotyped rAAV particle comprised of a capsid protein of an AAV serotype selected from AAV1, AAV2, AAV3, AAV4, AAV5, AAV6, AAV7, AAV8, AAV9, AAV10, AAV-11, AAV-12, AAV-13, AAV-14, AAV-15 and AAV-16, AAV.rh8, AAV.rh10, AAV.rh20, AAV.rh39, AAV.Rh74, AAV.RHM4-1, AAVhu.32, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, and AAV.HSC16. In additional embodiments, rAAV particles comprise a pseudotyped rAAV particle comprised of AAV-8 capsid protein. In additional embodiments, rAAV particles comprise a pseudotyped rAAV particle comprised of AAV-9 capsid protein. In additional embodiments, rAAV particles comprise a pseudotyped rAAV particle containing AAVhu.32 capsid protein. In some embodiments, the pseudotyped rAAV8 or rAAV9 or hu.32 particles are rAAV2/8 or rAAV2/9 or rAAV2/hu.32 pseudotyped particles. Methods for producing and using pseudotyped rAAV particles are known in the art (see, e.g., Duan et al., J. Virol., 75:7662-7671 (2001); Halbert et al., J. Virol., 74:1524-1532 (2000); Zolotukhin et al., Methods 28:158-167 (2002); and Auricchio et al., Hum. Molec. Genet. 10:3075-3081, (2001).

In additional embodiments, rAAV particles comprise a capsid containing a capsid protein chimeric of two or more AAV capsid serotypes. In further embodiments, the capsid protein is a chimeric of 2 or more AAV capsid proteins from AAV serotypes selected from AAV1, AAV2, rAAV3, AAV4, AAV5, AAV6, AAV7, AAV8, AAV9, AAV10, AAV11, AAV12, AAV13, AAV14, AAV15 and AAV16, AAV.rh8, AAV.rh10, AAV.rh20, AAV.rh39, AAV.Rh74, AAV.RHM4-1, AAVhu.32, AAV.hu37, AAV.Anc80, AAV.Anc80L65, AAV.7m8, AAV.PHP.B, AAV2.5, AAV2tYF, AAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, and AAV.HSC16. In further embodiments, the capsid protein is a chimeric of 2 or more AAV capsid proteins from AAV serotypes selected from AAV1, AAV2, AAV5, AAV6, AAV7, AAV8, AAV9, AAV10, AAVrh.8, and AAVrh.10.

In some embodiments, the rAAV particles comprise an AAV capsid protein chimeric of AAV-8 capsid protein and one or more AAV capsid proteins from an AAV serotype selected from AAV1, AAV2, AAV3, AAV4, AAV5, AAV6, AAV7, AAV8, AAV9, AAV10, AAV11, AAV12, AAV13, AAV14, AAV15 and AAV16, AAV.rh8, AAV.rh10, AAV.rh20, AAV.rh39, AAV.Rh74, AAV.RHM4-1, AAVhu32, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, and AAV.HSC16. In some embodiments, the rAAV particles comprise an AAV capsid protein chimeric of AAV-8 capsid protein and one or more AAV capsid proteins from an AAV serotype selected from AAV 1, AAV2, AAV5, AAV6, AAV7, AAV9, AAV10, AAVrh.8, and AAVrh.10.

In some embodiments, the rAAV particles comprise an AAV capsid protein chimeric of AAV-9 capsid protein the capsid protein of one or more AAV capsid serotypes selected from AAV1, AAV2, AAV3, AAV4, AAV5, AAV6, AAV7, AAV8, AAV9, AAV10, AAV11, AAV12, AAV13, AAV14, AAV15 and AAV16, AAV.rh8, AAV.rh10, AAV.rh20, AAV.rh39, AAV.Rh74, AAV.RHM4-1, AAVhu32, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, and AAV.HSC16.

In some embodiments, the rAAV particles comprise an AAV capsid protein chimeric of AAV-9 capsid protein the capsid protein of one or more AAV capsid serotypes selected from AAV1, AAV2, AAV3, AAV4, AAV5, AA6, AAV7, AAV8, AAV9, AAVrh.8, and AAVrh.10.

### 2. Methods of Making rAAV Vectors

Another aspect of the present invention involves making molecules disclosed herein. In some embodiments, a molecule according to the invention is made by providing a nucleotide comprising the nucleic acid sequence encoding an AAV capsid protein; and using a packaging cell system to prepare corresponding rAAV particles with capsid coats made up of the capsid protein. In some embodiments, the nucleic acid sequence encodes a sequence having at least 60%, 70%, 80%, 85%, 90%, or 95%, preferably 96%, 97%, 98%, 99% or 99.9%, identity to the sequence of a capsid proteins described herein, and retains (or substantially retains) biological function of the capsid protein and the inserted peptide from a heterologous protein or domain thereof. In some embodiments, the nucleic acid encodes a sequence having at least 60%, 70%, 80%, 85%, 90%, or 95%, preferably 96%, 97%, 98%, 99% or 99.9%, identity to a particular sequence of the AAV capsid protein, while retaining (or substantially retaining) biological function of the AAV capsid protein.

The capsid protein, coat, and rAAV particles may be produced by techniques known in the art. In some embodiments, the viral genome comprises at least one inverted terminal repeat to allow packaging into a vector, for example, those disclosed herein in Table 6. In some embodiments, the viral genome further comprises a cap gene and/or a rep gene for expression and splicing of the cap gene. In certain embodiments, the cap and rep genes are provided by a packaging cell and not present in the viral genome.

In some embodiments, the nucleic acid encoding the capsid protein is cloned into an AAV Rep-Cap helper plasmid in place of the existing capsid gene. When introduced together into host cells, this plasmid helps package an rAAV genome into the capsid protein as the capsid coat. Packaging cells can be any cell type possessing the genes necessary to promote AAV genome replication, capsid assembly, and packaging. Nonlimiting examples include HEK293 cells or derivatives thereof, HELA cells, or insect cells.

Standard techniques can be used for recombinant DNA, oligonucleotide synthesis, and tissue culture and transformation (e.g., electroporation, lipofection). Enzymatic reactions and purification techniques can be performed according to manufacturer's specifications or as commonly accomplished in the art or as described herein. The foregoing techniques and procedures can be generally performed according to conventional methods well known in the art and as described in various general and more specific references that are cited and discussed throughout the present specification. See, e.g., Sambrook et al., Molecular Cloning: A Laboratory Manual (2d ed., Cold Spring Harbor Laboratory Press, Cold Spring Harbor, N.Y. (1989)). Unless specific definitions are provided, the nomenclatures utilized in connection with, and the laboratory procedures and techniques of, analytical chemistry, synthetic organic chemistry, and medicinal and pharmaceutical chemistry described herein are those well-known and commonly used in the art. Standard techniques can be used for chemical syntheses, chemical analyses, pharmaceutical preparation, formulation, and delivery, and treatment of patients. Nucleic acid sequences of AAV-based viral vectors, and methods of making recombinant AAV and AAV capsids, are taught, e.g., in US 7,282,199; US 7,790,449; US 8,318,480; US 8,962,332; and PCT/EP2014/076466.

In preferred embodiments, the rAAVs provide transgene delivery vectors that can be used in therapeutic and prophylactic applications, as discussed in more detail below. The rAAV vector also includes the regulatory control elements discussed supra, including the modified Spc5-12 promoters of SEQ ID NOs: 1 and 2, to influence and promote the expression of the RNA and/or protein products encoded by nucleic acids (transgenes) within target cells, including muscle cells, of the subject.

Provided in particular embodiments are AAV vectors comprising a viral genome comprising an expression cassette for expression of the transgene, under the control of regulatory elements, and flanked by ITRs and an engineered viral capsid as described herein or is at least 95%, 96%, 97%, 98%, 99% or 99.9% identical to the amino acid sequence of the AAV capsid protein.

The recombinant adenovirus can be a first generation vector, with an E1 deletion, with or without an E3 deletion, and with the expression cassette inserted into either deleted region. The recombinant adenovirus can be a second generation vector, which contains full or partial deletions of the E2 and E4 regions. A helper-dependent adenovirus retains only the adenovirus inverted terminal repeats and the packaging signal (phi). The transgene generally is inserted between the packaging signal and the 3'ITR, with or without stuffer sequences to keep the genome close to wild-type size of approximately 36 kb. An exemplary protocol for production of adenoviral vectors may be found in Alba et al., 2005, "Gutless adenovirus: last generation adenovirus for gene therapy," Gene Therapy 12 S18-S27

The rAAV vector for delivering the transgene to target tissues, cells, or organs, may also have a tropism for that particular target tissue, cell, or organ, e.g. muscle, in conjunction with the use of tissue-specific promoters as described herein. The construct can further include additional expression control elements such as introns that enhance expression of the transgene (e.g., introns such as the chicken β-actin intron, minute virus of mice (MVM) intron, human factor IX intron (e.g., FIX truncated intron 1), β-globin splice donor/immunoglobulin heavy chain splice acceptor intron, adenovirus splice donor /immunoglobulin splice acceptor intron, SV40 late splice donor /splice acceptor (19S/16S) intron, and hybrid adenovirus splice donor/IgG splice acceptor intron and poly A signals such as the rabbit β-globin poly A signal, human growth hormone (hGH) polyA signal, SV40 late poly A signal, synthetic polyA (SPA) signal, and bovine growth hormone (bGH) poly A signal. See, e.g., Powell and Rivera-Soto, 2015, Discov. Med., 19(102):49-57.

In certain embodiments, nucleic acids sequences disclosed herein may be codon-optimized, for example, via any codon-optimization technique known to one of skill in the art (see, e.g., review by Quax et al., 2015, Mol Cell 59:149-161).

In a certain embodiment, the constructs described herein comprise the following components: (1) AAV inverted terminal repeats (ITRs) that flank the expression cassette; (2) control elements, which include a) one or more regulatory elements at least including one or more of the promoters of SEQ ID NO:1 or SEQ ID NO:2, alone or in combination with one or more enhancers, b) a poly A signal, and c) optionally an intron; (3) transgene providing (e.g., coding for) one or more RNA or protein products of interest. In another embodiment, the constructs described herein comprise the following components: (1) AAV2 inverted terminal repeats that flank the expression cassette; (2) control elements, which include a) one or more regulatory elements at least including one or more of the promoters of SEQ ID NO:1 or SEQ ID NO:2, alone or in combination with one or more enhancers, b) a rabbit β-globin poly A signal and e) optionally a chimeric intron derived from human β-globin and Ig heavy chain, or other intron; and (3) transgene providing (e.g., coding for) one or more RNA or protein products of interest, such as those in Tables 7A-7B or a microdystrophin protein. In certain embodiments, the transgene is a muscle-specific therapeutic. In certain embodiments, the muscle-specific therapeutic is a microdystrophin. In certain embodiments, the microdystrophin is Dys1 (SEQ ID NO: 35), Dys3 (SEQ ID NO: 36), Dys5 (SEQ ID NO: 37), MD1 (SEQ ID NO: 56), human microdystrophin (SEQ ID NO: 57), Dys3978 (SEQ ID NO: 58), MD3 (SEQ ID NO: 59), MD4 (SEQ ID NO: 60) or MD5 (SEQ ID NO: 71). In certain embodiments, the microdystrophin is Dys1 (SEQ ID NO: 35). In embodiments, the microdystrophin transgene is encoded by a nucleotide sequence comprising SEQ ID NO: 67, SEQ ID NO: 68, SEQ ID NO: 69, SEQ ID NO: 70 or SEQ ID NO: 71, or a nucleotide sequence at least about 90%, at least about 91%, at least about 92%, at least about 93%, at least about 94%, at least about 95%, at least about 96%, at least about 97%, at least about 98% or at least about 99% identical to SEQ ID NO: 67, SEQ ID NO: 68, SEQ ID NO: 69, SEQ ID NO: 70 or SEQ ID NO: 71. In certain embodiments, the expression cassette comprises a nucleic acid sequence comprising SEQ ID NO: 38. In certain embodiments, the expression cassette comprises a nucleic acid sequence comprising SEQ ID NO: 40. In certain embodiments, the constructs described herein comprise SEQ ID NO: 39. In certain embodiments, the constructs described herein comprise SEQ ID NO: 40. The expression cassettes may be within a plasmid that is appropriate for propagation in the target host cells and used for manufacturing of the recombinant AAV particles described herein.

The viral vectors provided herein may be manufactured using host cells, e.g., mammalian host cells, including host cells from humans, monkeys, mice, rats, rabbits, or hamsters. Nonlimiting examples include: A549, WEHI, 10T1/2, BHK, MDCK, COS1, COS7, BSC 1, BSC 40, BMT 10, VERO, W138, HeLa, 293, Saos, C2C12, L, HT1080, HepG2, primary fibroblast, hepatocyte, and myoblast cells. Typically, the host cells are stably transformed with the sequences encoding the transgene and associated elements (i.e., the vector genome), and genetic components for producing viruses in the host cells, such as the replication and capsid genes (e.g., the rep and cap genes of AAV). For a method of producing recombinant AAV vectors with AAV8 capsids, see Section IV of the Detailed Description of U.S. Patent No. 7,282,199 B2 Genome copy titers of said vectors may be determined, for example, by TAQMAN^{®} analysis. Virions may be recovered, for example, by CsCl2 sedimentation. Alternatively, baculovirus expression systems in insect cells may be used to produce AAV vectors. For a review, see Aponte-Ubillus et al., 2018, Appl. Microbiol. Biotechnol. 102:1045 1054.

In vitro assays, e.g., cell culture assays, can be used to measure transgene expression from a vector described herein, thus indicating, e.g., potency of the vector. Cell lines derived from muscle or other cell types may be used, for example, but not limited, to HuH-7, HEK293, fibrosarcoma HT-1080, HKB-11, C2C12 myoblasts, and CAP cells. Once expressed, characteristics of the expressed product (transgene product) can also be determined, including serum half-life, functional activity of the protein (e.g. enzymatic activity or binding to a target), determination of the glycosylation and tyrosine sulfation patterns, and other assays known in the art for determining protein characteristics. Provided are methods of manufacturing a recombinant AAV comprising culturing a host cell capable of producing a recombinant AAV described herein under conditions appropriate for production of the recombinant AAV comprising an artificial genome with an expression cassette comprising a synthetic promoter operably linked to a transgene. In particular, the method provides (1) culturing a host cell containing (i) an artificial genome comprising AAV ITRs flanking a recombinant *cis* expression cassette which comprises a nucleic acid regulatory element comprising a promoter having a nucleotide sequence of SEQ ID NO: 1 or 2, as disclosed herein operably linked to a transgene; (ii) a trans expression cassette lacking AAV ITRs which encodes an AAV rep and an AAV capsid protein operably linked to expression control elements that drive expression of the AAV rep and the AAV capsid protein in the host cell in culture and supply the AAV rep and the AAV capsid protein in trans; and (iii) sufficient adenovirus helper functions to permit replication and packaging of the artificial genome by the AAV capsid protein; and (2) recovering recombinant AAV encapsidating the artificial genome from the cell culture.

Also provided are host cells containing (i) an artificial genome comprising AAV ITRs flanking a recombinant *cis* expression cassette which comprises one or more regulatory elements comprising a promoter having a nucleotide sequence of SEQ ID NO: 1 or 2, as disclosed herein operably linked to a transgene; (ii) a trans expression cassette lacking AAV ITRs which encodes an AAV rep and an AAV capsid protein operably linked to expression control elements that drive expression of the AAV rep and the AAV capsid protein in the host cell in culture and supply the AAV rep and the AAV capsid protein in trans; and, optionally, (iii) sufficient adenovirus helper functions to permit replication and packaging of the artificial genome by the AAV capsid protein. In particular embodiments, the composite nucleic acid regulatory element consists of SEQ ID NO: 1 or SEQ ID NO: 2. In certain embodiments, the artificial genome comprises a transgene encoding one of the therapeutics listed in Tables 7A-7B or a microdystrophin.

Also provided are host cells comprising a plasmid comprising a *cis* expression cassette, wherein the *cis* expression cassette comprises a promoter operably linked to a transgene, wherein the promoter comprises a nucleotide sequence of SEQ ID NO:1 or SEQ ID NO:2. In certain embodiments, the artificial genome comprises a transgene encoding one of the therapeutics listed in Tables 7A-7B or a microdystrophin. In certain embodiments, the transgene is a muscle-specific therapeutic. In certain embodiments, the muscle-specific therapeutic is a microdystrophin. In certain embodiments, the microdystrophin is Dys1 (SEQ ID NO: 35), Dys3 (SEQ ID NO: 36), Dys5 (SEQ ID NO: 37), MD1 (SEQ ID NO: 56), human microdystrophin (SEQ ID NO: 57), Dys3978 (SEQ ID NO: 58), MD3 (SEQ ID NO: 59), MD4 (SEQ ID NO: 60) or MD5 (SEQ ID NO: 71). In certain embodiments, the microdystrophin is Dys1 (SEQ ID NO: 35). In embodiments, the microdystrophin transgene is encoded by a nucleotide sequence comprising SEQ ID NO: 67, SEQ ID NO: 68, SEQ ID NO: 69, SEQ ID NO: 70 or SEQ ID NO: 71, or a nucleotide sequence at least about 90%, at least about 91%, at least about 92%, at least about 93%, at least about 94%, at least about 95%, at least about 96%, at least about 97%, at least about 98% or at least about 99% identical to SEQ ID NO: 67, SEQ ID NO: 68, SEQ ID NO: 69, SEQ ID NO: 70 or SEQ ID NO: 71. In certain embodiments, the expression cassette comprises a nucleic acid sequence comprising SEQ ID NO: 38. In certain embodiments, the expression cassette comprises a nucleic acid sequence comprising SEQ ID NO: 40. In certain embodiments, the plasmid comprises SEQ ID NO: 39. In certain embodiments, the plasmid comprises SEQ ID NO: 40.

In some aspects, the disclosed methods of manufacturing or producing recombinant AAV vectors having an AAV genome with a transgene operably linked to a promoter having a nucleotide sequence of SEQ ID NO:1 or 2 results in a higher packaging efficiency, including resulting in a higher percentage of capsids with intact or complete genomes than a recombinant AAV vector with a comparable AAV genome comprising an unmodified Spc5-12 promoter. In other words, the recombinant AAVs disclosed herein comprise a promoter having the sequence of SEQ ID NO:1 or SEQ ID NO:2, or a variant thereof, and the manufacturing process results in 40, 45, 50, 55, 60, 65, 70, or 75% full capsids of the capsid population recovered. Thus, in some aspects, at least 30%, 40%, 50%, 60%, 70% 80%, 90% or 95% of the rAAVs produced encapsidate a full AAV genome. In some aspects, the production results in recombinant AAVs having at least 1 fold, 1.5 fold, 2 fold, 2.5 fold, 3 fold, or more full capsids than recombinant AAVs comprising a comparable recombinant expression cassette (i.e., having all of the same elements except the promoter) comprising an unmodified Spc5-12 promoter produced using the same method. In some aspects, the production results in rAAVs having at least 1 fold, 1.5 fold, 2 fold, 2.5 fold, 3 fold or more full capsids than partial capsids. In some aspects, the percent of full capsids produced can be 10-100%, 20-100%, 30-100%, 40-100%, 50-100%, 60-100%, 70-100%, 80-100%, 90-100%, 10-90%, 20-90%, 30-90%, 40-90%, 50-90%, 60-90%, 70-90%, 80-90%, 10-80%, 20-80%, 30-80%, 40-80%, 50-80%, 60-80%, 70-80%, 10-70%, 20-70%, 30-70%, 40-70%, 50-70%, 60-70%, 10-60%, 20-60%, 30-60%, 40-60%, 50-60%, 10-50%, 20-50%, 30-50%, or 40-50%.

### E. Therapeutic and Prophylactic Uses

Another aspect relates to therapies which involve administering a transgene via a rAAV vector according to the invention to a subject in need thereof, for delaying, preventing, treating, and/or managing a disease or disorder, and/or ameliorating one or more symptoms associated therewith, wherein the transgene is operably linked to one or more of the promoters described herein, such as the promoter having a nucleotide sequence of SEQ ID NO:1 or SEQ ID NO:2. A subject in need thereof includes a subject suffering from the disease or disorder, or a subject pre-disposed thereto, e.g., a subject at risk of developing or having a recurrence of the disease or disorder. Generally, a rAAV carrying a particular transgene will find use with respect to a given disease or disorder in a subject where the subject's native gene, corresponding to the transgene, is defective in providing the correct gene product, or correct amounts of the gene product. The transgene then can provide a copy of a gene that is defective in the subject.

Generally, the transgene comprises cDNA that restores protein function to a subject having a genetic mutation(s) in the corresponding native gene. In embodiments, the transgene encodes the heavy and light chains of a therapeutic antibody, or an antigen binding fragment thereof. In some embodiments, the cDNA comprises associated RNA for performing genomic engineering, such as genome editing via homologous recombination. In some embodiments, the transgene encodes a therapeutic RNA, such as a shRNA, artificial miRNA, or element that influences splicing.

In some aspects, the therapeutic encoded by one or more of the disclosed transgenes can by any of the microdystrophins disclosed herein. Microdystrophins include those having the amino acid sequence of SEQ ID NOs: 35-37, 57-60 (Table 5) and/or encoded by the nucleotide sequence of SEQ ID NOs: 38-41 (Table 6, which lists the nucleotide sequences of expression cassettes encoding a microdystrophin operably linked to regulatory elements), such as the microdystrophin of Dys1 (SEQ ID NO: 35), Dys3 (SEQ ID NO: 36), Dys5 (SEQ ID NO: 37), MD1 (SEQ ID NO: 56), human microdystrophin (SEQ ID NO: 57), Dys3978 (SEQ ID NO: 58), MD3 (SEQ ID NO: 59), MD4 (SEQ ID NO: 60) or MD5 (SEQ ID NO: 71). In embodiments, the microdystrophin transgene is encoded by a nucleotide sequence comprising SEQ ID NO: 67, SEQ ID NO: 68 SEQ ID NO: 69, SEQ ID NO: 70 or SEQ ID NO: 71, or a nucleotide sequence at least about 90%, at least about 91%, at least about 92%, at least about 93%, at least about 94%, at least about 95%, at least about 96%, at least about 97%, at least about 98% or at least about 99% identical to SEQ ID NO: 67, SEQ ID NO: 68, SEQ ID NO: 69, SEQ ID NO: 70 or SEQ ID NO: 71. Provided are methods of treating human subjects for any muscular dystrophy disease that can be treated by providing a functional dystrophin. In some aspects, the functional dystrophin is one or more of the microdystrophins disclosed herein. DMD is the most common muscular dystrophy disease, but other diseases can be treated such as, but not limited to, Becker muscular dystrophy (BMD), myotonic muscular dystrophy (Steinert's disease), Facioscapulohumeral disease (FSHD), limb-girdle muscular dystrophy, X-linked dilated cardiomyopathy, or oculopharyngeal muscular dystrophy.

Tables 7A-7B below provides a list of transgenes that may be used in any of the rAAV vectors described herein in which the transgene sequence is operably linked to the modified Spc5-12 promoter (Spc5-12V1 or Spc5-12V2), preferably to treat or prevent the disease with which it is associated, also listed in Tables 7A-7B. The appropriate AAV serotype may be chosen to engineer to optimize the tissue tropism and transduction of the vector.

**Table 7A**

| **Disease** | **Transgene** | **Possible AAV serotype for delivery of transgene** |
|---|---|---|
| Spinal Muscular Atrophy (SMA) | SMN | AAV9 |
| Duchenne Muscular Dystrophy | Mini- / Micro-Dystrophin Gene | AAV2, AAV8, AAV9, AAVrh74, AAVhu.32 |
| Limb Girdle Muscular Dystrophy Type 2C\|Gamma-sarcoglycanopathy | human-alpha-sarcoglycan | AAV1 |
| Limb Girdle Muscular Dystrophy Type 2C\|Gamma-sarcoglycanopathy | gamma-sarcoglycan | AAV1 |
| Becker Muscular Dystrophy and Sporadic Inclusion Body Myositis | huFollistatin344 | AAV1 |
| Duchenne Muscular Dystrophy | GALGT2 | AAVrh74 |
| Limb-Girdle Muscular Dystrophy, Type 2E | hSGCB | rh74 |

**Table 7B**

| **ANTIGENS** | | **TRANSGENE/ANTIBODIES** | **INDICATIONS** |
|---|---|---|---|
| *Repulsive guidance molecule-A* | | elezanumab | multiple sclerosis |
| *Complement Component 5* | | ravulizumab | Myasthenia Gravis |
| *Connective tissue growth factor (CTGF)* | | pamrevlumab | fibrotic diseases, e.g. diabetic nephropathy, liver fibrosis, idiopathic pulmonary fibrosis |
| *Integrin beta 7* | | etrolizumab | ulcerative colitis, Crohn's disease |
| *Sclerostin* | | romosozumab (EVENITY^{®}) | Osteoporosis, abnormal bone loss or weakness |
| *Interleukin receptor 6 (IL6R) or Interleukin 6 (IL6)* | | Satralizumab | Adverse immune responses (e.g. cytokine storm, CAR-T therapy) |
| | | Sarilumab | |
| | | Tocilizumab | |
| | | siltuximab, | |
| | | clazakizumab | |
| | | sirukumab | |
| | | olokizumab | |
| | | gerilimzumab | |
| *Immunoglobin E (IgE)* | | omolizumab | Asthma, COPD, eosinophilic asthma, |
| | | | chronic idiopathic urticaria |
| *Thymic stromal lymphopoietin* (TSLP) | | tezelipumab | Asthma, COPD |
| *Interleukin 5 (IL5)* | | benralizumab | Asthma, COPD |
| *Interleukin 5 receptor (IL5R)* | | reslizumab | Asthma, COPD, eosinophilic asthma |
| *Interleukin 13 (IL13)* | | tralokinumab | Atopic dermatitis |
| *Interleukin 31 recptor alpha (IL31RA)* | | nemolizumab | Atopic dermatitis |
| IL17A | | Ixekizumab | Plaque psoriasis, psoriatic arthritis, ankylosing sponylitis |
| | | secukinumab | |
| *Interleukins or interleukin receptors* | *IL-17A* | ixekizumab (TALTZ^{®}) | Plaque psoriasis, psoriatic arthritis, ankylosing sponylitis |
| | | secukinumab (COSENTYX^{®}) | |
| | *IL-5* | mepolizumab (NUCALA^{®}) | Asthma |
| | *IL-12*/*IL-23* | ustekinumab (STELARA^{®}) | Psoriasis & Crohn's disease |
| | *IL-4R* | dupilumab | Atopic dermatitis |
| *Integrin* | | vedolizumab (ENTYVIO^{®}) | Ulcerative colitis & Crohn's disease |
| | | Natalizumab (anti-integrin alpha 4) | Multiple sclerosis & Crohn's disease |
| *Cardiovascular Targets* | *PCSK9* | alirocumab (PRALUENT^{®}) | HeFH & HoFH |
| | | evolucomab (REPATHA^{®}) | |
| | *ANGPTL3* | evinacumab | HoFH & severe forms of dyslipidema |
| | *Proinflammatory*/ *proatherogenic phospholipids* | E06-scFv | Cardiovascular diseases such as atherosclerosis |
| *RANKI,* | | denosumab (XGEVA^{®} and PROLIA^{®}) | Osteoporosis, increasing bone mass in breast and prostate cancer patients, & preventing skeletal-related events due to bone metastasis |
| *PD-1, or PD-L1 or PD-L2* | | nivolumab (OPDIVO^{®}) | Metastatic melanoma, lymphoma, non-small cell lung carcinoma |
| | | pembrolizumab (KEYTRUDA^{®}) | |
| *BLyS (B-lymphocyte stimulator, also known as B-cell activating factor (BAFF))* | | belimumab (BENLYSTA^{®}) | Systemic lupus erythromatosis |
| *TNF-alpha* | | adalimumab (HUMIRA^{®}) and infliximab (REMICADE^{®}) | Rheumatoid arthritis, psoriatic arthritis, askylosing spondylitis, Crohn's disease, plaque psoriasis, ulcerative colitis |
| *Plasma Protein targets* | *C5, C5a* | eculizumab (SOLIRIS^{®}) | Paroxysmal nocturnal hemoglobinuria, atypical hemolytic uremic syndrome, complement-mediated thrombotic microangiopathy |
| | *Plasma kallikrein* | lanadelumab | Hereditary angioedema (HAE) |

Generally, the rAAV vector is administered systemically, and following transduction, the vector's production of the protein product is enhanced by an expression cassette employing a modified Spc5-12 promoter, for example, the promoter of SEQ ID NO:1 or SEQ ID NO:2 can be operably linked to the transgene. In some aspects, the rAAV vector may be provided by intravenous, intramuscular, and/or intra-peritoneal administration.

With respect to the therapeutic antibodies in Table 7B expression in both muscle and liver may be desirable to deliver the expressed therapeutic antibody systemically. Accordingly, provided are expression cassettes in which the regulatory sequences operably linked to the transgene encoding the therapeutic antibody are composite or tandem regulatory sequences which, in addition to one of the Spc5-12 promoters (Spc5-12 V1 or Spc5-12 V2) comprise an additional liver specific promoter in tandem (e.g., hAAT promoter or TBG promoter) and/or liver specific enhancer elements, such as, ApoE enhancers, or Mic/BiKE elements, the sequences of which are provided in Table 1. In addition, the expression cassettes comprising the regulatory sequences operably linked to the transgene encoding the therapeutic antibody may be packaged in an rAAV for delivery that preferably has an AAV8 capsid, an AAV9 capsid or an AAVrh10 capsid for targeting or expression in liver and/or muscle cells.

In some aspects, the rAAVs of the present invention find use in delivery to target tissues associated with the disorder or disease to be treated/prevented. A disease or disorder associated with a particular tissue or cell type is one that largely affects the particular tissue or cell type, in comparison to other tissue of cell types of the body, or one where the effects or symptoms of the disorder appear in the particular tissue or cell type. Methods of delivering a transgene to a target tissue of a subject in need thereof involve administering to the subject the an rAAV where the expression cassette comprises a nucleic acid regulatory element comprising SEQ ID NO:1 or SEQ ID NO:2 operably linked to a transgene.

In some aspects, the transgene is a muscle-specific disease therapeutic. One or more of the transgene listed in Tables 7A-7B are muscle-specific disease therapeutics. A muscle-specific disease therapeutic can be a therapeutic that treats a muscle-specific disease. A muscle-specific disease can be diseases such as myopathies. In some aspects, a myopathy includes muscular dystrophies (e.g. Duchenne Muscular Dystrophy (DMD), Becker muscular dystrophy (BMD), human limb girdle muscular dystrophy (LGMD)), X-linked dilated cardiomyopathy, Myasthenia Gravis, Rhabdomyolysis, Amyotrophic Lateral Sclerosis (ALS), and Sarcopenia.

Disclosed are methods for enhancing expression of a transgene, comprising delivery of viral vectors comprising one or more of the disclosed recombinant expression cassettes. Thus, in some aspects, disclosed are methods for enhancing expression of a transgene, comprising delivery of viral vectors comprising a promoter comprising the nucleotide sequence of SEQ ID NO:1 or SEQ ID NO:2 operably linked to the transgene. In some aspects, the transgene can be any one or more of the transgenes disclosed herein, for example, a gene or nucleic acid encoding any of the therapeutics listed in Tables 7A-7B or a microdystrophin (for example, having an amino acid sequence of Table 5).

In some aspects, the delivery of viral vectors is to a cell. In some aspects, the cell can be in a subject and therefore the delivery can be administering to a subject. In some aspects, the viral vector is administered intravenously or intramuscularly.

In some aspects of the disclosed methods for enhancing expression of a transgene, the transgene expression is enhanced in the circulation or systemically. In some aspects of the disclosed methods for enhancing expression of a transgene, the transgene expression is enhanced in the skeletal muscle or cardiac muscle.

In some aspects, disclosed are methods of treatment by delivery of rAAVs comprising the one or more of the recombinant expression cassettes disclosed herein, one or more vectors disclosed herein or one or more of the disclosed rAAV. Also provided are methods for treating a disease or disorder in a subject in need thereof comprising the administration of recombinant AAV particles comprising an expression cassette comprising a promoter comprising the nucleotide sequence of SEQ ID NO:1 or SEQ ID NO:2 operably linked to a transgene encoding a therapeutic for treatment of said disease or disorder. In some aspects, the transgene can be any one or more of the transgenes disclosed herein, for example, a gene or nucleic acid encoding any of the therapeutics listed in Tables 7A-7B. In some aspects, the transgene encodes a muscle-specific disease therapeutic, such as a Microdystrophin, for example the microdystrophin Dys1, having an amino acid sequence of SEQ ID NO:35, Dys3 (having an amino acid sequence of SEQ ID NO: 36), Dys5 (having an amino acid sequence of SEQ ID NO: 37), MD1 (having an amino acid sequence of SEQ ID NO: 56), human microdystrophin (having an amino acid sequence of SEQ ID NO: 57), Dys3978 (having an amino acid sequence of SEQ ID NO: 58), MD3 (having an amino acid sequence of SEQ ID NO: 59),MD4 (having an amino acid sequence of SEQ ID NO: 60) or MD5 (SEQ ID NO: 71). In some aspects, the microdystrophin transgene is encoded by a nucleotide sequence comprising SEQ ID NO: 67, SEQ ID NO: 68, SEQ ID NO: 69, SEQ ID NO: 70 or SEQ ID NO: 71, or a nucleotide sequence at least about 90%, at least about 91%, at least about 92%, at least about 93%, at least about 94%, at least about 95%, at least about 96%, at least about 97%, at least about 98% or at least about 99% identical to SEQ ID NO: 67, SEQ ID NO: 68, SEQ ID NO: 69, SEQ ID NO: 70 or SEQ ID NO: 71. In some aspects, the recombinant AAV particles comprise an expression cassette of SEQ ID NO:38 or SEQ ID NO: 40. In some aspects, the recombinant AAV particles comprise SEQ ID NO:39 or SEQ ID NO: 41. In some aspects of the treatment methods, the disease or disorder is any of the diseases or disorders listed in Tables 7A-7B. In some aspects, the disease or disorder is a muscle-specific disease or disorder, such as, but not limited to, a muscular dystrophy.

Following transduction of target cells, the expression of the protein product is enhanced by employing such muscle-specific expression cassettes. Such enhancement may be measured by the following non-limiting list of determinations such as 1) protein titer by assays known to the skilled person, not limited to sandwich ELISA, Western Blot, histological staining, and liquid chromatography tandem mass spectrometry (LC-MS/MS); 2) protein activity, by assays such as binding assays, functional assays, enzymatic assays and/or substrate detection assays; and/or 3) serum half-life or long-term expression; and/or (4) detection of mRNA encoding the therapeutic protein of the transgene. Enhancement of transgene expression may be determined as efficacious and suitable for human treatment (Hintze, J.P. et al, Biomarker Insights 2011:6 69-78). Assessment of the quantitative and functional properties of a transgene using such in vitro and in vivo cellular, blood and tissue studies have been shown to correlate to the efficacy of certain therapies (Hintze, J.P. et al, 2011, supra), and are utilized to evaluate response to gene therapy treatment of the transgene with the vectors described herein.

rAAV vectors of the invention also can facilitate delivery, in particular, targeted delivery, of transgenes operably linked to the regulatory sequences described herein, including but not limited to oligonucleotides, drugs, imaging agents, inorganic nanoparticles, liposomes, antibodies to target cells or tissues. The rAAV vectors also can facilitate delivery, in particular, targeted delivery, of non-coding DNA, RNA, or oligonucleotides to target tissues.

The agents may be provided as pharmaceutically acceptable compositions as known in the art and/or as described herein. In some embodiments, the rAAV molecule may be administered alone or in combination with other prophylactic and/or therapeutic agents.

The dosage amounts and frequencies of administration provided herein are encompassed by the terms therapeutically effective and prophylactically effective. The dosage and frequency will typically vary according to factors specific for each patient depending on the specific therapeutic or prophylactic agents administered, the severity and type of disease, the route of administration, as well as age, body weight, response, and the past medical history of the patient, and should be decided according to the judgment of the practitioner and each patient's circumstances. Suitable regimens can be selected by one skilled in the art by considering such factors and by following, for example, dosages reported in the literature and recommended in the Physician 's Desk Reference (56th ed., 2002). Prophylactic and/or therapeutic agents can be administered repeatedly. Several aspects of the procedure may vary such as the temporal regimen of administering the prophylactic or therapeutic agents, and whether such agents are administered separately or as an admixture.

The amount of an agent of the invention that will be effective can be determined by standard clinical techniques. Effective doses may be extrapolated from dose-response curves derived from in vitro or animal model test systems. For any agent used in the method the therapeutically effective dose can be estimated initially from cell culture assays. A dose may be formulated in animal models to achieve a circulating plasma concentration range that includes the IC50 (i.e., the concentration of the test compound that achieves a half-maximal inhibition of symptoms) as determined in cell culture. Such information can be used to more accurately determine useful doses in humans. Levels in plasma may be measured, for example, by high performance liquid chromatography.

Prophylactic and/or therapeutic agents, as well as combinations thereof, can be tested in suitable animal model systems prior to use in humans. Such animal model systems include, but are not limited to, rats, mice, chicken, cows, monkeys, pigs, dogs, rabbits, etc. Any animal system well-known in the art may be used. Such model systems are widely used and well known to the skilled artisan. In some preferred embodiments, animal model systems for a muscle condition are used that are based on rats, mice, or other small mammal other than a primate, such as, but not limited to, *mdx* mouse models.

Once the prophylactic and/or therapeutic agents of the invention have been tested in an animal model, they can be tested in clinical trials to establish their efficacy. Establishing clinical trials will be done in accordance with common methodologies known to one skilled in the art, and the optimal dosages and routes of administration as well as toxicity profiles of agents of the invention can be established. For example, a clinical trial can be designed to test a rAAV molecule of the invention for efficacy and toxicity in human patients.

Toxicity and efficacy of the prophylactic and/or therapeutic agents of the instant invention can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Prophylactic and/or therapeutic agents that exhibit large therapeutic indices are preferred. While prophylactic and/or therapeutic agents that exhibit toxic side effects may be used, care should be taken to design a delivery system that targets such agents to the site of affected tissue in order to minimize potential damage to uninfected cells and, thereby, reduce side effects.

A rAAV molecule of the invention generally will be administered for a time and in an amount effective for obtain a desired therapeutic and/or prophylactic benefit. The data obtained from the cell culture assays and animal studies can be used in formulating a range and/or schedule for dosage of the prophylactic and/or therapeutic agents for use in humans. The dosage of such agents lies preferably within a range of circulating concentrations that include the ED50 with little or no toxicity. The dosage may vary within this range depending upon the dosage form employed and the route of administration utilized.

A therapeutically effective dosage of an rAAV vector for patients is generally from about 0.1 ml to about 100 ml of solution containing concentrations of from about 1x10⁹ to about 1x10¹⁶ genomes rAAV vector, or about 1x10¹⁰ to about 1x10¹⁵, about 1x10¹² to about 1x10¹⁶, or about 1x10¹⁴ to about 1x10¹⁶ AAV genomes. Levels of expression of the transgene can be monitored to determine/adjust dosage amounts, frequency, scheduling, and the like.

Treatment of a subject with a therapeutically or prophylactically effective amount of the agents of the invention can include a single treatment or, in certain embodiments, can include one or more administrations.

Methods of administering agents of the invention include, but are not limited to, parenteral administration (e.g., intradermal, intramuscular, intraperitoneal, intravenous, and subcutaneous, including infusion or bolus injection), epidural, and by absorption through epithelial or mucocutaneous or mucosal linings (e.g., intranasal, oral mucosa, rectal, and intestinal mucosa, etc.).

In certain embodiments, the agents of the invention are administered intravenously or intramuscularly and may be administered together with other biologically active agents.

In another specific embodiment, agents of the invention may be delivered in a sustained release formulation, e.g., where the formulations provide extended release and thus extended half-life of the administered agent. Controlled release systems suitable for use include, without limitation, diffusion-controlled, solvent-controlled, and chemically-controlled systems. Diffusion controlled systems include, for example reservoir devices, in which the molecules of the invention are enclosed within a device such that release of the molecules is controlled by permeation through a diffusion barrier. Common reservoir devices include, for example, membranes, capsules, microcapsules, liposomes, and hollow fibers. Monolithic (matrix) device are a second type of diffusion controlled system, wherein the dual antigen-binding molecules are dispersed or dissolved in a rate-controlling matrix (e.g., a polymer matrix). Agents of the invention can be homogeneously dispersed throughout a rate-controlling matrix and the rate of release is controlled by diffusion through the matrix. Polymers suitable for use in the monolithic matrix device include naturally occurring polymers, synthetic polymers and synthetically modified natural polymers, as well as polymer derivatives.

Any technique known to one of skill in the art can be used to produce sustained release formulations comprising one or more agents described herein. See, e.g. U.S. Pat. No. 4,526,938; PCT publication WO 91/05548; PCT publication WO 96/20698; Ning et al., "Intratumoral Radioimmunotheraphy of a Human Colon Cancer Xenograft Using a Sustained-Release Gel," Radiotherapy & Oncology, 39:179 189, 1996; Song et al., "Antibody Mediated Lung Targeting of Long-Circulating Emulsions," PDA Journal of Pharmaceutical Science & Technology, 50:372 397, 1995; Cleek et al., "Biodegradable Polymeric Carriers for a bFGF Antibody for Cardiovascular Application," Pro. Intl. Symp. Control. Rel. Bioact. Mater., 24:853 854, 1997; and Lam et al., "Microencapsulation of Recombinant Humanized Monoclonal Antibody for Local Delivery," Proc. Int'l. Symp. Control Rel. Bioact. Mater., 24:759 760, 1997. In one embodiment, a pump may be used in a controlled release system (see Langer, supra; Sefton, CRC Crit. Ref. Biomed. Eng., 14:20, 1987; Buchwald et al., Surgery, 88:507, 1980; and Saudek et al., N. Engl. J. Med., 321:574, 1989). In another embodiment, polymeric materials can be used to achieve controlled release of agents comprising dual antigen-binding molecule, or antigen-binding fragments thereof (see e.g., Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, N.Y. (1984); Ranger and Peppas, J., Macromol. Sci. Rev. Macromol. Chem., 23:61, 1983; see also Levy et al., Science, 228:190, 1985; During et al., Ann. Neurol., 25:351, 1989; Howard et al., J. Neurosurg., 7 1:105, 1989); U.S. Pat. No. 5,679,377; U.S. Pat. No. 5,916,597; U.S. Pat. No. 5,912,015; U.S. Pat. No. 5,989,463; U.S. Pat. No. 5,128,326; PCT Publication No. WO 99/15154; and PCT Publication No. WO 99/20253). In yet another embodiment, a controlled release system can be placed in proximity of the therapeutic target (e.g., an affected joint), thus requiring only a fraction of the systemic dose (see, e.g., Goodson, in Medical Applications of Controlled Release, supra, vol. 2, pp. 115 138 (1984)). Other controlled release systems are discussed in the review by Langer, Science, 249:1527 1533, 1990.

In addition, the rAAVs can be used for in vivo delivery of transgenes for scientific studies such as gene knock-down with miRNAs, recombinase delivery for conditional gene deletion, gene editing with CRISPRs, and the like.

### F. Methods of Increasing Packaging Efficiency

Disclosed are methods of increasing packaging efficiency of recombinant AAVs by using one or more of the modified Spc5-12 promoters described herein, Spc5-12v1 (SEQ ID NO: 1) or Spc5-12v2 (SEQ ID NO: 2) in place of an unmodified Spc5-12 promoter. Packaging efficiency includes increasing the proportion or percentage of produced capsids that encapsidate a full rAAV genome, with reduced proportion of produced capsids being empty or containing genome fragments or contaminating non-genome sequences.

For example, disclosed are methods of increasing packaging efficiency of recombinant AAVs by making the recombinant AAVs using one or more of the methods described herein. For example, disclosed are methods of increasing packaging efficiency of recombinant AAVs comprising (a) culturing a host cell containing: (i) an artificial genome comprising a *cis* expression cassette flanked by AAV ITRs, wherein the *cis* expression cassette comprises comprising a promoter operably linked to a transgene coding for one or more RNA or protein products, wherein the promoter comprises the nucleotide sequence of SEQ ID NO: 1 or SEQ ID NO:2; (ii) *a trans* expression cassette lacking AAV ITRs, wherein the trans expression cassette encodes an AAV rep and an AAV capsid protein operably linked to expression control elements that drive expression of the AAV rep and the AAV capsid protein in the host cell in culture and supply the AAV rep and the AAV capsid protein in *trans*; (iii) sufficient adenovirus helper functions to permit replication and packaging of the artificial genome by the AAV capsid protein; and (b) recovering recombinant AAV encapsidating the artificial genome from the cell culture; wherein the presence of the promoter comprising the nucleotide sequence of SEQ ID NO:1 or SEQ ID NO:2 allows for increased packaging efficiency. In certain embodiments, the transgene is a muscle-specific therapeutic. In certain embodiments, the muscle-specific therapeutic is a microdystrophin. In certain embodiments, the microdystrophin is Dys1 (SEQ ID NO: 35), Dys3 (SEQ ID NO: 36), Dys5 (SEQ ID NO: 37), MD1 (SEQ ID NO: 56), human microdystrophin (SEQ ID NO: 57), Dys3978 (SEQ ID NO: 58), MD3 (SEQ ID NO: 59),MD4 (SEQ ID NO: 60) or MD5 (SEQ ID NO: 72). In certain embodiments, the microdystrophin is Dys1 (SEQ ID NO: 35). In embodiments, the microdystrophin transgene is encoded by a nucleotide sequence comprising SEQ ID NO: 67, SEQ ID NO: 68, SEQ ID NO: 69, SEQ ID NO: 70 or SEQ ID NO: 71, or a nucleotide sequence at least about 90%, at least about 91%, at least about 92%, at least about 93%, at least about 94%, at least about 95%, at least about 96%, at least about 97%, at least about 98% or at least about 99% identical to SEQ ID NO: 67, SEQ ID NO: 68, SEQ ID NO: 69, SEQ ID NO: 70 or SEQ ID NO: 71. In certain embodiments, the expression cassette comprises a nucleic acid sequence comprising SEQ ID NO: 38. In certain embodiments, the expression cassette comprises a nucleic acid sequence comprising SEQ ID NO: 40. In certain embodiments, the artificial genome comprises SEQ ID NO: 39. In certain embodiments, the artificial genome comprises SEQ ID NO: 40.

In some aspects, the increased packaging efficiency is compared to production of recombinant AAVs comprising an unmodified Spc5-12 promoter (e.g., the only difference in the *cis* plasmid construct being the promoter).

In some aspects, increased packaging efficiency is production of a population of rAAVs in which at least 30% of the recombinant AAVs have the full AAV genome. In some aspects, increased packaging efficiency is production of at least 20%, 25%, 30%, 35%, 40%, 45%, 50%, 55%, 60%, 65%, 70%, 75%, 80%, 85%, 90% or 95% of the recombinant AAVs having the full (e.g. intact) AAV genome rather than an empty capsid or containing a fragment of the AAV genome or contaminating nucleic acid. In some aspects, increased packaging efficiency means there are at least one-fold, two-fold, three-fold, 4-fold or 5-fold more full capsids produced than rAAVs comprising a comparable genome having a recombinant expression cassette comprising an unmodified Spc5-12 promoter produced using the same method.

In some aspects, increased packaging efficiency means the recombinant AAVs have at least two-fold more full capsids than partial capsids.

### G. Pharmaceutical Compositions and Kits

The invention further provides a pharmaceutical composition comprising a pharmaceutically acceptable carrier and an agent of the invention, said agent comprising a rAAV molecule of the invention comprising a transgene cassette wherein the transgene expression is driven by the chimeric regulatory elements described herein. In preferred embodiments, the pharmaceutical composition comprises rAAV combined with a pharmaceutically acceptable carrier for administration to a subject. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant (e.g., Freund's complete and incomplete adjuvant), excipient, or vehicle with which the agent is administered. Such pharmaceutical carriers can be sterile liquids, such as water and oils, including those of petroleum, animal, vegetable, or synthetic origin, including, e.g., peanut oil, soybean oil, mineral oil, sesame oil and the like. Water is a common carrier when the pharmaceutical composition is administered intravenously or intramuscularly. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Additional examples of pharmaceutically acceptable carriers, excipients, and stabilizers include, but are not limited to, buffers such as phosphate, citrate, and other organic acids; antioxidants including ascorbic acid; low molecular weight polypeptides; proteins, such as serum albumin and gelatin; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, polyethylene glycol (PEG), and PLURONICS^{™} as known in the art. The pharmaceutical composition of the present invention can also include a lubricant, a wetting agent, a sweetener, a flavoring agent, an emulsifier, a suspending agent, and a preservative, in addition to the above ingredients. These compositions can take the form of solutions, suspensions, emulsion, tablets, pills, capsules, powders, sustained-release formulations and the like.

In certain embodiments of the invention, pharmaceutical compositions are provided for use in accordance with the methods of the invention, said pharmaceutical compositions comprising a therapeutically and/or prophylactically effective amount of an agent of the invention along with a pharmaceutically acceptable carrier.

In preferred embodiments, the agent of the invention is substantially purified (i.e., substantially free from substances that limit its effect or produce undesired side-effects). In a specific embodiment, the host or subject is an animal, preferably a mammal such as non-primate (e.g., cows, pigs, horses, cats, dogs, rats etc.) and a primate (e.g., monkey such as, a cynomolgus monkey and a human). In a preferred embodiment, the host is a human.

Further disclosed are kits that can be used in the above methods. In one embodiment, a kit comprises one or more agents of the invention, e.g., in one or more containers. In another embodiment, a kit further comprises one or more other prophylactic or therapeutic agents useful for the treatment of a condition, in one or more containers.

The agents of the invention may be packaged in a hermetically sealed container such as an ampoule or sachette indicating the quantity of the agent or active agent. In one embodiment, the agent is supplied as a dry sterilized lyophilized powder or water free concentrate in a hermetically sealed container and can be reconstituted, e.g., with water or saline, to the appropriate concentration for administration to a subject. Typically, the agent is supplied as a dry sterile lyophilized powder in a hermetically sealed container at a unit dosage of at least 5 mg, more often at least 10 mg, at least 15 mg, at least 25 mg, at least 35 mg, at least 45 mg, at least 50 mg, or at least 75 mg. The lyophilized agent should be stored at between 2 and 8°C in its original container and the agent should be administered within 12 hours, usually within 6 hours, within 5 hours, within 3 hours, or within 1 hour after being reconstituted. In an alternative embodiment, an agent of the invention is supplied in liquid form in a hermetically sealed container indicating the quantity and concentration of agent or active agent. Typically, the liquid form of the agent is supplied in a hermetically sealed container at least 1 mg/ml, at least 2.5 mg/ml, at least 5 mg/ml, at least 8 mg/ml, at least 10 mg/ml, at least 15 mg/kg, or at least 25 mg/ml.

The compositions of the invention include bulk drug compositions useful in the manufacture of pharmaceutical compositions (e.g., impure or non-sterile compositions) as well as pharmaceutical compositions (i.e., compositions that are suitable for administration to a subject or patient). Bulk drug compositions can be used in the preparation of unit dosage forms, e.g., comprising a prophylactically or therapeutically effective amount of an agent disclosed herein or a combination of those agents and a pharmaceutically acceptable carrier.

Further disclosed are a pharmaceutical pack or kit comprising one or more containers filled with one or more of the agents of the invention. Additionally, one or more other prophylactic or therapeutic agents useful for the treatment of the target disease or disorder can also be included in the pharmaceutical pack or kit. Additionally disclosed are a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use, or sale for human administration.

Generally, the ingredients of compositions of the invention are supplied either separately or mixed together in unit dosage form, for example, as a dry lyophilized powder or water-free concentrate in a hermetically sealed container such as an ampoule or sachette indicating the quantity of agent or active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

### Examples

### A. Example 1: Analysis of promoter chimeric repeat sequences and loop formation

Synthetic Promoter c5-12 (Li, X. et al. Nature Biotechnology Vol. 17, pp. 241-245, MARCH 1999), known as the SPc5-12 promoter, has been shown to have cell type restricted expression, specifically muscle-cell specific expression. At less than 350 bp in length, the SPc5-12 promoter is smaller in length than most endogenous promoters, which can be advantageous when the length of the nucleic acid encoding the therapeutic protein is relatively long. The nucleotide sequence of one strand of the Spc5-12 promoter is disclosed herein as SEQ ID NO: 3 (see Table 1, *supra*).

Xie et al (2017, Molecular Therapy, 25, 1363-1375) hypothesized that hairpin loops, such as shRNA transgenes, within or proximal to inverted terminal repeats (ITRs) of an AAV genome packaging cassette may produce a population of truncated or defective genomes. The SPc5-12 promoter sequence was analysed to identify chimeric repeat sequences that may be prone to hairpin loop formation. MEF-2 tandem repeats in opposite orientation (5'-CGCTCTAAAAAATAACTCCCGGGAGTTATTTTTAGAGCG-3'; SEQ ID NO: 4) within the Spc5-12 synthetic promoter were, for example, analysed for predictive thermodynamic ensemble (hairpin assembly). The free energy of the thermodynamic ensemble is -22.84 kcal/mol, the frequency of the minimum free energy (MFE) structure in the ensemble is 79.08%, and the ensemble diversity is 0.36 for this particular tandem TFRE orientation of MEF-2 repeats. In some aspects, RNAfold can be used to calculate the free energy. The RNAfold program was used to calculate secondary structures of RNAs. The free energy of the thermodynamic ensemble (each potential structure), as performed by the RNAfold server, http://ma.tbi.univie.ac.at/cgi-bin/RNAWebSuite/RNAfold.cgi, is calculated to determine the lowest energy structure, i.e. minimum free energy (MFE), that is the most stable thermodynamically (Mathews DH, Disney MD, Childs JL, Schroeder SJ, Zuker M, Turner DH (2004)) Incorporating chemical modification constraints into a dynamic programming algorithm for prediction of RNA secondary structure. Proc Natl Acad Sci U S A 101(19):7287-92.; Gruber AR, Lorenz R, Bernhart SH, Neuböck R, Hofacker IL. The Vienna RNA Websuite. Nucleic Acids Res. 2008). The frequency of the MFE structure is the probability of a single structure in the Boltzmann weighted ensemble of all structures. The ensemble diversity reveals the average base-pair distance between all structures in the thermodynamic ensemble. Low frequency of the MFE structure and low diversity indicate the favorability of a thermodynamically stable structure.

Analysis of SRE-TEF-1-SRE tandem sequences (5'-GGACACCCAAATATGGCGACGGTTCCTCACCCGTCGCCATATTTGGGTGTCC-3'; SEQ ID NO: 14) within the Spc5-12 synthetic promoter revealed that the free energy of the thermodynamic ensemble is -43.25 kcal/mol, the frequency of the MFE structure in the ensemble is 92.80%, and the ensemble diversity is 0.29 for this particular tandem arrangement of transcription factor regulatory elements (TFREs) containing SRE repeats in opposite orientation.

Based on the calculated energies, stem-stem formation of MEF-2 tandem repeats and stem-loop-stem formation of SRE-TEF-1-SRE elements were predicted, and modifications were sought to ameliorate potential genome instability that may interfere with rAAV vector replication.

### B. Example 2: Characterization of partially-filled capsids

Recombinant AAV vectors were produced by triple transfection of three plasmids 1) a plasmid containing a polynucleotide encoding a functional rep gene and a polynucleotide encoding a capsid gene, 2) a plasmid containing a genome to be packaged into a capsid (comprises at least one AAV inverted terminal repeat (ITR) and a non-AAV nucleic acid sequence encoding a therapeutic gene product operably linked to sequences which direct expression of the gene product in a target cell, such as the Spc5-12 promoter (SEQ ID NO:3), and 3) a polynucleotide comprising sufficient helper functions to permit packaging of the genome into the AAV capsid protein under conditions which permit packaging of the genome into the AAV capsid. Following transfection in HEK293 cells, the cells were maintained in cell culture under conditions that allow production of the rAAV particles.

### 1. Analytical Ultracentrifugation (AUC) Characterization

The presence of vectors with fragmented genomes and non-transgene-related DNA contaminants, often referred to as partially-filled capsids, can be resolved by Analytical Ultracentrifugation (AUC). Separate purification fractions were tested: ultracentrifugation (UC) Pool 1, UC Pool 3 and UC Pool 4. UC Pool 1 (contained 98% full particles, whereas, UC Pool 3 contained the highest number of partially-filled capsids at 51%, and UC Pool 3 contained 87% empty capsids.

### 2. Next-Generation Sequencing (NGS)

Next generation sequencing of the genomes within the produced AAV capsids can be used to assess the completeness of the packaged genomes. Long read information for AAV capsids containing genomes with the Spc-5-12 promoter was generated using an Oxford Nanopore technique.

The partially-filled and empty capsids do not support transgene expression and NGS data suggests that the DNA fragments in these partially-filled capsids primarily consist of chimeric DNA with repeated sequences of various sizes from the SPc5-12 promoter region (not the therapeutic gene). However, overall low ratio of full particles compromises total yield of viral particles in the production process.

### C. Example 3: Improved design for Spc5-12 promoter to minimize secondary structure and reduce partial AAV genome

To minimize the formation of hairpin loops in the Spc5-12 promoter, the position of MEF-2 and SRE regulatory sequences were modified and resulted in two variant promoters, Spc5-12v1 (SEQ ID NO: 1) and Spc5-12v2 (SEQ ID NO: 2).

FIG. 1 shows Spc5-12 Mutant1 (SEQ ID NO:1) in which the MEF-2 and SRE elements are exchanged relative to the Spc5-12 promoter sequence. Results for thermodynamic ensemble prediction of the structure on the left shows the free energy of the thermodynamic ensemble is -8.40 kcal/mol. The frequency of the MFE structure in the ensemble is 72.26%. The ensemble diversity is 0.84. Results for thermodynamic ensemble prediction of the structure on the right shows the free energy of the thermodynamic ensemble is -21.42 kcal/mol. The frequency of the MFE structure in the ensemble is 30.84%. The ensemble diversity is 2.55.

FIG. 2 shows Spc5-12 Mutant2 (SEQ ID NO:2) with MEF-2 and SRE in reverse orientation relative to Spc5-12. Results for thermodynamic ensemble prediction of the structure on the left shows the free energy of the thermodynamic ensemble is -3.11 kcal/mol. The frequency of the MFE structure in the ensemble is 26.99%. The ensemble diversity is 9.65. Results for thermodynamic ensemble prediction of the structure on the right shows the free energy of the thermodynamic ensemble is -7.79 kcal/mol. The frequency of the MFE structure in the ensemble is 6.49%. The ensemble diversity is 13.48.

FIG. 3 summarizes the assessment of the secondary structures of Spc5-12 promoter and variants 1 and 2, from FIGs. 1 and 2. Switching position or reversing orientation of the SRE and MEF2 elements as shown is predicted to reduce hairpin formation while not impacting TFRE binding and, therefore, not alter Spc5-12 promoter strength and specificity. Table 8 summarizes the number of nucleotides forming a stem pair, the free energy, and the probability of stem pair formation of the first and second hairpins of Spc5-12, Spc5-12v1 and Spc5-12v2.

**Table 8**

| | Changes | 1^{st} hair pin stem pair | 1^{st} hairpin free energy (kcal/mol) | 1^{st} hairpin probability | 2^{nd} hairpin stem pair | 2^{nd} hairpin free energy (kcal/mol) | 2^{nd} hairpin probability |
|---|---|---|---|---|---|---|---|
| Original Spc5-12 | N/A | 19 | -22.84 | 79.08% | 22 | -43.25 | 92.80% |
| Mutant 1 Spc5-12 | Switch position SRE and MEF2 | 5 | -8.40 | 72.26% | 9 | -21.42 | 30.84% |
| Mutant 2 Spc5-12 | Change orientation of SRE and MEF2 | N/A | -3.11 | 26.99% | 3 | -7.79 | 6.49% |

### D. Example 4: Assessment of modified Spc5-12 promoters on production process and genome integrity

Impact of the modified promoters compared to unmodified Spc5-12 on production of recombinant AAV virions containing a microdystrophin encoding transgene was assessed.

Small scale rAAV production was carried using *cis* plasmids having a microdystrophin (Dys1) transgene (encoding the amino acid sequence of SEQ ID NO:35) and varying only by the promoter (VC160: Spc5-12 mutant 1 promoter; VS161: Spc5-12 mutant 2 promoter; VC162: Syn100 promoter; VS163: CK7 promoter and Control: Spc5-12 promoter). The production was carried out in 2L WV in 5L Thomson Shake Flasks (no baffles) inoculated at ρ: 1.3x10⁶vc/mL; dilution a day before transfection; and transfection at ρ: 5.0x10⁶vc/mL.

FIG. 4 shows the viable cell density and cell viability for the cells having the different promoters in the *cis* plasmids, showing that the VCD and cell viability are comparable. Pre-lysis and post-lysis titers are provided in Table 9:

**Table 9 - Titers**

| Promoter | Pre-Lysis Titer (gc/mL) | Post-Lysis Titer (gc/mL) |
|---|---|---|
| Spc5-12 mutant 1 | 1.21E+11 | 1.75E+11 |
| Spc5-12 mutant 2 | 1.3E+11 | 1.98E+11 |
| Syn100 | 9.1E+10 | 1.31E+11 |
| CK7 | 7.5E+10 | 1.11E+11 |
| Spc5-12 | 5.9E+10 | 8.83E+10 |

The produced capsid samples were then analyzed for total capsid numbers after affinity purification step and at the end of the batch production process (bulk drug substance (BDS)) (FIG. 5 and Tables 10 and 11). Comparing the constructs with the modified promoters compared to the construct with the Spc5-12 promoter, the Spc5-12 mutant promoters had higher amounts of capsid, particularly for the constructs with the mutant 2 (Spc5-12 v2; SEQ ID NO: 2) promoter.

**Table 10 - Capsid Number (Fold)**

| Promoter | Affinity Peak Area | BDS ddPCR |
|---|---|---|
| Spc5-12 mutant 1 | 1.0 | 2.3 |
| Spc5-12 mutant 2 | 1.1 | 3.3 |
| Syn100 | 1.1 | 1.8 |
| CK7 | 1.0 | 1.1 |

**Table 11 - Percentage of Full Capsid**

| Promoter | Affinity (% full by OD) | Bulk Drug Substance (BDS) (% full by OD) |
|---|---|---|
| Spc5-12 mutant 1 | 14.6 | 33.7 |
| Spc5-12 mutant 2 | 15.9 | 34.9 |
| Syn100 | 11.9 | 26.6 |
| CK7 | 9.9 | 19.4 |
| Control | 10.4 | 22.8 |

Genome integrity was also assessed by viral DNA extraction and tape station analysis. FIG. 6 shows the results of a DNA TapeStation analysis (Agilent Technologies, Santa Clara, CA) for analyzing annealed double strand DNA, which data is also summarized in Table 12 below.

**Table 12 - Genomic Integrity**

| Promoter | Full | Partial | %Full |
|---|---|---|---|
| Spc5-12 | 11.5 | 71.46 | 13.8% |
| Spc5-12 mutant 1 | 19.92 | 26.89 | 42.5% |
| Spc5-12 mutant 2 | 29.93 | 15.49 | 65.3% |
| Syn100 | 24.17 | 34.89 | 40.9% |
| CK7 | 22.23 | 36.47 | 37.8% |

The DNA tape kit analysis permits the assessment of the length of viral DNA to provide an assessment of the proportion of partial (containing genome fragments) versus full (intact genomes). The construct having the mutant 2 promoter (Spc5-12 v2) had the highest percentage of full capsids-65.3% as compared to 13.9% for the rAAVs from the construct with the unmodified Spc5-12 promoter. The mutant 1 construct resulted in 42.5% full capsids.

The produced capsids were also analyzed by ultracentrifugation on CsCl gradients for the proportion of empty, partial and full capsids in the preparations generated from constructs having the mutant 2 promoter as compared to the unmodified Spc5-12 promoter. FIGs. 7A and 7B show the sedimentation coefficient profile of the Spc5-12 (control) (A) and Mutant 2 (B) samples. The full to partial capsid ratio increased ~4 fold with the Mutant 2 redesign, and resulted in 42% full capsids as compared to 22% full for the control.

Next generation sequencing (by Oxford Nanopore) was also used to assess the integrity of the genomes in capsids having the redesigned promoters compared to the unmodified Spc5-12 control. FIG. 8 shows that control Spc5-12 promoter has a much lower percent of full length reads compared to the mutant 1 and mutant 2 promoters.

### E. Example 5: Transgene expression and biodistribution of rAAV constructs containing the modified Spc5 promoters

C57Bl/6J mice at 5-6 weeks of age were administered constructs as indicated in Table 13 below.

**Table 13**

| Dose | # mice | transgene | promoter | Capsid | Titer | % similarly processed DMD002 |
|---|---|---|---|---|---|---|
| 1.00E+14 | 5 | DYS1 | Spc5.12 | AAV8 | 1.13E+14 | 100.00 |
| 1.00E+14 | 5 | DYS1 | Spc5.12-mutant 1 | AAV8 | 2.17E+14 | 192.04 |
| 1.00E+14 | 5 | DYS1 | Spc5.12-mutant 2 | AAV8 | 3.11E+14 | 275.22 |

Six weeks after injection, mice were euthanized and tissue samples were taken. rAAV genome DNA and transgene expressed mRNA were analyzed in in left TA (tibialis anterior) muscle, left GAS (gastrocnemius) muscle, diaphragm, liver, and/or heart. Microdystrophin protein levels were assessed by JESS (ProteinSimple) capillary-based Western immunoassay analysis in right gastrocnemius, diaphragm and heart tissue. Immunohistochemistry was also performed in the right quadricep for microdystrophin and degeneration/regeneration (DAPI/eMyHC). This study also compares µDys DNA/RNA levels in select muscles after systemic administration of µDys under control of modified Spc5.12, ensures plasma membrane localization of µDys in skeletal muscle, and compares µDys DNA/RNA in non-muscle tissues.

The expression (as detected by microdystrophin RNA copies by ddPCR) of the microdystrophin transgene was slightly increased from the constructs with the Spc5.12 mutants V1 and V2 in heart and skeletal muscle as compared to Spc5-12 (FIGs. 9 and 10). Liver expression in mice from all three experimental groups is low and remains unaffected by promoter modification. Figures 9 and 10 differ by the normalization of RNA loading, which shows similar results.

Distribution of the AAV genomes in the tissues of the administered mice was also assessed by ddPCR and normalized to diploid genomes or micrograms of DNA. FIGs. 11 (normalized to diploid genomes) and 12 (normalized to micrograms of DNA) show that Spc5.12 mutants V1 and V2 do not significantly alter AAV-DNA biodistribution compared to constructs having the unmodified Spc5-12 promoter. A large amount of the AAV DNA is present in the liver. FIG. 13 shows ratio of the transgene RNA to DNA copies of the AAV genome per diploid cell based upon the data in FIGs. 9 and 11. The data demonstrate the mutant promoters Spc5-12v1 and Spc5-12 v2 have promoter (transcriptional) activity that is essentially equivalent to Spc5-12.

µDys expression is heterogeneous and present in roughly half of muscle fibers (quadricep) as determined using immunohistochemistry. Expression of the µDys is localized to sarcolemma. The muscle was healthy is all mice treated with the AAV vectors.

## Claims

1. A nucleic acid comprising a) the nucleotide sequence of SEQ ID NO:1 or b) a nucleotide sequence having muscle specific promoter activity, at least 80% sequence identity to SEQ ID NO:1, and 100% sequence identity over nucleotides 121-129 and 197-209 of SEQ ID NO:1, wherein the nucleotide sequence promotes expression of an operably linked nucleotide sequence in muscle cells.

2. A nucleic acid comprising a) the nucleotide sequence of SEQ ID NO:2 or b) a nucleotide sequence having muscle-specific promoter activity, at least 80% sequence identity to SEQ ID NO:2, and 100% sequence identity over nucleotides 113-131 and 191-212 of SEQ ID NO:2, wherein the nucleotide sequence promotes expression of an operably linked nucleotide sequence in muscle cells.

3. A nucleic acid comprising a nucleotide sequence of SEQ ID NO:38 or SEQ ID NO:40.

4. A recombinant expression cassette comprising the nucleic acid of any one of claims 1-2.

5. The recombinant expression cassette of claim 4, further comprising a transgene, wherein the transgene comprises a nucleotide sequence encoding SMN, minidystrophin, microdystrophin, human-alpha-sarcoglycan, gamma-sarcoglycan, huFollistatin344, GALGT2, hSGCB, elezanumab, ravulizumab, pamrevlumab, etrolizumab, romosozumab (EVENITY^{®}), Satralizumab, Sarilumab, Tocilizumab, siltuximab, , clazakizumab, sirukumab, olokizumab, gerilimzumab, omolizumab, tezelipumab, benralizumab, reslizumab, tralokinumab, nemolizumab, Ixekizumab, secukinumab, ixekizumab (TALTZ^{®}), secukinumab (COSENTYX^{®}), mepolizumab (NUCALA^{®}), ustekinumab (STELARA^{®}), dupilumab, vedolizumab (ENTYVIO^{®}), Natalizumab (anti-integrin alpha 4), alirocumab (PRALUENT^{®}), evolucomab (REPATHA^{®}), evinacumab, E06-scFv, denosumab (XGEVA^{®} and PROLIA^{®}), nivolumab (OPDIVO^{®}), pembrolizumab (KEYTRUDA^{®}), belimumab (BENLYSTA^{®}), adalimumab (HUMIRA^{®}) and, infliximab (REMICADE^{®}), eculizumab (SOLIRIS^{®}), or lanadelumab.

6. The recombinant expression cassette of claim 5, wherein the microdystrophin comprises Dys1 having an amino acid sequence of SEQ ID NO:35, Dys3 having an amino acid sequence of SEQ ID NO: 36, Dys5 having an amino acid sequence of SEQ ID NO: 37, MD1, having an amino acid sequence of SEQ ID NO: 56, human microdystrophin, having an amino acid sequence of SEQ ID NO: 57, Dys3978, having an amino acid sequence of SEQ ID NO: 58, MD3 having an amino acid sequence of SEQ ID NO: 59, MD4 having an amino acid sequence of SEQ ID NO: 60, or MD5 having an amino sequence of SEQ ID NO: 72, optionally wherein the microdystrophin is encoded by a nucleotide sequence comprising SEQ ID NO: 67, SEQ ID NO: 68, SEQ ID NO: 69, SEQ ID NO: 70 or SEQ ID NO: 71, or a sequence at least 90% identical to SEQ ID NO: 67, SEQ ID NO: 68, SEQ ID NO: 69, SEQ ID NO: 70, or SEQ ID NO: 71.

7. A vector comprising the recombinant expression cassette of any one of claims 4-6 and AAV ITRs flanking the recombinant expression cassette.

8. A rAAV particle comprising the vector of claim 7 and a capsid protein from an AAV capsid serotype selected from AAV1, AAV1, AAV2, rAAV3, AAV4, AAV5, AAV6, AAV7, AAV8, AAV9, AAV10, AAV-11, AAV-12, AAV-13, AAV-14, AAV-15 and AAV-16, AAV.rh8, AAV.rh10, AAV.rh20, AAV.rh39, AAV.Rh74, AAV.RHM4-1, AAVhu32, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, or AAV.HSC16, or a derivative, modification, or pseudotype thereof, optionally wherein the AAV capsid serotype is AAV8 or AAV9.

9. A rAAV particle comprising a nucleic acid comprising SEQ ID NO: 38, SEQ ID NO: 39, SEQ ID NO: 40, or SEQ ID NO: 41, optionally wherein the rAAV particle is an AAV8 or AAV9 serotype.

10. A cell comprising the nucleic acid of any one of claims 1-3, the expression cassette of any one of claims 4-6, or the vector of claim 7.

11. A pharmaceutical composition comprising the recombinant AAV particle of claim 8 for use in treating a disease or disorder in a subject in need thereof, wherein the disease or disorder is Spinal Muscular Atrophy (SMA), Duchenne Muscular Dystrophy, Limb Girdle Muscular Dystrophy Type 2C|Gamma-sarcoglycanopathy, Limb Girdle Muscular Dystrophy Type 2C|Gamma-sarcoglycanopathy, Becker Muscular Dystrophy and Sporadic Inclusion Body Myositis, Duchenne Muscular Dystrophy, Limb-Girdle Muscular Dystrophy, Type 2E, multiple sclerosis, Myasthenia Gravis, fibrotic diseases, e.g. diabetic nephropathy, liver fibrosis, idiopathic pulmonary fibrosis, ulcerative colitis, Crohn's disease, Osteoporosis, abnormal bone loss or weakness, Adverse immune responses (e.g. cytokine storm, CAR-T therapy), Asthma, COPD, eosinophilic asthma, chronic idiopathic urticaria, Asthma, COPD, Asthma, COPD, Asthma, COPD, eosinophilic asthma, Atopic dermatitis, Atopic dermatitis, Plaque psoriasis, psoriatic arthritis, ankylosing sponylitis, Plaque psoriasis, psoriatic arthritis, ankylosing sponylitis, Asthma, Psoriasis & Crohn's disease, Atopic dermatitis, Ulcerative colitis & Crohn's disease, Multiple sclerosis & Crohn's disease, HeFH & HoFH, HoFH & severe forms of dyslipidema, Cardiovascular diseases such as atherosclerosis, Osteoporosis, increasing bone mass in breast and prostate cancer patients, & preventing skeletal-related events due to bone metastasis, Metastatic melanoma, lymphoma, non-small cell lung carcinoma, Systemic lupus erythromatosis, Rheumatoid arthritis, psoriatic arthritis, askylosing spondylitis, Crohn's disease, plaque psoriasis, ulcerative colitis, Paroxysmal nocturnal hemoglobinuria, atypical hemolytic uremic syndrome, complement-mediated thrombotic microangiopathy, or Hereditary angioedema (HAE).

12. A pharmaceutical composition comprising rAAV particles comprising an expression cassette, wherein the expression cassette comprises a promoter comprising the nucleotide sequence of SEQ ID NO: 1 or SEQ ID NO: 2, operably linked to a transgene encoding Dys1 having an amino acid sequence of SEQ ID NO:35, and wherein the rAAV particles are AAV8 for use in treating DMD in a subject in need thereof.

13. A host cell comprising a plasmid comprising a *cis* expression cassette, wherein the *cis* expression cassette comprises a promoter operably linked to a transgene, wherein the promoter comprises a nucleotide sequence of SEQ ID NO:1 or SEQ ID NO:2 or wherein the *cis* expression cassette comprises a nucleotide sequence comprising the nucleotide sequence of SEQ ID NO: 38 or SEQ ID NO: 40.

14. A method of producing recombinant AAVs comprising:
(a) culturing a host cell containing:
(i) an artificial genome comprising a *cis* expression cassette flanked by AAV ITRs, wherein the *cis* expression cassette comprises comprising a promoter operably linked to a transgene coding for one or more RNA or protein products, wherein the promoter comprises the nucleotide sequence of SEQ ID NO:1 or SEQ ID NO:2;
(ii) a *trans* expression cassette lacking AAV ITRs, wherein the trans expression cassette encodes an AAV rep and an AAV capsid protein operably linked to expression control elements that drive expression of the AAV rep and the AAV capsid protein in the host cell in culture and supply the AAV rep and the AAV capsid protein in *trans*;
(iii) sufficient adenovirus helper functions to permit replication and packaging of the artificial genome by the AAV capsid protein; and
(b) recovering recombinant AAV encapsidating the artificial genome from the cell culture,
optionally wherein at least 40% of the rAAVs produced encapsidate a full genome.

15. A method of increasing packaging efficiency of intact AAV genomes in recombinant AAVs comprising
(a) culturing a host cell containing:
(i) an artificial genome comprising a *cis* expression cassette flanked by AAV ITRs, wherein the *cis* expression cassette comprises comprising a promoter operably linked to a transgene coding for one or more RNA or protein products, wherein the promoter comprises the nucleotide sequence of SEQ ID NO:1 or SEQ ID NO:2;
(ii) a *trans* expression cassette lacking AAV ITRs, wherein the trans expression cassette encodes an AAV rep and an AAV capsid protein operably linked to expression control elements that drive expression of the AAV rep and the AAV capsid protein in the host cell in culture and supply the AAV rep and the AAV capsid protein in *trans*;
(iii) sufficient adenovirus helper functions to permit replication and packaging of the artificial genome by the AAV capsid protein; and
(b) recovering recombinant AAV encapsidating the artificial genome from the cell culture;
wherein the presence of the promoter comprising the nucleotide sequence of SEQ ID NO:1 or SEQ ID NO:2 allows for increased packaging efficiency of intact AAV genomes, optionally wherein the increased packaging efficiency of intact AAV genomes is compared to production of recombinant AAVs comprising a wild-type Spc5-12 promoter.

## Patentansprüche

1. Nukleinsäure, umfassend a) die Nukleotidsequenz unter SEQ ID NO:1 oder b) eine Nukleotidsequenz mit muskelspezifischer Promotoraktivität, mindestens 80 % Sequenzidentität mit SEQ ID NO:1 und 100 % Sequenzidentität über die Nukleotide 121-129 und 197-209 von SEQ ID NO:1, wobei die Nukleotidsequenz die Expression einer in operativer Verknüpfung stehenden Nukleotidsequenz in Muskelzellen fördert.

2. Nukleinsäure, umfassend a) die Nukleotidsequenz unter SEQ ID NO:2 oder b) eine Nukleotidsequenz mit muskelspezifischer Promotoraktivität, mindestens 80 % Sequenzidentität mit SEQ ID NO:2 und 100 % Sequenzidentität über die Nukleotide 113-131 und 191-212 von SEQ ID NO:2, wobei die Nukleotidsequenz die Expression einer in operativer Verknüpfung stehenden Nukleotidsequenz in Muskelzellen fördert.

3. Nukleinsäure, umfassend eine Nukleotidsequenz unter SEQ ID NO:38 oder SEQ ID NO:40.

4. Rekombinante Expressionskassette, umfassend die Nukleinsäure nach einem der Ansprüche 1-2.

5. Rekombinante Expressionskassette nach Anspruch 4, ferner umfassend ein Transgen, wobei das Transgen eine Nukleotidsequenz umfasst, die SMN, Minidystrophin, Mikrodystrophin, Human-alpha-Sarkoglykan, Gamma-Sarkoglykan, huFollistatin344, GALGT2, hSGCB, Elezanumab, Ravulizumab, Pamrevlumab, Etrolizumab, Romosozumab (EVENITY^{®}), Satralizumab, Sarilumab, Tocilizumab, Siltuximab, Clazakizumab, Sirukumab, Olokizumab, Gerilimzumab, Omolizumab, Tezelipumab, Benralizumab, Reslizumab, Tralokinumab, Nemolizumab, Ixekizumab, Secukinumab, Ixekizumab (TALTZ^{®}), Secukinumab (COSENTYXO), Mepolizumab (NUCALA^{®}), Ustekinumab (STELARA^{®}), Dupilumab, Vedolizumab (ENTYVIO^{®}), Natalizumab (Anti-Integrin alpha 4), Alirocumab (PRALUENT^{®}), Evolucomab (REPATHA^{®}), Evinacumab, E06-scFv, Denosumab (XGEVA^{®} und PROLIA^{®}), Nivolumab (OPDIVO^{®}), Pembrolizumab (KEYTRUDA^{®}), Belimumab (BENLYSTA^{®}), Adalimumab (HUMIRA^{®}) und, Infliximab (REMICADE^{®}), Eculizumab (SOLIRIS^{®}) oder Lanadelumab codiert.

6. Rekombinante Expressionskassette nach Anspruch 5, wobei das Mikrodystrophin Dys1 mit einer Aminosäuresequenz unter SEQ ID NO:35, Dys3 mit einer Aminosäuresequenz unter SEQ ID NO: 36, Dys5 mit einer Aminosäuresequenz unter SEQ ID NO: 37, MD1 mit einer Aminosäuresequenz unter SEQ ID NO: 56, menschliches Mikrodystrophin mit einer Aminosäuresequenz unter SEQ ID NO: 57, Dys3978 mit einer Aminosäuresequenz unter SEQ ID NO: 58, MD3 mit einer Aminosäuresequenz unter SEQ ID NO: 59, MD4 mit einer Aminosäuresequenz unter SEQ ID NO: 60 oder MD5 mit einer Aminosäuresequenz unter SEQ ID NO: 72 umfasst, gegebenenfalls wobei das Mikrodystrophin durch eine Nukleotidsequenz, die SEQ ID NO: 67, SEQ ID NO: 68, SEQ ID NO: 69, SEQ ID NO: 70 oder SEQ ID NO: 71 umfasst, oder eine Sequenz, die zu mindestens 90 % mit SEQ ID NO: 67, SEQ ID NO: 68, SEQ ID NO: 69, SEQ ID NO: 70 oder SEQ ID NO: 71 identisch ist, codiert ist.

7. Vektor, umfassend die rekombinante Expressionskassette nach einem der Ansprüche 4-6 und AAV-ITRs, die die rekombinante Expressionskassette flankieren.

8. rAAV-Partikel, umfassend den Vektor nach Anspruch 7 und ein Kapsidprotein aus einem AAV-Kapsid-Serotyp, der aus AAV1, AAV1, AAV2, rAAV3, AAV4, AAV5, AAV6, AAV7, AAV8, AAV9, AAV10, AAV-11, AAV-12, AAV-13, AAV-14, AAV-15 und AAV-16, AAV.rh8, AAV.rh10, AAV.rh20, AAV.rh39, AAV.Rh74, AAV.RHM4-1, AAVhu32, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15 oder AAV.HSC16 oder einem Derivat, einer Modifikation, oder einem Pseudotyp davon ausgewählt ist, gegebenenfalls wobei es sich bei dem AAV-Kapsid-Serotyp um AAV8 oder AAV9 handelt.

9. rAAV-Partikel, umfassend eine Nukleinsäure, die SEQ ID NO: 38, SEQ ID NO: 39, SEQ ID NO: 40 oder SEQ ID NO: 41 umfasst, gegebenenfalls wobei es sich bei dem rAAV-Partikel um einen AAV8- oder AAV9-Serotyp handelt.

10. Zelle, umfassend die Nukleinsäure nach einem der Ansprüche 1-3, die Expressionskassette nach einem der Ansprüche 4-6 oder den Vektor nach Anspruch 7.

11. Pharmazeutische Zusammensetzung, umfassend das rekombinante AAV-Partikel nach Anspruch 8, zur Verwendung bei der Behandlung einer Krankheit oder Störung bei einem behandlungsbedürftigen Individuum, wobei es sich bei der Krankheit bzw. Störung um spinale Muskelatrophie (SMA), Duchenne-Muskeldystrophie, Gliedergürtel-Muskeldystrophie Typ 2C|Gamma-Sarkoglykanopathie, Gliedergürtel-Muskeldystrophie Typ 2C|Gamma-Sarkoglykanopathie, Becker-Muskeldystrophie und sporadische Einschlusskörper-Myositis, Duchenne-Muskeldystrophie, Gliedergürtel-Muskeldystrophie Typ 2E, multiple Sklerose, Myasthenia gravis, fibrotische Erkrankungen, z. B. diabetische Nephropathie, Leberfibrose, idiopathische Lungenfibrose, Colitis ulcerosa, Morbus Crohn, Osteoporose, abnormalen Knochenschwund bzw. abnormale Knochenschwäche, unerwünschte Immunreaktionen (z. B. Cytokinsturm, CAR-T-Therapie), Asthma, COPD, eosinophiles Asthma, chronische idiopathische Urtikaria, Asthma, COPD, Asthma, COPD, Asthma, COPD, eosinophiles Asthma, atopische Dermatitis, atopische Dermatitis, Plaque-Psoriasis, Psoriasis-Arthritis, ankylosierende Sponylitis, Plaque-Psoriasis, Psoriasis-Arthritis, ankylosierende Sponylitis, Asthma, Psoriasis & Morbus Crohn, atopische Dermatitis, Colitis ulcerosa & Morbus Crohn, multiple Sklerose & Morbus Crohn, HeFH & HoFH, HoFH & schwere Formen von Dyslipidämie, Herz-Kreislauf-Erkrankungen wie Atherosklerose, Osteoporose, zunehmende Knochenmasse bei Patienten mit Brust- und Prostatakrebs & Vorbeugen skelettbezogener Ereignisse aufgrund von Knochenmetastasen, metastasiertes Melanom, Lymphom, nichtkleinzelliges Lungenkarzinom, systemischen Lupus erythematodes, rheumatoide Arthritis, Psoriasis-Arthritis, Spondylitis ankylosans, Morbus Crohn, Plaque-Psoriasis, Colitis ulcerosa, paroxysmale nächtliche Hämoglobinurie, atypisches hämolytisch-urämisches Syndrom, komplementvermittelte thrombotische Mikroangiopathie oder hereditäres Angioödem (HAE) handelt.

12. Pharmazeutische Zusammensetzung, umfassend eine Expressionskassette umfassende rAAV-Partikel, wobei die Expressionskassette einen Promotor, der die Nukleotidsequenz unter SEQ ID NO: 1 oder SEQ ID NO: 2 umfasst, in operativer Verknüpfung mit einem Transgen, das Dys1 mit einer Aminosäuresequenz unter SEQ ID NO: 35 codiert, umfasst und wobei es sich bei den rAAV-Partikeln um AAV8 handelt, zur Verwendung bei der Behandlung von DMD bei einem behandlungsbedürftigen Individuum.

13. Wirtszelle, umfassend ein Plasmid, das eine cis-Expressionskassette umfasst, wobei die cis-Expressionskassette einen Promotor umfasst, der in operativer Verknüpfung mit einem Transgen steht, wobei der Promotor eine Nukleotidsequenz unter SEQ ID NO:1 oder SEQ ID NO:2 umfasst oder wobei die cis-Expressionskassette eine Nukleotidsequenz umfasst, die die Nukleotidsequenz unter SEQ ID NO: 38 oder SEQ ID NO: 40 umfasst.

14. Verfahren zur Erzeugung rekombinanter AAVs, umfassend:
(a) Kultivieren einer Wirtszelle, die Folgendes enthält:
(i) ein künstliches Genom, das eine cis-Expressionskassette flankiert von AAV-ITRs umfasst, wobei die cis-Expressionskassette einen Promotor in operativer Verknüpfung mit einem Transgen, das ein oder mehrere RNA- oder Proteinprodukte codiert, umfasst, wobei der Promotor die Nukleotidsequenz unter SEQ ID NO:1 oder SEQ ID NO:2 umfasst;
(ii) eine trans-Expressionskassette ohne AAV-ITRs, wobei die trans-Expressionskassette ein AAV-rep und ein AAV-Kapsidprotein in operativer Verknüpfung mit Expressionskontrollelementen, die die Expression des AAV-rep und des AAV-Kapsidproteins in der Wirtszelle in Kultur treiben und das AAV-rep und das AAV-Kapsidprotein in trans liefern;
(iii) hinreichende Adenovirus-Helferfunktionen, um die Replikation und Verpackung des künstlichen Genoms durch das AAV-Kapsidprotein zu ermöglichen; und
(b) Gewinnen von rekombinantem AAV, in dem das künstliche Genom verkapselt ist, aus der Zellkultur, gegebenenfalls wobei in mindestens 40 % der erzeugten rAAV ein vollständiges Genom verkapselt ist.

15. Verfahren zur Erhöhung der Verpackungseffizienz intakter AAV-Genome in rekombinanten AAV, umfassend
(a) Kultivieren einer Wirtszelle, die Folgendes enthält:
(i) ein künstliches Genom, das eine cis-Expressionskassette flankiert von AAV-ITRs umfasst, wobei die cis-Expressionskassette einen Promotor in operativer Verknüpfung mit einem Transgen, das ein oder mehrere RNA- oder Proteinprodukte codiert, umfasst, wobei der Promotor die Nukleotidsequenz unter SEQ ID NO:1 oder SEQ ID NO:2 umfasst;
(ii) eine trans-Expressionskassette ohne AAV-ITRs, wobei die trans-Expressionskassette ein AAV-rep und ein AAV-Kapsidprotein in operativer Verknüpfung mit Expressionskontrollelementen, die die Expression des AAV-rep und des AAV-Kapsidproteins in der Wirtszelle in Kultur treiben und das AAV-rep und das AAV-Kapsidprotein in trans liefern;
(iii) hinreichende Adenovirus-Helferfunktionen, um die Replikation und Verpackung des künstlichen Genoms durch das AAV-Kapsidprotein zu ermöglichen; und
(b) Gewinnen von rekombinantem AAV, in dem das künstliche Genom verkapselt ist, aus der Zellkultur; wobei das Vorhandensein des Promotors, der die Nukleotidsequenz unter SEQ ID NO:1 oder SEQ ID NO:2 umfasst, eine erhöhte Verpackungseffizienz intakter AAV-Genome ermöglicht, gegebenenfalls wobei die erhöhte Verpackungseffizienz intakter AAV-Genome mit der Produktion rekombinanter AAV, die einen Wildtyp-Spc5-12-Promotor umfassen, verglichen wird.

## Revendications

1. Acide nucléique comprenant a) la séquence nucléotidique de SEQ ID NO:1 ou b) une séquence nucléotidique ayant une activité de promoteur spécifique du muscle, au moins 80 % d'identité de séquence avec SEQ ID NO:1, et 100 % d'identité de séquence sur les nucléotides 121-129 et 197-209 de SEQ ID NO:1, dans lequel la séquence nucléotidique favorise l'expression d'une séquence nucléotidique liée de manière fonctionnelle dans des cellules musculaires.

2. Acide nucléique comprenant a) la séquence nucléotidique de SEQ ID NO:2 ou b) une séquence nucléotidique ayant une activité de promoteur spécifique du muscle, au moins 80 % d'identité de séquence avec SEQ ID NO:2, et 100 % d'identité de séquence sur les nucléotides 113-131 et 191-212 de SEQ ID NO:2, dans lequel la séquence nucléotidique favorise l'expression d'une séquence nucléotidique liée de manière fonctionnelle dans des cellules musculaires.

3. Acide nucléique comprenant une séquence nucléotidique de SEQ ID NO:38 ou SEQ ID NO:40.

4. Cassette d'expression recombinante comprenant l'acide nucléique selon l'une quelconque des revendications 1 à 2.

5. Cassette d'expression recombinante selon la revendication 4, comprenant en outre un transgène, dans laquelle le transgène comprend une séquence nucléotidique codant pour SMN, minidystrophine, microdystrophine, alpha-sarcoglycane humain, gamma-sarcoglycane, huFollistatin344, GALGT2, hSGCB, élézanumab, ravulizumab, pamrevlumab, étrolizumab, romosozumab (EVENITY^{®}), Satralizumab, Sarilumab, Tocilizumab, siltuximab, clazakizumab, sirukumab, olokizumab, gérilimzumab, omolizumab, tezelipumab, benralizumab, reslizumab, tralokinumab, némolizumab, Ixekizumab, sécukinumab, ixékizumab (TALTZ^{®}), sécukinumab (COSENTYX^{®}), mépolizumab (NUCALA^{®}), ustékinumab (STELARA^{®}), dupilumab, védolizumab (ENTYVIO^{®}), Natalizumab (anti-intégrine alpha 4), alirocumab (PRALUENT^{®}), évolucomab (REPATHA^{®}), évinacumab, E06-scFv, dénosumab (XGEVA^{®} et PROLIA^{®}), nivolumab (OPDIVO^{®}), pembrolizumab (KEYTRUDA^{®}), belimumab (BENLYSTA^{®}), adalimumab (HUMIRA^{®}) et infliximab (REMICADE^{®}), éculizumab (SOLIRIS^{®}) ou lanadélumab.

6. Cassette d'expression recombinante selon la revendication 5, dans laquelle la microdystrophine comprend Dys1 ayant une séquence d'acides aminés de SEQ ID NO:35, Dys3 ayant une séquence d'acides aminés de SEQ ID NO: 36, Dys5 ayant une séquence d'acides aminés de SEQ ID NO: 37, MD1 ayant une séquence d'acides aminés de SEQ ID NO: 56, la microdystrophine humaine ayant une séquence d'acides aminés de SEQ ID NO: 57, Dys3978 ayant une séquence d'acides aminés de SEQ ID NO: 58, MD3 ayant une séquence d'acides aminés de SEQ ID NO: 59, MD4 ayant une séquence d'acides aminés de SEQ ID NO: 60, ou MD5 ayant une séquence d'acides aminés de SEQ ID NO: 72, la microdystrophine étant éventuellement codée par une séquence nucléotidique comprenant SEQ ID NO: 67, SEQ ID NO: 68, SEQ ID NO: 69, SEQ ID NO: 70 ou SEQ ID NO: 71, ou une séquence au moins 90 % identique à SEQ ID NO: 67, SEQ ID NO: 68, SEQ ID NO: 69, SEQ ID NO: 70 ou SEQ ID NO: 71.

7. Vecteur comprenant la cassette d'expression recombinante selon l'une quelconque des revendications 4 à 6 et des ITR AAV flanquant la cassette d'expression recombinante.

8. Particule de rAAV comprenant le vecteur selon la revendication 7 et une protéine de capside d'un sérotype de capside AAV choisi parmi AAV1, AAV1, AAV2, rAAV3, AAV4, AAV5, AAV6, AAV7, AAV8, AAV9, AAV10, AAV-11, AAV-12, AAV-13, AAV-14, AAV-15 et AAV-16, AAV.rh8, AAV.rh10, AAV.rh20, AAV.rh39, AAV.Rh74, AAV.RHM4-1, AAVhu32, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSCS, AAV.HSC6, AAV.HSC7, AAV.HSCS, AAV.HSC9, AAV.HSC10, AAV.HSC11, AAV.HSC12, AAV.HSC13, AAV.HSC14, AAV.HSC15 ou AAV.HSC16, ou un dérivé, une modification ou un pseudotype de ceux-ci, le sérotype de capside AAV étant éventuellement AAV8 ou AAV9.

9. Particule de rAAV comprenant un acide nucléique comprenant SEQ ID NO: 38, SEQ ID NO: 39, SEQ ID NO: 40 ou SEQ ID NO: 41, facultativement où la particule de rAAV est un sérotype AAV8 ou AAV9.

10. Cellule comprenant l'acide nucléique selon l'une quelconque des revendications 1 à 3, la cassette d'expression selon l'une quelconque des revendications 4 à 6, ou le vecteur selon la revendication 7.

11. Composition pharmaceutique comprenant la particule d'AAV recombinante selon la revendication 8, destinée à être utilisée dans le traitement d'une maladie ou d'un trouble chez un sujet qui en a besoin, dans laquelle la maladie ou le trouble est : amyotrophie spinale (SMA), dystrophie musculaire de Duchenne, dystrophie musculaire des ceintures de type 2C (gamma-sarcoglycanopathie), dystrophie musculaire de Becker et myosite à inclusions sporadique, dystrophie musculaire de Duchenne, dystrophie musculaire des ceintures de type 2E, sclérose en plaques, myasthénie grave, maladies fibrotiques (par ex. néphropathie diabétique, fibrose hépatique, fibrose pulmonaire idiopathique), colite ulcéreuse, maladie de Crohn, ostéoporose, perte osseuse anormale ou fragilité osseuse, réactions immunitaires indésirables (par ex. tempête de cytokines, thérapie par cellules CAR-T), asthme, BPCO, asthme éosinophilique, urticaire chronique idiopathique, dermatite atopique, psoriasis en plaques, rhumatisme psoriasique, spondylarthrite ankylosante, asthme, psoriasis et maladie de Crohn, dermatite atopique, colite ulcéreuse et maladie de Crohn, sclérose en plaques et maladie de Crohn, HeFH et HoFH, HoFH et formes sévères de dyslipidémie, maladies cardiovasculaires telles que l'athérosclérose, ostéoporose, augmentation de la masse osseuse chez les patients atteints de cancer du sein ou de la prostate et prévention des événements squelettiques liés aux métastases osseuses, mélanome métastatique, lymphome, carcinome pulmonaire non à petites cellules, lupus érythémateux disséminé, polyarthrite rhumatoïde, rhumatisme psoriasique, spondylarthrite ankylosante, maladie de Crohn, psoriasis en plaques, colite ulcéreuse, hémoglobinurie paroxystique nocturne, syndrome hémolytique et urémique atypique, microangiopathie thrombotique médiée par le complément ou angio-œdème héréditaire (AOH).

12. Composition pharmaceutique comprenant des particules de rAAV comprenant une cassette d'expression, dans laquelle la cassette d'expression comprend un promoteur comprenant la séquence nucléotidique de SEQ ID NO: 1 ou SEQ ID NO: 2, liée de manière fonctionnelle à un transgène codant pour Dys1 ayant une séquence d'acides aminés de SEQ ID NO: 35, et dans laquelle les particules de rAAV sont AAV8 pour une utilisation dans le traitement de DMD chez un sujet qui en a besoin.

13. Cellule hôte comprenant un plasmide comprenant une cassette d'expression cis, dans laquelle la cassette d'expression cis comprend un promoteur lié de manière fonctionnelle à un transgène, dans laquelle le promoteur comprend une séquence nucléotidique de SEQ ID NO:1 ou SEQ ID NO:2 ou dans laquelle la cassette d'expression cis comprend une séquence nucléotidique comprenant la séquence nucléotidique de SEQ ID NO:38 ou SEQ ID NO:40.

14. Procédé de production d'AAV recombinants comprenant :
(a) la culture d'une cellule hôte contenant :
(i) un génome artificiel comprenant une cassette d'expression cis flanquée par des ITR AAV, où la cassette d'expression cis comprend un promoteur lié de manière fonctionnelle à un transgène codant pour un ou plusieurs produits ARN ou protéiques, où le promoteur comprend la séquence nucléotidique de SEQ ID NO:1 ou SEQ ID NO:2 ;
(ii) une cassette d'expression trans ne comportant pas d'ITR AAV, où la cassette d'expression trans code pour une rep d'AAV et une protéine de capside d'AAV liées de manière fonctionnelle à des éléments de contrôle de l'expression qui conduisent l'expression de la rep d'AAV et de la protéine de capside d'AAV dans la cellule hôte en culture et fournissent la rep d'AAV et la protéine de capside d'AAV en trans ;
(iii) des fonctions auxiliaires d'adénovirus suffisantes pour permettre la réplication et l'encapsidation du génome artificiel par la protéine de capside AAV ; et
(b) la récupération d'un AAV recombinant encapsidant le génome artificiel de la culture cellulaire, éventuellement, au moins 40 % des rAAV produits encapsidant un génome complet.

15. Procédé d'augmentation de l'efficacité d'encapsidation de génomes d'AAV intacts dans des AAV recombinants, comprenant
(a) la culture d'une cellule hôte contenant :
(i) un génome artificiel comprenant une cassette d'expression cis flanquée par des ITR AAV, où la cassette d'expression cis comprend un promoteur lié de manière fonctionnelle à un transgène codant pour un ou plusieurs produits ARN ou protéiques, où le promoteur comprend la séquence nucléotidique de SEQ ID NO:1 ou SEQ ID NO:2 ;
(ii) une cassette d'expression trans ne comportant pas d'ITR AAV, où la cassette d'expression trans code pour une rep d'AAV et une protéine de capside d'AAV liées de manière fonctionnelle à des éléments de contrôle de l'expression qui conduisent l'expression de la rep d'AAV et de la protéine de capside d'AAV dans la cellule hôte en culture et fournissent la rep d'AAV et la protéine de capside d'AAV en trans ;
(iii) des fonctions auxiliaires d'adénovirus suffisantes pour permettre la réplication et l'encapsidation du génome artificiel par la protéine de capside AAV ; et
(b) la récupération d'un AAV recombinant encapsidant le génome artificiel à partir de la culture cellulaire ; dans lequel la présence du promoteur comprenant la séquence nucléotidique de SEQ ID NO:1 ou SEQ ID NO:2 permet une efficacité accrue d'encapsidation de génomes d'AAV intacts, éventuellement dans lequel l'efficacité accrue d'encapsidation de génomes d'AAV intacts est comparée à la production d'AAV recombinants comprenant un promoteur Spc5-12 de type sauvage.
